# EUROPEAN PATENT APPLICATION

(11) **EP 4 529 926 A1**
(43) Date of publication of application: **02.04.2025**
(21) Application number: 23811030.8
(22) Date of filing: 23.05.2023
(51) Int. Cl.: A61K 31/664, A61K 31/675, A61K 31/519, A61K 31/4535, A61P 35/00

(54) **METHOD FOR TREATING CANCER BY COMBINING ALKYLATING AGENT PRODRUG AND CELL CYCLE INHIBITOR**

(30) Priority: 23.05.2022 CN 202210564809; 11.10.2022 CN 202211241825
(71) Applicant: Ascentawits Pharmaceuticals, Ltd., Shenzhen, Guangdong 518118 (CN)
(72) Inventor: MENG, Fanying, San Francisco California 94121 (US); QI, Tianyang, Shenzhen, Guangdong 518118 (CN); DUAN, Jianxin, Shenzhen, Guangdong 518118 (CN); LIU, Xing, Shenzhen, Guangdong 518118 (CN)
(74) Representative: karo IP
(86) International application number: PCT/CN2023/095699
(87) International publication number: WO 2023/226959

(57) **Abstract**

Provided is a method for treating cancer by combining an alkylating agent prodrug and a cell cycle inhibitor. The alkylating agent prodrug may be a prodrug of an AKR1C3 enzyme-activated alkylating agent or a prodrug of a hypoxia-activated alkylating agent. The cell cycle inhibitor may be a CDK inhibitor, a WEE inhibitor, a CHK inhibitor, or an ATR inhibitor.

## Description

### TECHNICAL FIELD

The present invention relates to a method of treating cancer, particularly to treating cancer by using two drugs in combination therapy, and pertains to the field of cancer treatment.

### BACKGROUND

AST-3424 (CAS. No 2097713-69-2), a DNA-alkylating cancer-treatment drug that targets overexpressed aldo-keto reductase family 1 member C3 (AKR1C3), developed by our company (see PCT Pat. App. No. PCT/US2016/021581, published as Pub. No. WO2016/145092, or Chinese Pat. App. No. 2016800150788, published as Pub. No. CN107530556A, entitled "DNA Alkylating Agents", in which the compound TH2870 is disclosed; PCT Pat. App. No. PCT/US2016/062114, published as Pub. No. WO2017087428A1, or Chinese Pat. App. No. 2016800446081, published as Pub. No. CN108290911A, entitled "(R)- and (S)-1-(3-(3-N,N-dimethylaminocarbonyl)phenoxyl-4-nitrophenyl)-1-ethyl -N,N'-bis(ethylene)phosphoramidate, Compositions and Methods for Their Use and Preparation", in which the S-isomer of TH-2870 is disclosed, named as (S)-1-(3-(3-N,N-dimethylaminocarbonyl)phenoxyl -4- nitrophenyl)-1-ethyl -N,N'-bis(ethylene)phosphoramidate, also known as OBI-3424), has a structure represented by the following chemical Formula:

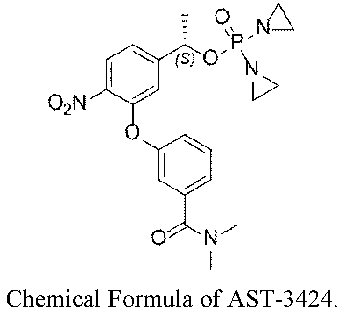

AST-3424 (also known as OBI-3424 and TH-3424), when entering a cancer cell, will be activated by overexpressed AKR1C3-enzyme in the cancer cell to release the metabolite AST2660 (also known as AST-2660) (Meng, F., Li, W. F., Jung, D., Wang, C. C., Qi, T., Shia, C. S., Hsu, R. Y., Hsieh, Y. C., & Duan, J. (2021). A novel selective AKR1C3 enzyme-activated prodrug AST-3424/OBI-3424 exhibits broad anti-tumor activity. American journal of cancer research, 11(7), 3645-3659.). AST-2660 is an alkylating agent and an active chemical ingredient of the prodrug AST-3424, which crosslinks with double-stranded DNA and destroys its structure, inducing cell apoptosis:

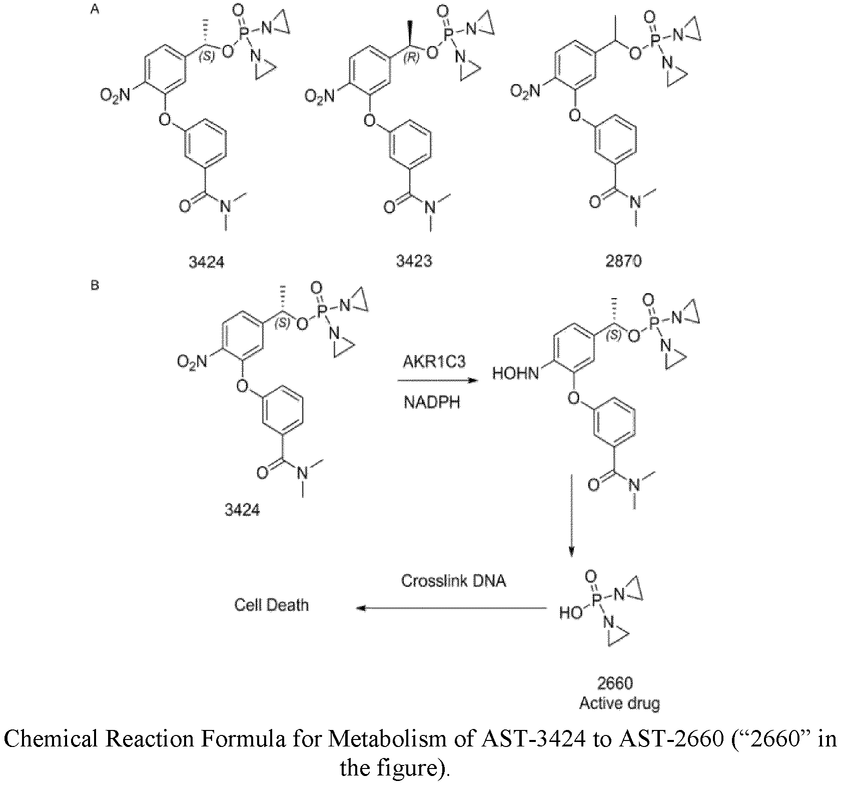

In clinical trials on AST-3424 (Clinical Trials Registry No. NCT03592264, a Phase II study of OBI-3424 being conducted in the United State in subjects with castrate-resistant prostate cancer (CRPC) and hepatocellular carcinoma, sponsored by OBI Pharma, Inc. (Taiwan, China)); Clinical Trials Registry No. NCT04315324, a Phase II study of OBI-3424 being conducted in the United State in subjects with T-cell acute lymphoblastic leukemia (T-ALL), sponsored by Southwest Oncology Group (SWOG, USA); Clinical Trials Registry No. CTR20191399, a Phase II study of AST-3424 being conducted in China in subjects with various solid tumors, sponsored by Ascentawits Pharmaceuticals Ltd. (Shenzhen, China); and Clinical Trials Registry No. CTR20201915, a Phase II study of AST-3424 being conducted in China in subjects with T-ALL and B-cell acute lymphoblastic leukemia (B-ALL), sponsored by Ascentawits Pharmaceuticals Ltd. (Shenzhen, China)), the indications of AST-3424 comprise a wide range of solid tumors and blood tumors including various leukemias. The reason for this is related to the mechanism of action of AST-2660, an alkylating agent, which ultimately works.

### SUMMARY

From in-depth preclinical and clinical trials and studies on AST-3424 and similar AKR1C3 enzyme-activated alkylating agent prodrug and on TH-302 and similar hypoxia-activated alkylating agent prodrugs, the applicant has found that these prodrug compounds, when used in combination therapy with a cell cycle inhibitor, can provide a better tumor inhibition effect.

The present invention provides a method of treatment, a drug for cancer/tumor treatment and pharmaceutical use, as described below.

The method of treatment comprises the step of treating a cancer/tumor patient by using a drug containing an alkylating agent prodrug compound or salt, ester, solvate or isotopomer thereof in combination therapy with a drug containing a cell cycle inhibitor compound or salt, ester, solvate or isotopomer thereof.

The pharmaceutical use comprises, by using an alkylating agent prodrug compound or salt, ester, solvate or isotopomer thereof, preparing a drug for treating a cancer/tumor patient in combination therapy with a drug containing a cell cycle inhibitor compound or salt, ester, solvate or isotopomer thereof.

The drug for cancer/tumor treatment contains an alkylating agent prodrug compound or salt, ester, solvate or isotopomer thereof and the indications thereof comprise treating a cancer/tumor patient in combination therapy with a drug containing a cell cycle inhibitor compound or salt, ester, solvate or isotopomer thereof.

The present invention also provides a pharmaceutical composition for treating a cancer/tumor patient, which comprises an alkylating agent prodrug compound or salt, ester, solvate or isotopomer thereof and a cell cycle inhibitor compound or salt, ester, solvate or isotopomer thereof.

The present invention also provides pharmaceutical use, wherein an alkylating agent prodrug compound or salt, ester, solvate or isotopomer thereof is used for preparing a drug for treating a cancer/tumor patient in combination therapy with a cell cycle inhibitor compound or salt, ester, solvate or isotopomer thereof.

The cell cycle inhibitor may be selected from a CDK inhibitor, a CHK inhibitor, an ATR inhibitor or a Wee inhibitor.

The Wee inhibitors may be a Wee1 inhibitor.

The alkylating agent prodrug compound may be selected from an AKR1C3 enzyme-activated alkylating agent prodrug compound or a hypoxia-activated alkylating agent prodrug compound, preferably from an AKR1C3 enzyme-activated DNA alkylating agent prodrug compound or a hypoxia-activated DNA alkylating agent prodrug compound.

Preferably, the AKR1C3 enzyme-activated alkylating agent prodrug compound is used in combination therapy with a CDK inhibitor, a Wee inhibitor, CHK inhibitor or an ATR inhibitor.

Preferably, the hypoxia-activated alkylating agent prodrug compound is used in combination therapy with a CDK inhibitor, a Wee inhibitor or an ATR inhibitor.

The cell cycle is a highly regulated process that facilitates cell growth, genetic material replication and cell division. The core cell cycle machinery operating in the cell nucleus drives transition from one phase of the cell cycle to the next. Cell cycle inhibitors are substances that inhibit the normal progression of the cell cycle.

The cell cycle can be divided into the following phases: pre-DNA synthesis (G1), DNA synthesis (S), post-DNA synthesis (G2) and mitosis (M). In the G1 phase, celles are mainly engaged in synthesis of ribosomes, proteins and other materials necessary for mitosis than DNA. In the S phase, cells are mainly engaged in DNA synthesis and replication. In this way, structures capable of facilitating DNA replication are formed usually with the assistance of topoisomerase. In the G2 phase, cells continue to synthesize materials necessary for mitosis, including microtubules and the like. In the M phase, under the action of microtubules and the like, cells divide into two daughter cells.

The cell cycle is closely related to cyclins and their catalytic partners, cyclin-dependent kinases, Wee, cell-cycle checkpoint kinases (CHKs) and the ataxia telangiectasia and Rad3-related (ATR) kinase.

The four cell cycle phases require different cyclin-dependent kinases (CDKs). For example, cyclin-CDK4/6 controls the G1/S transition, and CDK1 controls the G2/M transition.

Cell cycle inhibitors specifically target specific cell cycle phases. Specifically, such an inhibitor blocks a specific substance or process that controls a targeted cell cycle phase from performing its function properly.

CDK inhibitors (CDKis) inactivate corresponding CDKs involved in cell cycle progression of cancer cells, thereby preventing their replication and proliferation and ultimately inducing their apoptosis.

Different inhibitors have been developed against the known CDK isoforms: CDK1, CDK2, CDK3, CDK4, CDK5, CDK6, CDK7, CDK8, CDK9, CDK11, CDK12, CDK13, CDK14, CDK16, CDK19, CDC and CLK.

Wee (Wee1) is identified as an important kinase that participates in DNA damage repair (DDR) pathways. Wee1 is a serine/threonine protein kinase that inactivates CDK1/2 and blocks the cell cycle at G2/M, providing time for DNA repair. Wee1 is one of the targets that have been preliminarily proven in anti-cancer drug studies to have a synthetic lethality effect with TP53 gene mutations widely observed in tumor cells.

As a cell cycle surveillance system, the DNA damage checkpoints can prevent genomic instability. As part of the DNA damage checkpoints, the DNA damage checkpoint kinases CHK1 and CHK2 are core factors in inducing cell cycle arrest, DNA repair and cell apoptosis.

When DNA in a cell is damaged, the cell cycle checkpoints are activated to induce cell cycle arrest to assist in DNA repair for maintaining the cell's genomic integrity and stability. In the long-term biological evolution, the cell cycle checkpoints have developed a conservative signaling and regulation system involving various regulatory checkpoint kinases (CHKs), which can induce cell cycle arrest for cellular DNA repair.

The ataxia telangiectasia and Rad3-related kinase (ATR) is a serine-threonine kinase and is a member of the phosphatidylinositol 3-kinase-related kinase (PIKK) family. It consists of 2644 amino acid residues and has an N-terminal ATR-interacting protein (ATRIP) domain, which is an important region required for the activation of the ATR kinase. It also has a C-terminal kinase domain capable of phosphorylating a downstream protein. For example, the domain can phosphorylate a serine or threonine residue in a targeted protein, such as the cell cycle checkpoint kinase 1 (CHK1). When activated, the ATR kinase can regulate cellular biological processes, including cell cycle arrest, inhibition of replication origin firing, promotion of deoxynucleotide synthesis, replication fork firing and repair of DNA double-strand breaks, through a variety of signaling pathways. In the event of DNA damage, ATR can activate cellular response to arrest the cell cycle, and then stabilize the replication fork and repair DNA, thereby preventing cell apoptosis. Therefore, it is very important to life activities.

The alkylating agent prodrug compound is a prodrug compound that can be converted in vivo to an alkylating agent.

The alkylating agent prodrug compound may be selected from an AKR1C3 enzyme-activated alkylating agent prodrug compound and a hypoxia-activated alkylating agent prodrug compound, preferably from an AKR1C3 enzyme-activated DNA alkylating agent prodrug compound and a hypoxia-activated DNA alkylating agent prodrug compound.

The alkylating agent prodrug compound comprises the chemical Formulae (1) to (9): 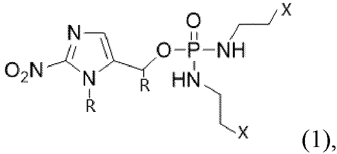 wherein the Formula (1) is a hypoxia-activated alkylating agent prodrug compound. More specifically, it is a hypoxia-activated DNA alkylating agent prodrug compound.

In this formula, each R is independently selected from H, -CH₃, -CH₂CH₃ and -CF₃, and each X is independently selected from leaving functional groups including Cl, Br, MsO and TsO.

Formulations related to TH-302 or its analog compound 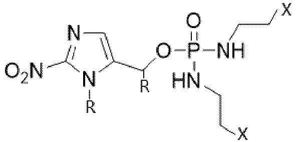 include oral formulations, lyophilized formulations and concentrated injectable solutions, and related regimens, methods of preparation, clinical compatibility and modes of administration have been detailed and disclosed in the following related patent applications filed by Threshold: WO2010048330A1, WO2012142520A2 and WO2008083101A1, the entirety of which is hereby incorporated by reference.

TH-302 or its analog compound 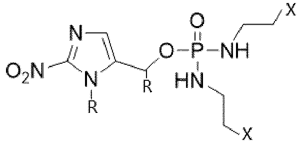 is a DNA-alkylating anti-cancer drug with an extensive cancer treatment potential. Experiments on such related cancer indications and clinical trials have been disclosed in related patent applications filed by Threshold and other pharmaceutical companies (e.g., WO2016011195A2, WO2004087075A1, WO2007002931A1, WO2008151253A2, WO2009018163A1, WO2009033165A2, WO2010048330A2, WO2012142520A1, WO2008083101A2, WO2020007106A1, WO2020118251A1, WO2014169035A1, WO2013116385A1, WO2019173799A2, WO2016081547A1, WO2014062856A1, WO2015069489A1, WO2012006032A2, WO2018026606A2, WO2010048330A2, WO2015171647A1, WO2013096687A1, WO2013126539A2, WO2013096684A2, WO2012009288A2, WO2012145684A2, WO2016014390A2, WO2019055786A2, WO2012135757A2, WO2015013448A2, WO2016011328A2, WO2013177633A2, WO2016011195A2, WO2015051921A2), as well as in FDA-registered clinical trials (NCT02402062, NCT02020226, NCT02076230, NCT01381822, NCT02093962, NCT01440088, NCT02255110, NCT02342379, NCT01864538, NCT01149915, NCT02433639, NCT00743379, NCT01485042, NCT01721941, NCT02047500, NCT00742963, NCT01497444, NCT00495144, NCT01746979, NCT01144455, NCT01403610, NCT01522872, NCT01833546, NCT02598687, NCT03098160, NCT02496832, NCT02712567). The entirety of the foregoing related applications and clinical trial information is hereby incorporated by reference. 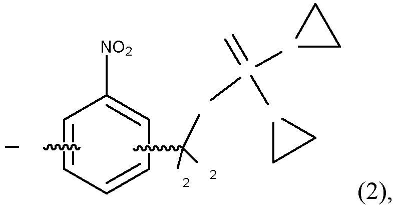 wherein Formula (2) is a hypoxia-activated alkylating agent prodrug compound. More specifically, it is s hypoxia-activated DNA alkylating agent prodrug compound.

In this formula, R₁, R₂, R₃ and Cx are defined as in claims of Pat. App. No. PCT/CN2020/114519, published as Pub. No. WO2021120717A1. This publication also describes synthesis of these compounds, and its entirety is hereby incorporated by reference. Detailed definitions are as follows.

Cx is a 5-10 membered aromatic or heteroaromatic ring, heteroaliphatic ring or cycloalkane, which shares two carbon atoms with the nitrobenzene ring so that they together form a fused ring structure.

R₁ is attached to any ring atom of the Cx ring, and is selected from hydrogen, a halo atom, cyano or isocyano, hydroxyl, thiol, amino, OTs, C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, 5-15 membered or Z-substituted heteroaryl, alkoxyl or Z-substituted alkoxyl comprising 1-6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷ and -NR⁶SO₂NR⁶R⁷.

R₂ and R₃ are each independently hydrogen, a C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, or 5-15 membered or Z-substituted heteroaryl. Alternatively, R₂ and R₃ form a 3-6 membered ring together with the benzyl carbon atom to which they are bonded.

Any of the hydrogen atoms on the fused carbon atoms can be mono-substituted with the 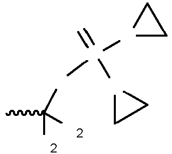 group.

Each Z substituent is a halo atom, cyano or isocyano, hydroxyl, thiol, amino, C₁-C₃ or substituted alkyl, C₁-C₃ or substituted alkoxyl, C₂-C₃ or substituted alkenyl, C₂-C₃ or substituted alkynyl, or C₃-C₈ or substituted cycloalkyl.

R⁶ and R⁷ are each independently hydrogen, a C₁-C₆ alkyl or Z-substituted C₁-C₆ alkyl, C₂-C₆ alkenyl or Z-substituted C₂-C₆ alkenyl, C₂-C₆ alkynyl or Z-substituted C₂-C₆ alkynyl, C₃-C₈ cycloalkyl or Z-substituted C₃-C₈ cycloalkyl, C₆-C₁₀ aryl or Z-substituted C₆-C₁₀ aryl, 4-15 membered heterocycle or Z-substituted 4-15 membered heterocycle, or 5-15 membered heteroaryl or Z-substituted 5-15 membered heteroaryl. Alternatively, R⁶ and R⁷ form a 5-7 membered heterocycle or Z-substituted 5-7 membered heterocycle together with the atom to which they are bonded.

The alkylating agent prodrug compound of formula 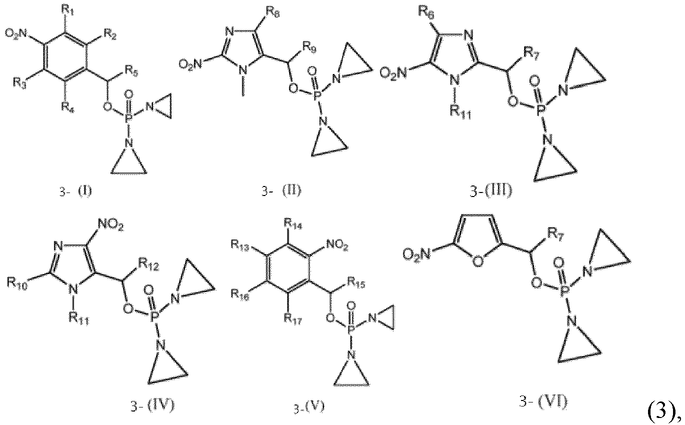 wherein Formula (3) is a hypoxia-activated alkylating agent prodrug compound. More specifically, it is a hypoxia-activated DNA alkylating agent prodrug compound.

In this formula, R₁, R₂, R₃, R₄, R₅, R₆, R₇, R₈, R₉, R₁₀, R₁₁, R₁₂, R₁₃, R₁₄, R₁₅, R₁₆ and R₁₇ are defined as in claims of Pat. App. No. PCT/US2016/039092, published as Pub. No. WO2016210175A1 (or Chinese Pat. App. No. 2016800368985, published as Pub. No. CN108024974A). This publication also describes synthesis of these compounds, and its entirety is hereby incorporated by reference. Detailed definitions are as follows.

R₁ is hydrogen, -N₃, CN, a halo group, NR²¹R²², -OR²³, -SO₂(C₁-C₆ alkyl), C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or ether.

R²¹ and R²² are each independently hydrogen, a hydroxyl, C₁-C₆ alkyl, C₂-C₆ alkenyl, C₂-C₆ alkynyl, C₃-C₈ cycloalkyl, C₆-C₁₀ aryl, 4-15 membered heterocycle, 5-15 membered heteroaryl or -SO₂(C₁-C₆ alkyl). Alternatively, R²¹ and R²² form a 4-15 membered heterocycle or 5-15 membered heteroaryl together with the nitrogen atom to which they are bonded.

R²³ is hydrogen, a C₁-C₆ alkyl or C₆-C₁₀ aryl.

R₂ and R₃ are each independently hydrogen or a halo group.

R₄ is hydrogen, a halo group, C₁-C₆ alkoxyl, C₁-C₆ alkyl or C₆-C₁₀ aryl.

R₅, R₇, R₉, R₁₂ and R₁₅ are each independently hydrogen, a C₁-C₆ alkyl, C₂-C₆ alkenyl, C₂-C₆ alkynyl, C₃-C₈ cycloalkyl, C₆-C₁₀ aryl, 4-15 membered heterocycle or 5-15 membered heteroaryl. Alternatively, R₄ and R₅ form a C₅-C₆ cycloalkyl ring together with intervening carbon atom(s) therebetween.

R₆ and R₁₀ are each independently hydrogen or a halo group.

R₈ is hydrogen, a C₁-C₆ alkyl, C₂-C₆ alkenyl, C₂-C₆ alkynyl or 5-15 membered heteroaryl.

Each R₁₁ is independently a C₁-C₆ alkyl, C₂-C₆ alkenyl, C₂-C₆ alkynyl, C₃-C₈ cycloalkyl or C₆-C₁₀ aryl.

R₁₃, R₁₄, R₁₆ and R₁₇ are each independently hydrogen, a halo group, C₁-C₆ alkyl, C₂-C₆ alkenyl, C₂-C₆ alkynyl or C₁-C₆ alkoxyl.

The alkyl, alkenyl, alkynyl, cycloalkyl, aryl, heterocyclic, heteroaryl, alkoxyl and ether groups may be optionally substituted. 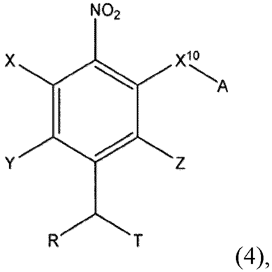 wherein Formula (4) is an AKR1C3 enzyme-activated alkylating agent prodrug compound. More specifically, it is an AKR1C3 enzyme-activated DNA alkylating agent prodrug compound.

In this formula, X, Y, Z, R, T, A and X¹⁰ are defined as in claims of Pat. App. No. PCT/US2016/021581, published as Pub. No. WO2016145092A1 (or Chinese Pat. App. No. 2016800150788, published as Pub. No. CN107530556A). This publication also describes synthesis of these compounds, and its entirety is hereby incorporated by reference. Detailed definitions are as follows.

X¹⁰ is O, S, SO or SO₂.

A is a C₆-C₁₀ aryl, 5-15 membered heteroaryl or -N=CR¹R².

R¹ and R² are each independently hydrogen, a C₁-C₆ alkyl, C₃-C₈ cycloalkyl, C₆-C₁₀ aryl, 4-15 membered heterocycle, ether, -CONR¹³R¹⁴ or -NR¹³COR¹⁴.

X, Y and Z are each independently hydrogen, CN, a halo group, C₁-C₆ alkyl, C₂-C₆ alkenyl, C₂-C₆ alkynyl, C₃-C₈ cycloalkyl, C₆-C₁₀ aryl, 4-15 membered heterocycle, ether, - CONR¹³R¹⁴ or -NR¹³COR¹⁴.

R is hydrogen, a C₁-C₆ alkyl, C₂-C₆ alkenyl, C₂-C₆ alkynyl, C₃-C₈ cycloalkyl, C₆-C₁₀ aryl, 4-15 membered heterocycle, ether, -CONR¹³R¹⁴ or -NR¹³COR¹⁴.

R¹³ and R¹⁴ are each independently hydrogen, a C₁-C₆ alkyl, C₃-C₈ cycloalkyl, C₆-C₁₀ aryl, 4-15 membered heterocycle or ether.

T comprises an alkylating phosphoramidate moiety containing one or more alkylating moieties bonded to a Z⁵-X⁵-Y⁵ moiety of a -O-P(Z¹) moiety, where Z⁵ is a heteroatom comprising nitrogen, sulfur or oxygen; X⁵ is a substituted or unsubstituted ethylene group; Y⁵ is a halo group or another leaving group. Alternatively, Z⁵-X⁵-Y⁵ forms an aziridinyl (NCH₂CH₂) moiety, where Z¹ is O or S.

Moreover, the alkyl, alkenyl, alkynyl, cycloalkyl, aryl, heterocyclic, heteroaryl and ether groups may be substituted or not. 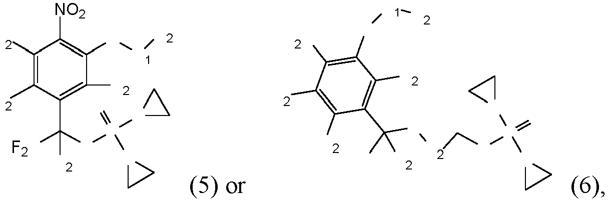 wherein Formula (5) and Formula (6) are AKR1C3 enzyme-activated alkylating agent prodrug compounds. More specifically, they are AKR1C3 enzyme-activated DNA alkylating agent prodrug compounds.

In the Formula (5) and Formula (6), R₁, R₂, R₃, R₄, R₅, R₈, R₉ and R₁₀ are defined as in claims of Pat. App. No. PCT/CN2020/089692, published as Pub. No. WO2020228685A1. This publication also describes synthesis of these compounds, and its entirety is hereby incorporated by reference. Detailed definitions are as follows.

R₁ is a C₆-C₁₀ or Z-substituted aryl, 4-15 membered or Z-substituted heterocycle, 5-15 membered or Z-substituted heteroaryl, or 7-15 membered or Z-substituted fused ring.

R₂ is hydrogen, a halo atom, cyano or isocyano, hydroxyl, thiol, amino, OTs, OMS, C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, 5-15 membered or Z-substituted heteroaryl, ether containing 1-6 carbon atoms, Z-substituted alkoxyl containing 1-6 carbon atoms, -CONR⁶R⁷, -SO₂NR⁶R⁷, -SO₂R⁶, -OCOO-R⁶, -COOR⁶, -NR⁶COR⁷, -OCOR⁶, -NR⁶SO₂R⁷ or -NR⁶SO₂NR⁶R⁷. Alternatively, R₂ forms a 7-15 membered or Z-substituted fused ring together with the atoms in the R₁ group to which it is bonded.

R₃ is hydrogen, a halo atom, cyano or isocyano, hydroxyl, thiol, amino, OTs, OMS, C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, 5-15 membered or Z-substituted heteroaryl, C₁-C₆ alkoxyl or Z-substituted C₁-C₆ alkoxyl, -CONR⁶R⁷, -SO₂NR⁶R⁷, -SO₂R⁶, -OCO-R⁶, -OCOO-R⁶, - COOR⁶, -NR⁶COR⁷, -OCOR⁶ or -NR⁶SO₂R⁷.

R₄ and R₅ are each independently hydrogen, a halo atom, cyano or isocyano, hydroxyl, thiol, amino, OTs, OMS, C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, 5-15 membered or Z-substituted heteroaryl, C₁-C₆ alkoxyl or Z-substituted C₁-C₆ alkoxyl, -CONR⁶R⁷, - SO₂NR⁶R⁷, -SO₂R⁶, -OCOO-R⁶, -COOR⁶, -NR⁶COR⁶, -OCOR⁶ or -NR⁶SO₂R⁷. Alternatively, R₄ and R₅ form a 7-15 membered or Z-substituted fused ring together with the atoms of the phenyl ring to which they are bonded.

R⁶ and R⁷ are each independently hydrogen, a cyano or isocyano, C₁-C₆ or Z-substituted alkyl, C₂-C₆ or Z-substituted alkenyl, C₂-C₆ or Z-substituted alkynyl, C₃-C₈ or Z-substituted cycloalkyl, C₆-C₁₀ or Z-substituted aryl, 4-15 membered or Z-substituted heterocycle, 5-15 membered or Z-substituted heteroaryl, or C₁-C₆ alkoxyl or Z-substituted C₁-C₆ alkoxyl. Alternatively, R⁶ and R⁷ form a 5-7 membered heterocycle or Z-substituted 5-7 membered heterocycle together with the atom to which they are bonded.

R₅ and R₁₀ are each independently a hydrogen or deuterium atom, aryl or Z-substituted aryl, C₁-C₆ or Z-substituted alkyl, C₂-C₆ or Z-substituted alkenyl, C₂-C₆ or Z-substituted alkynyl, or C₃-C₈ or Z-substituted cycloalkyl, and one of them must be a hydrogen or deuterium atom.

R₉ is a substituted C₆-C₁₀ aryl substituted with at least one fluorine atom or nitro group, a substituted 4-15 membered heterocycle substituted with at least one fluorine atom or nitro group, or a substituted 5-15 membered heteroaryl substituted with at least one fluorine atom or nitro group.

Each Z substituent is a halo atom, cyano or isocyano, hydroxyl, thiol, amino, OTs, OMS, C₁-C₃ or substituted alkyl, C₁-C₃ or substituted alkoxyl, C₂-C₃ or substituted alkenyl, C₂-C₃ or substituted alkynyl, C₃-C₈ or substituted cycloalkyl, aromatic, heterocyclic, heteroaromatic or fused ring, or substituted aromatic, heterocyclic, heteroaromatic or fused ring. Each Z substitution may be a mono-substitution or geminal di-substitution.

The substituted C₆-C₁₀ aryl, 4-15 membered heterocycle or 5-15 membered heteroaryl in R₉ is substituted with a halo atom, nitro, cyano or isocyano, hydroxyl, amino, C₁-C₃ alkyl or alkoxyl, alkenyl, alkynyl, cycloalkyl or phenyl, substituted phenyl, C₁-C₃ alkoxyl or haloalkoxyl. 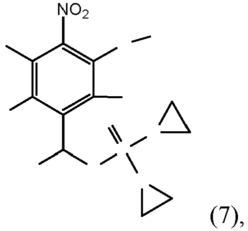 wherein Formula (7) is an AKR1C3 enzyme-activated alkylating agent prodrug compound. More specifically, it is an AKR1C3 enzyme-activated DNA alkylating agent prodrug compound.

In this formula:
A is a substituted or unsubstituted C₆-C₁₀ aryl, biaryl or substituted biaryl, 5-15 membered heteroaryl or -N=CR¹R², wherein each of the substituted groups is substituted with a substituent selected from the group consisting of a halo group, -CN, -NO₂, -O-(CH₂)-O-, -CO₂H or salt thereof, -OR¹⁰⁰, -CO₂R¹⁰⁰, -CONR¹⁰¹R¹⁰², -NR¹⁰¹R¹⁰², - NR¹⁰⁰SO₂R¹⁰⁰, -SO₂R¹⁰⁰, -SO₂NR¹⁰¹R¹⁰², C₁-C₆ alkyl and C₃-C₁₀ heterocycle,
wherein R¹⁰⁰, R¹⁰¹ and R¹⁰² are each independently hydrogen, a C₁-C₈ alkyl or C₆-C₁₂ aryl, or R¹⁰¹ and R¹⁰² together with the nitrogen atom to which they are attached form a 5-7 membered heterocycle,
wherein the alkyl and aryl are each substituted with 1-3 halo groups or 1-3 C₁-C₆ alkyls, and
R¹ and R² are each independently a phenyl or methyl;
X, Y and Z are each independently hydrogen or a halo group; and
R is hydrogen, a C₁-C₆ alkyl or haloalkyl. 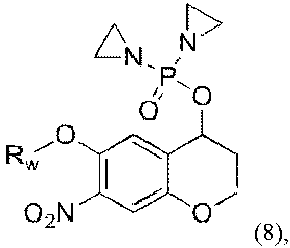
wherein Formula (8) is an AKR1C3 enzyme-activated alkylating agent prodrug compound. More specifically, it is an AKR1C3 enzyme-activated DNA alkylating agent prodrug compound.

In this formula, Rw is defined as in claims of Pat. App. No. PCT/CN2020/120281, published as Pub. No. WO2021068952A1. This publication also describes synthesis of these compounds, and its entirety is hereby incorporated by reference. Detailed definitions are as follows.

Rw is

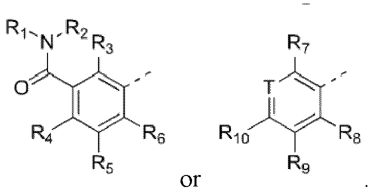

R₁ is H, a C₁₋₆ alkyl, C₃₋₆ cycloalkyl, 4-6 membered heterocyclic alkyl, 5-6 membered heteroaryl or phenyl, where each of the C₁₋₆ alkyl, C₃₋₆ cycloalkyl, 4-6 membered heterocyclic alkyl, 5-6 membered heteroaryl and phenyl is optionally substituted with 1, 2 or 3 R^{a}'s.

Each R^{a} group is independently H, F, Cl, Br, I, -CN, -OH, C₁₋₃ alkoxyl or C₁₋₃ alkyl.

R₂ is H or C₁₋₆ alkyl.

Alternatively, R₁ and R₂ are connected together and form, together with the N atom to which they are attached, a 4-6 membered heterocyclic alkyl, which may be optionally substituted with 1, 2 or 3 R^{b}'s.

Each R^{b} is independently H, F, Cl, Br, I, -CN, -OH, -NH₂, -OCH₃, -OCH₂CH₃, -CH₃ or -CH₂CH₃.

R₃ is H, F, Cl, Br, I, -OH, -NH₂, C₁₋₃ alkoxyl or C₁₋₃ alkyl.

Alternatively, R₂ and R₃ are connected together, turning the moiety 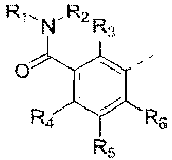 into 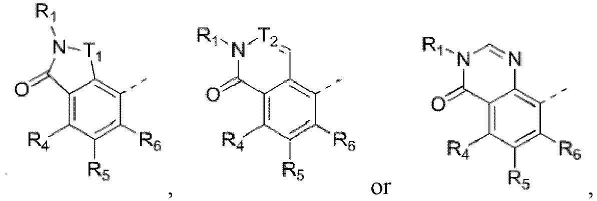 where:
T₁ is -(CR^{c}R^{d})ₘ- or -(CR^{c}R^{d})ₙ-O-;
m is 1, 2 or 3;
n is 1 or 2;
T₂ is N or CH;
R^{c} and R^{d} are each independently H, F, C₁₋₃ alkyl or C₁₋₃ alkoxyl;
R₄, R₅ and R₆ are each independently H, F, Cl, Br, I, C₁₋₃ alkyl or C₁₋₃ alkoxyl;
T is N or CH;
R₇ and R₈ are each independently H, F, Cl, Br or I;
R₉ and R₁₀ are each independently H, F, Cl, Br, I or -CN; or
the 4-6 membered heterocyclic alkyl and the 5-6 membered heteroaryl each contain 1, 2, 3 or 4 heteroatoms independently selected from N, -O- and -S-.

The alkylating agent prodrug compound of formula 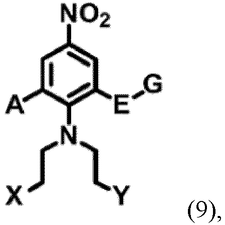 wherein Formula (9) is an AKR1C3 enzyme-activated alkylating agent prodrug compound. More specifically, it is an AKR1C3 enzyme-activated DNA alkylating agent prodrug compound.

In the formula, A, E, G, X and Y are defined as in claims of Pat. App. No. PCT/NZ2019/050030, published as Pub. No. WO2019190331A1 (or Chinese Pat. App. No. 2019800234236, published as Pub. No. CN111918864A). This publication also describes synthesis of these compounds, and its entirety is hereby incorporated by reference. Detailed definitions are as follows.

A is H, a C₁-C₆ alkyl, C₁-C₆ alkenyl, C₁-C₆ alkynyl, CFH₂, CF₂H, CF₃, F, Cl, Br, I, OCF₃, COR or CON(R)₂.

E is SO or SO₂.

X is Cl, Br, I or OSO₂R.

Y is Cl, Br, I or OSO₂R.

Each R is independently H or C₁-C₆ alkyl.

G is selected from radicals including those of formulae (B) to (AA): 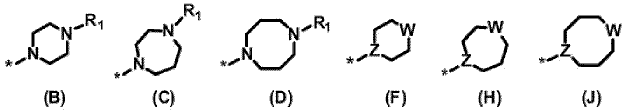 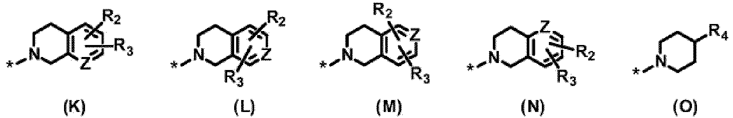 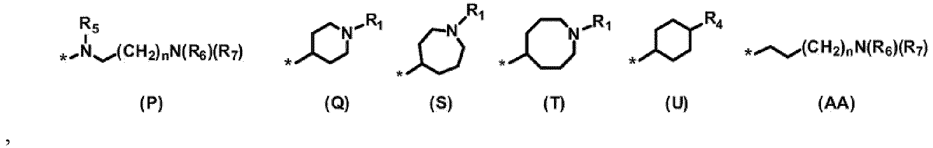 where:
R₁ is H, a C₁-C₆ alkyl, CH₂(CH₂)nOH, CH₂CH(OH)CH₂OH, phenyl, pyridyl, benzyl or picolyl, provided that when it is a phenyl, pyridyl, benzyl or picolyl, it is optionally substituted at any available position with a C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO₂R₆, SO₂N(R₆)(R₇), CN or NO₂;
R₂ and R₃ are each independently H, C₁-C₆ alkyl, C₁-C₆ alkenyl, C₁-C₆ alkynyl, OR₆, N(R₆)(R₇), CFH₂, CF₂H, CF₃, F, Cl, Br, I, OCF₃, COR₆, CON(R₆)(R₇), SOR₆, SON(R₆)(R₇), SO2R₆, SO2N(R₆)(R₇), CN or NO₂;
R₄ is N(R₆)(R₇), OH, OCH₂(CH₂)nN(R₆)(R₇) or CH₂(CH₂)nN(R₆)(R₇);
R₅ is H or a C₁-C₆ alkyl;
R₆ and R₇ are each independently H or a C₁₋₆ alkyl, or R₆ and R₇ together form a substituted or unsubstituted 5 or 6 membered heterocycle;
Z is CH or N;
W is CH₂, O, S, SO or SO₂;
n is 0-6;
* represents a linking position to formula (I).

In the context herein, the "drug" refers to a pharmaceutical product or formulation. The pharmaceutical product is prepared so as to contain a hypoxia activated compound of formula (I), or a salt or solvate thereof, as an active ingredient, within a particular dose range, and/or the drug is prepared in a particular form of dosage, or for a particular mode of administration.

In addition to the alkylating agent prodrug compound of any of formulae (1) to (9), the drug may also contain a pharmaceutically acceptable adjuvant or excipient, depending on the specific requirements of a pharmaceutical product, medication or formulation. The drug may be in any dosage form for clinical administration, such as tablets, suppositories, dispersible tablets, enteric-coated tablets, chews, orally disintegrating tablets, capsules, dragees, granules, dry powders, oral solutions, solutions for injection in vials or pre-filled syringes, lyophilized powders for injection or infusion solutions. Depending on the dosage form and mode of administration of the drug, the pharmaceutically acceptable adjuvant or excipient may include one or more of a diluent, a solubilizing agent, a disintegrator, a suspending agent, a lubricant, a binding agent, filler, a flavouring agent, a sweetener, an antioxidant, a surfactant, a preservative, a coating agent, a coloring agent and the like.

"Cancer" refers to leukemias, lymphomas, carcinomas and other malignant tumors (including solid tumors) of potentially unlimited growth, which can expand locally by invasion and systemically by metastasis.

Examples of cancers that can be treated with AST-3424 or other such AKR1C3 enzyme-activated DNA alkylating agent prodrugs, as listed herein, include (but are not limited to) cancer of the adrenal gland, bone, brain, breast, bronchi, colon and/or rectum, gallbladder, head and neck, kidneys, larynx, liver, lung, neural tissue, pancreas, prostate, parathyroid, skin, stomach, and thyroid. Some other examples of cancers include, acute and chronic lymphocytic and granulocytic tumors, adenocarcinoma, adenoma, basal cell carcinoma, cervical dysplasia and in situ carcinoma, Ewing's sarcoma, epidermoid carcinomas, giant cell tumor, glioblastoma multiforme, hairy-cell tumor, intestinal ganglioneuroma, hyperplastic corneal nerve tumor, islet cell carcinoma, Kaposi's sarcoma, leiomyoma, leukemias, lymphomas, malignant carcinoid, malignant melanomas, malignant hypercalcemia, marfanoid habitus tumor, medullary carcinoma, metastatic skin carcinoma, mucosal neuroma, myeloma, mycosis fungoides, neuroblastoma, osteosarcoma, osteogenic and other sarcoma, ovarian tumor, pheochromocytoma, polycythemia vera, primary brain tumor, small-cell lung cancer, squamous cell carcinoma of both ulcerating and papillary type, hyperplasia, seminoma, soft tissue sarcoma, retinoblastoma, rhabdomyosarcoma, renal cell tumor, topical skin lesion, reticulum cell sarcoma and Wilm's tumor.

"Combination therapy" is also known as "combination drug treatment". "Single-drug treatment" refers to using only one anti-cancer drug in one course of treatment. "Combination therapy" refers to simultaneously or successively using two or more anti-cancer drugs in one course of treatment.

In general, for combination therapy, it is necessary to attempt various doses and periods of administration according to the characteristics of the condition to be treated and the drugs to be used in combination. A combination therapy regimen can achieve better therapeutic outcomes than monotherapy only if it has been obtained from attempts made according to the aforementioned factors.

Doses and periods of administration for combination therapy regimens should be determined by clinical trials made with reference to doses and dosage regimens for the alkylating agent prodrug compound and the other drug in combination therapy therewith.

The cancer/tumor patient or a biological sample thereof has been detected with loss or damage of a specific gene selected from genes involved in cell cycle checkpoint regulation, preferably from p53, p21, CCNB1, WIP1, 14-3-3 sigma protein and cdc2/cycB.

These genes have been confirmed to be involved in cell cycle checkpoint regulation, according to relevant research literature (Molinari, M. (2000), Cell cycle checkpoints and their inactivation in human cancer. Cell Proliferation, 33: 261-274. https://doi.org/10.1046/j.1365-2184.2000.00191.x; Donehower L. A. (2014). Phosphatases reverse p53-mediated cell cycle checkpoints. Proceedings of the National Academy of Sciences of the United States of America, 111(20), 7172-7173. https://doi.org/10.1073/pnas.1405663111).

The cell cycle inhibitor may be a CDK inhibitor, a Wee inhibitor, a CHK inhibitor or an ATR inhibitor.

Preferably, the CDK inhibitor may be selected from palbociclib, ribociclib, abemaciclib, trilaciclib, dalpiciclib, adavosertib, Ro-3306, dinaciclib, cirtuvivint, rintodestrant, DS96432529, THZ1, THZ531, seliciclib, flavopiridol, AZD4573, SR-4835, simurosertib, fadraciclib, NVP-2, SNS-032, (E/Z)-zotiraciclib, AZD-5438, AT7519, mevociclib, kenpaullone, YKL-5-124 TFA, NG 52, GSK-3 Inhibitor IX, OTS964, samuraciclib, flavopiridol, KB-0742 dihydrochloride, (+)-enitociclib, AUZ 454, SY-5609, SEL120-34A monohydrochloride, CCT-251921, MBQ-167, XL413 hydrochloride, BI-1347, THAL-SNS-032, JNJ-7706621, TG003, LDC4297, BMS-265246, roniciclib, CGP60474, (R)-CR8 trihydrochloride, R547, milciclib, T025, AS2863619, Senexin A, BSJ-4-116, CLK-IN-T3, CDK12-IN-3, CVT-313, atuveciclib, PHA-793887, indirubin-3'-monoxime, YKL-5-124, PHA-767491 hydrochloride, KH-CB19, cucurbitacin E, purvalanol A, BSJ-03-204, ON123300, CDK5 inhibitor 20-223, riviciclib, FN-1501, CP-10, THZ2, abemaciclib metabolite M2, BS-181, CDK12-IN-E9, samuraciclib, RGB-286638, CDK2-IN-4, LDC000067, ML167, purvalanol B, NU6300, CLK1-IN-1, FMF-04-159-2, CKI-7, CDKI-73, MSC2530818, BSJ-04-132, NU6102, voruciclib, olomoucine and so forth. The list of these compounds, as well as their detailed structures and supply information, can be found on the websites of commercial reagent suppliers, such as MedChemExpress (MCE) (https://www.medchemexpress.cn/Targets/CDK.html?effectName=Inhibitor).

Preferably, the Wee inhibitor may be selected from Wee1-IN-5, Wee1-IN-3, Wee1-IN-4, LEB-03-146, LEB-03-144, adavosertib, LEB-03-153, LEB-03-145, PD407824, PD0166285, PD0166285 dihydrochloride, pomalidomide-C3-adavosertib, DB0614, FMF-06-098-1, and so forth. The list of these compounds, as well as their detailed structures and supply information, can be found on the websites of commercial reagent suppliers, such as MedChemExpress (MCE) (https://www.medchemexpress.cn/search.html?q=Wee&ft=&fa=&fp=&fsp=&ftag=&fsc=)

Preferably, the CHK inhibitor may be selected from AZD7762, prexasertib, SCH900776, GDC-0425, CHK1-IN-6, CCT245737, BML-277, CCT241533, PD407824, CHIR-124, CCT244747, PF477736, GDC-0575, SB-218078, MRT00033659, ANI-7, SAR-020106, CCT241533, CHK1-IN-4, VER-00158411, CHK1-IN-3, CHK1-IN-5, CHK1-IN-2, CHK-IN-1 and so forth. The list of these compounds, as well as their detailed structures and supply information, can be found on the websites of commercial reagent suppliers, such as MedChemExpress (MCE) (https://www.medchemexpress.cn/Targets/Checkpoint%20Kinase%20(Chk).html).

Preferably, the ATR inhibitor may be selected from ceralasertib, berzosertib, gartisertib, BAY1895344, BAY-937, AZ20, ETP-46464, dactolisib, VE-821, M1774, ATRN-199, RP-3500 and ART-0380. Information of these compounds can be founded in the publication by Yuan Yinghui, Duan Jilong, Hui Zi, et al. (Research Progress of ATR Kinase-Targeted Inhibitors in the Cancer Therapy [J]. Acta Pharmaceutica Sinica, 2022(057-003)).

In particular, a compound of formula (1) may be selected from the group of the following structures:

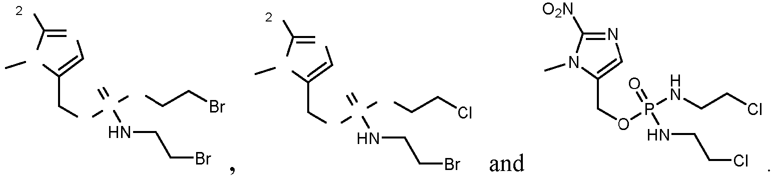

The chemical structures of particular examples of compounds of formulae (2) to (9) may be as defined in claim 7, and further description thereof is omitted here.

Reference can be made to claim 9 of Pat. App. No. PCT/CN2020/114519, published as Pub. No. WO2021120717A1, for particular examples of compounds of formula (2), and the entirety of this publication is hereby incorporated by reference.

Reference can be made to the table described in paragraphs [0073] to [0093] of Pat. App. No. CN2016800368985, published as Pub. No. CN108024974A, for particular examples of compounds of formula (3), and the entirety of this publication is hereby incorporated by reference.

Reference can be made to Pat. App. No. PCT/US2016/021581, published as Pub. No. WO2016145092A1 (or Chinese Pat. App. No. 2016800150788, published as Pub. No. CN107530556A), for particular examples of compounds of formula (4), and the entirety of this publication is hereby incorporated by reference.

Reference can be made to Pat. App. No. PCT/CN2020/089692, published as Pub. No. WO2020228685A1, for particular examples of compounds of formulae (5) and (6), and the entirety of the publication is hereby incorporated by reference.

Reference can be made to Pat. App. No. PCT/US2016/021581, published as Pub. No. WO2016145092A1 (or Chinese Pat. App. No. 2016800150788, published as Pub. No. CN107530556A) and Pat. App. No. PCT/CN2020/089692, published as Pub. No. WO2020228685A1, for particular examples of compounds of formula (7), and the entirety of the two publications is hereby incorporated by reference.

Reference can be made to Pat. App. No. PCT/CN2020/120281, published as Pub. No. WO2021068952A1, for particular examples of compounds of formula (8), and the entirety of the publication is hereby incorporated by reference.

Reference can be made to Pat. App. No. PCT/NZ2019/050030, published as WO2019190331A1 (or Chinese Pat. App. No. 2019800234236, published as Pub. No. CN111918864A) for particular examples of compounds of formula (9), and the entirety of the publication is hereby incorporated by reference.

### BRIEF DESCRIPTION OF THE DRAWINGS

Fig. 1 shows HT29 cell line proliferation inhibition curves of AST-3424 as monotherapy and in combination therapy with adavosertib.
Fig. 2 shows HT29 cell proliferation inhibition rates of AST-3424 and adavosertib separately as monotherapy and in combination therapy at selected concentrations (in each set of histogram bars, the left bar represents AST-3424 as monotherapy, the middle bar represents adavosertib as monotherapy, and the right bar represents AST-3424 and adavosertib in combination therapy).
Fig. 3 shows HT29 cell line proliferation inhibition curves of AST-3424 as monotherapy and in combination therapy with AZD7762.
Fig. 4 shows HT29 cell proliferation inhibition rates of AST-3424 and AZD7762 separately as monotherapy and in combination therapy at selected concentrations (in each set of histogram bars, the left bar represents AST-3424 as monotherapy, the middle bar represents AZD7762 as monotherapy, and the right bar represents AST-3424 and AZD7762 in combination therapy).
Fig. 5 shows HT29 cell line proliferation inhibition curves of AST-3424 as monotherapy and in combination therapy with palbociclib.
Fig. 6 shows HT29 cell proliferation inhibition rates of AST-3424 and palbociclib separately as monotherapy and in combination therapy at selected concentrations (in each set of histogram bars, the left bar represents AST-3424 as monotherapy, the middle bar represents palbociclib as monotherapy, and the right bar represents AST-3424 and palbociclib in combination therapy).
Fig. 7 shows H460 cell line proliferation inhibition curves of AST-3424 as monotherapy and in combination therapy with adavosertib.
Fig. 8 shows H460 cell proliferation inhibition rates of AST-3424 and adavosertib separately as monotherapy and in combination therapy at selected concentrations (in each set of histogram bars, the left bar represents AST-3424 as monotherapy, the middle bar represents adavosertib as monotherapy, and the right bar represents AST-3424 and adavosertib in combination therapy).
Fig. 9 shows H460 cell line proliferation inhibition curves of AST-3424 as monotherapy and in combination therapy with AZD7762.
Fig. 10 shows H460 cell proliferation inhibition rates of AST-3424 and AZD7762 separately as monotherapy and in combination therapy at selected concentrations (in each set of histogram bars, the left bar represents AST-3424 as monotherapy, the middle bar represents AZD7762 as monotherapy, and the right bar represents AST-3424 and AZD7762 in combination therapy).
Fig. 11 shows H460 cell line proliferation inhibition curves of AST-3424 as monotherapy and in combination therapy with palbociclib.
Fig. 12 shows H460 cell proliferation inhibition rates of AST-3424 and palbociclib separately as monotherapy and in combination therapy at selected concentrations (in each set of histogram bars, the left bar represents AST-3424 as monotherapy, the middle bar represents palbociclib as monotherapy, and the right bar represents AST-3424 and palbociclib in combination therapy).
Fig. 13 shows HT29 cell line proliferation inhibition curves of AST as monotherapy and in combination therapy with adavosertib.
Fig. 14 shows HT29 cell proliferation inhibition rates of AST and adavosertib separately as monotherapy and in combination therapy at selected concentrations (in each set of histogram bars, the left bar represents AST as monotherapy, the middle bar represents adavosertib as monotherapy, and the right bar represents AST and adavosertib in combination therapy).
Fig. 15 shows HT29 cell line proliferation inhibition curves of AST as monotherapy and in combination therapy with AZD7762.
Fig. 16 shows HT29 cell proliferation inhibition rates of AST and AZD7762 separately as monotherapy and in combination therapy at selected concentrations (in each set of histogram bars, the left bar represents AST as monotherapy, the middle bar represents AZD7762 as monotherapy, and the right bar represents AST and AZD7762 in combination therapy).
Fig. 17 shows HT29 cell line proliferation inhibition curves of AST as monotherapy and in combination therapy with palbociclib.
Fig. 18 shows HT29 cell proliferation inhibition rates of AST and palbociclib separately as monotherapy and in combination therapy at selected concentrations (in each set of histogram bars, the left bar represents AST as monotherapy, the middle bar represents palbociclib as monotherapy, and the right bar represents AST and palbociclib in combination therapy).
Fig. 19 shows H460 cell line proliferation inhibition curves of AST as monotherapy and in combination therapy with adavosertib.
Fig. 20 shows H460 cell proliferation inhibition rates of AST and adavosertib separately as monotherapy and in combination therapy at selected concentrations (in each set of histogram bars, the left bar represents AST as monotherapy, the middle bar represents adavosertib as monotherapy, and the right bar represents AST and adavosertib in combination therapy).
Fig. 21 shows H460 cell line proliferation inhibition curves of AST as monotherapy and in combination therapy with AZD7762.
Fig. 22 shows H460 cell proliferation inhibition rates of AST and AZD7762 separately as monotherapy and in combination therapy at selected concentrations (in each set of histogram bars, the left bar represents AST as monotherapy, the middle bar represents AZD7762 as monotherapy, and the right bar represents AST and AZD7762 in combination therapy).
Fig. 23 shows H460 cell line proliferation inhibition curves of AST as monotherapy and in combination therapy with palbociclib.
Fig. 24 shows H460 cell proliferation inhibition rates of AST and palbociclib separately as monotherapy and in combination therapy at selected concentrations (in each set of histogram bars, the left bar represents AST as monotherapy, the middle bar represents palbociclib as monotherapy, and the right bar represents AST and palbociclib in combination therapy).
Fig. 25 shows HT29/H460 cell line proliferation inhibition curves of AST-3424 as monotherapy and in combination therapy with ceralasertib, in which the lowermost curve corresponds to AST-3424 as monotherapy.
Fig. 26 shows HT29/H460 cell line proliferation inhibition curves of AST-3424 as monotherapy and in combination therapy with ceralasertib in another assay, in which the lowermost curve corresponds to AST-3424 as monotherapy.
Fig. 27 shows HT29 cell line proliferation inhibition curves of AST-3424 as monotherapy and in combination therapy with ceralasertib in an assay where ceralasertib and AST-3424 are added in different orders, in which the lowermost curve corresponds to AST-3424 as monotherapy.
Fig. 28 shows HT29 cell line proliferation inhibition curves of TH-302 as monotherapy and in combination therapy with adavosertib, in which "<0.01% O₂" corresponds to hypoxia, and the left uppermost curve corresponds to TH-302 in combination therapy with adavosertib at 10 µM under hypoxia.
Fig. 29 shows H460 cell line proliferation inhibition curves of TH-302 as monotherapy and in combination therapy with adavosertib, in which "<0.01% O₂" corresponds to hypoxia, and the left curves correspond to TH-302 in combination therapy with adavosertib at 30 µM under hypoxia.
Fig. 30 shows HT29 cell line proliferation inhibition curves of TH-302 as monotherapy and in combination therapy with ceralasertib, in which "<0.01% O₂" corresponds to hypoxia, and the left uppermost curve corresponds to TH-302 in combination therapy with ceralasertib at 2000 µM under hypoxia.
Fig. 31 shows H460 cell line proliferation inhibition curves of TH-302 as monotherapy and in combination therapy with ceralasertib, in which "<0.01% O₂" corresponds to hypoxia, and the right curves correspond to TH-302 as monotherapy under normoxia.

A table showing the Chinese explanations of the English terms as shown in the drawings

| No. | Term | Denotation |
|---|---|---|
| 1 | Inhibition | Inhibition rate |
| 2 | Pretreatment | Pretreatment |
| 3 | Cotreatment | Simultaneous treatment in combination therapy |
| 4 | conc.Log (nM) | The logarithm of a measured concentration in nmol/L to base 10 |

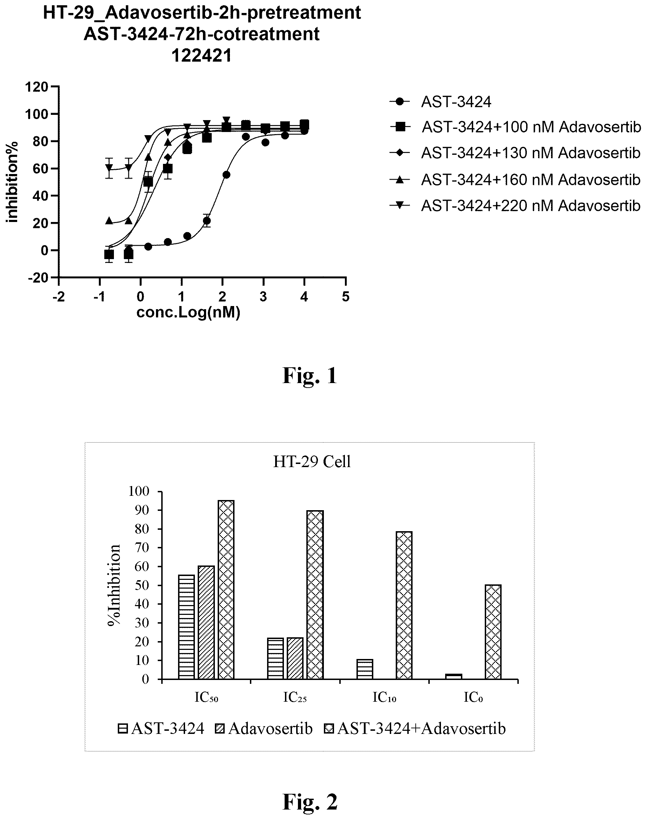

The titles of some figures describe conditions of corresponding assays. For example, the title of Fig. 1, "HT-29_Adavosertib-2h-pretreatment AST-3424-72h-cotreatment 122421" consists of the parts explained below.
"HT-29" indicates an assay carried out on a HT29 cell line;
"Adavosertib-2h-pretreatment AST-3424-72h-cotreatment" indicates treatment with AST-3424 in combination therapy with adavosertib, in which 2-h adavosertib treatment was followed by 72-h cotreatment of adavosertib and AST-3424, or 72-h treatment with AST-3424 as monotherapy;
"122421" represents the series number of the assay.

The other figures have similar titles, which, however may indicate different cell lines, test compounds and/or series numbers. Reference can be made to the above description for more details.

### Examples

AST-3424 has a structure represented by the following formula: 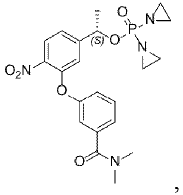 and its synthesis method is described in Pat. App. No. PCT/US2016/062114, published as Pub. No. WO2017087428A1 (or Chinese Pat. App. No. 2016800200132, published as Pub. No. CN108136214A).

AST has a structure represented by the following formula: 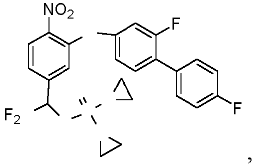 and its synthesis method is described in Pat. App. No. PCT/CN2020/089692, published as Pub. No. WO2020228685A1.

TH-302 has a structure represented by the following formula: 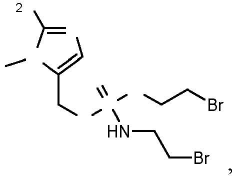 and its synthesis method is described in Pat. App. No. CN2012102515573, published as Pub. No. CN102746336A.

Adavosertib (also known as AZD1775 or MK1775) is a selective inhibitor of the Wee1 kinase, a crucial kinase for the G2/M cell cycle checkpoint and DNA damage repair. Cyclin-dependent kinases (CDKs) regulate the cell cycle by phosphorylating specific substrates, and their activity depends on that of the complexes resulting from their binding with cyclins. Moreover, their activity is regulated by phosphorylation, and the phosphorylation of CDK1 further controls the G2/M checkpoint. Wee1 acts like a "gatekeeper" of the G2/M cell cycle checkpoint to allow cells to undergo DNA damage repair before progressing to the mitosis phase. Wee1 maintains CDK1 inactive by phosphorylating Tyr15 in CDK1, thereby inhibiting the activity of the CDK1-cyclin B complex. In this way, it arrests cell division at the G2/M checkpoint and negatively regulates the cell cycle. Its biological significance is to repair DNA damage that failed to be repaired in time to prevent cells from progressing to the mitosis phase with such DNA damage. Adavosertib can arrest p53 or other gene-deficient tumors at the DNA damage repair stage at the G2/M checkpoint by selectively inhibiting the Wee1kinase. This will ultimately cause tumor cell death and achieve tumor treatment. Adavosertib has a structure represented by the following formula: 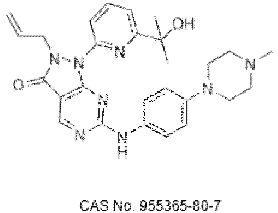 and can be directly purchased from commercial reagent suppliers.

AZD7762 is an effective ATP-competitive inhibitor of cell cycle checkpoint kinases (CHKs). This CHK inhibitor can eliminate DNA damage-induced S and G2 checkpoints and has a structure represented by the following formula: 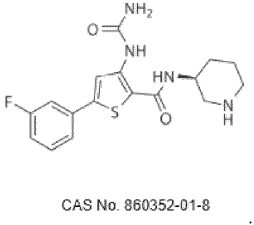 AZD7762 can be directly purchased from commercial reagent suppliers.

Palbociclib is an orally active selective inhibitor of CDK4 and CDK6 and has a structure represented by the following formula: 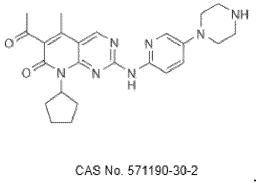 It can be directly purchased from commercial reagent suppliers.

Ceralasertib (AZD6738) is an effective inhibitor of the ATR kinase, which controls the G2/M checkpoint. It has a structure represented by the following formula: 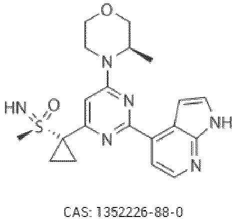 and can be directly purchased from commercial reagent suppliers.

### I. In Vitro Cell Assays

CTG cell proliferation assays were performed on wild-type p53-expressing (H460) and p53-deficient (HT29) cell lines to test whether cell cycle inhibitors could enhance in vitro cytotoxicity of the DNA alkylating agent prodrugs AST, AST-3424 AND TH-302 by inhibiting major DNA repair checkpoints in the cell cycle of tumor cells.

Each CTG cell proliferation assay included the steps as follows:
1) An H460 or HT29 cell suspension was added to a 96-well plate at 100 µL per well so that each well contained 2000 or 10000 cells.
2) The cells were incubated overnight at 37 °C in an incubator with 5% CO₂.
3) Treatment with Compounds

Combination therapy: 1µL of adavosertib (at four concentrations), AZD7762 (at four concentrations), palbociclib (at four concentrations) or ceralasertib (at three concentrations) was added to each well and shaken gently until homogeneous mixing. After incubation for 2 hours, 1 µL of the test compound AST-3424 or AST was added at various concentrations, followed by gentle shaking until homogeneous mixing and incubation at 37 °C in an incubator with 5% CO₂.

Monotherapy: After the cells were plated for 24 hours, 99 µL of growth medium was added to each well, as well as 1 µL of the test compound AST-3424 or AST at various concentrations, followed by gentle shaking until homogeneous mixing and incubation at 37 °C in an incubator with 5% CO₂.
4) The cells were incubated for 72 hours.
5) The cells were equilibrated at room temperature for 30 min, and 100 µL of medium was discarded from each well.
6) After a CTG reagent was added to each well at an amount of 25 µL, the well plate was shaken on a rapid shaker for 2 min and placed in dark at room temperature for 30 min.
7) Chemiluminescence was read for 1000 ms on a multimode microplate reader.
8) IC₅₀ (half-maximal inhibitory concentration) values of the compounds were calculated using the GraphPad Prism 5 software.

### 1. Results of HT29 Cell Line Proliferation Inhibition Assay of AST-3424 as Monotherapy and in Combination Therapy with Adavosertib

HT29 cell line proliferation inhibition IC₅₀ values of AST-3424 as monotherapy and in combination therapy with adavosertib were derived by fitting the numerical results of the proliferation inhibition assay at different concentrations shown in Fig. 1 and summarized in Table 1. The IC₅₀ values of AST-3424 in combination therapy with adavosertib at different concentrations were significantly lower than those of AST-3424 as monotherapy and showed a certain degree of adavosertib dose dependence.

**Table 1: HT29 Cell Line Proliferation Inhibition IC₅₀ Values of AST-3424 as Monotherapy and in Combination Therapy with Adavosertib**

| **Compounds** | **IC₅₀ (nM)** | **Ratio single/combo** |
|---|---|---|
| **AST-3424** | **84.75** | **/** |
| **AST-3424 + 100 nM adavosertib** | **2.11** | **40.17** |
| **AST-3424 + 130 nM adavosertib** | **1.52** | **55.39** |
| **AST-3424 + 160 nM adavosertib** | **1.20** | **70.63** |
| **AST-3424 + 220 nM adavosertib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with adavosertib. | | |

From further statistical analysis of the data in Fig. 1, we obtained inhibition rates of AST-3424 and adavosertib in combination therapy at several selected concentrations, at which AST-3424 and adavosertib separately as monotherapy achieved about 50% (IC₅₀), about 25% (IC₂₅), about 10% (IC₁₀) and about 0% (IC₀) inhibition of HT29 cells. The results are shown in Fig. 2.

Conclusions: the above results demonstrated that, in the p53-deficient (HT29) cell line, when AST-3424 at various concentrations were respectively used in combination therapy with the cell cycle inhibitor adavosertib at various concentrations, they exhibited significant additive cytotoxic effects, and such additive cytotoxic effects were still significant even when both AST-3424 and the cell cycle inhibitor adavosertib were used in combination therapy at low concentrations.

### 2. Results of HT29 Cell Line Proliferation Inhibition Assay of AST-3424 as Monotherapy and in Combination Therapy with AZD7762

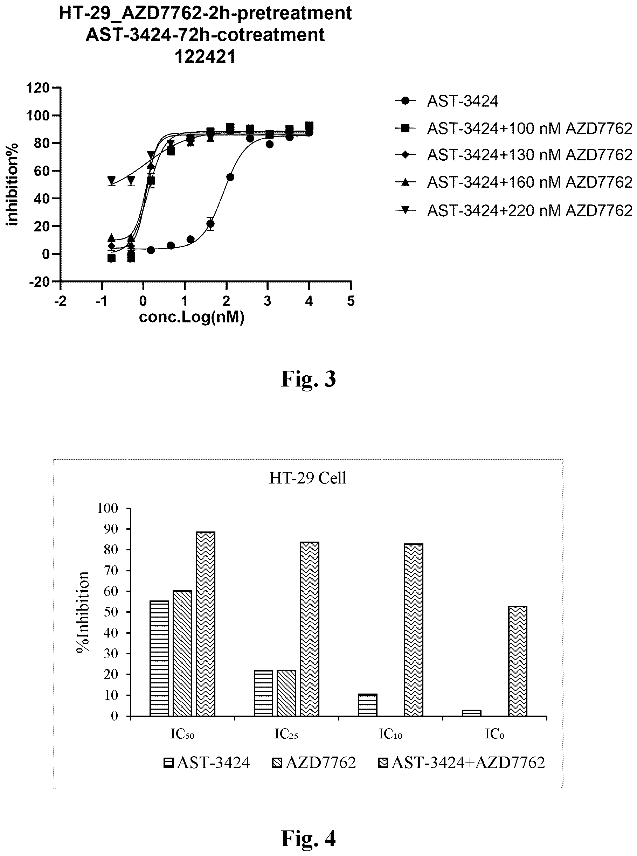

HT29 cell line proliferation inhibition IC₅₀ values of AST-3424 as monotherapy and in combination therapy with AZD7762 were derived by fitting the numerical results of the proliferation inhibition assay at different concentrations shown in Fig. 3 and summarized in Table 2. The IC₅₀ values of AST-3424 in combination therapy with AZD7762 at different concentrations were significantly lower than those of AST-3424 as monotherapy and showed a certain degree of AZD7762 dose dependence.

**Table 2: HT29 Cell Line Proliferation Inhibition IC₅₀ Values of AST-3424 as Monotherapy and in Combination Therapy with AZD7762**

| **Compounds** | **IC₅₀ (nM)** | **Ratio single/combo** |
|---|---|---|
| **AST-3424** | **84.75** | **/** |
| **AST-3424 + 100 nM AZD7762** | **1.29** | **65.70** |
| **AST-3424 + 130 nM AZD7762** | **1.20** | **70.63** |
| **AST-3424 + 160 nM AZD7762** | **1.18** | **71.82** |
| **AST-3424 + 220 nM AZD7762** | **NA** | **NA** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with AZD7762. | | |

From further statistical analysis of the data in Fig. 3, we obtained inhibition rates of AST-3424 and AZD7762 in combination therapy at several selected concentrations, at which AST-3424 and AZD7762 separately as monotherapy achieved about 50% (IC₅₀), about 25% (IC₂₅), about 10% (IC₁₀) and about 0% (IC₀) inhibition of HT29 cells. The results are shown in Fig. 4.

Conclusions: the above results demonstrated that, in the p53-deficient (HT29) cell line, when AST-3424 at various concentrations were respectively used in combination therapy with the cell cycle inhibitor AZD7762 at various concentrations, they exhibited significant additive cytotoxic effects, and such additive cytotoxic effects were still significant even when both AST-3424 and the cell cycle inhibitor AZD7762 were used in combination therapy at low concentrations.

### 3. Results of HT29 Cell Line Proliferation Inhibition Assay of AST-3424 as Monotherapy and in Combination Therapy with Palbociclib

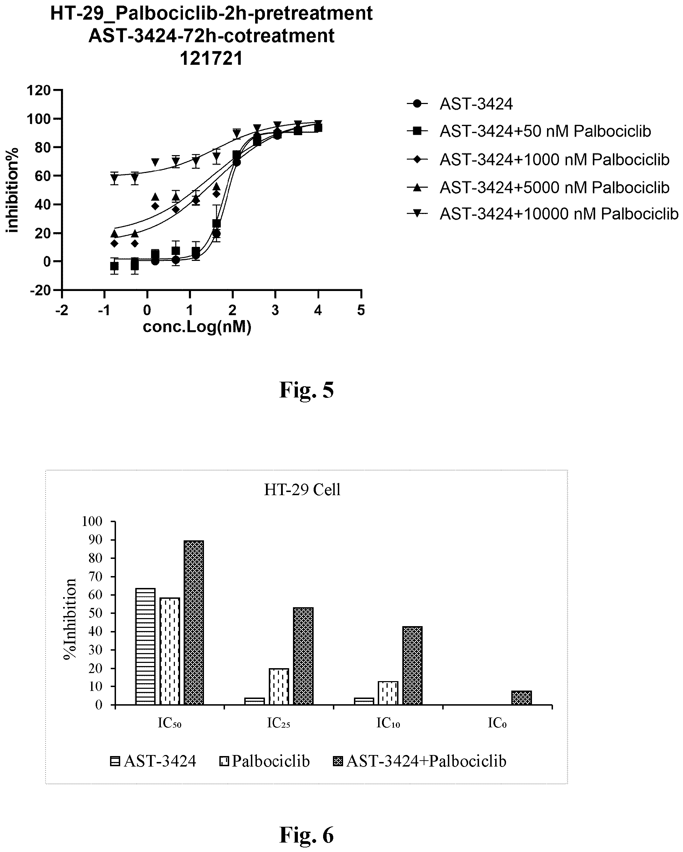

HT29 cell line proliferation inhibition IC₅₀ values of AST-3424 as monotherapy and in combination therapy with palbociclib were derived by fitting the assay results of Fig. 5 and summarized in Table 3. The IC₅₀ values of AST-3424 in combination therapy with palbociclib at different concentrations were lower than those of AST-3424 as monotherapy and showed a certain degree of palbociclib dose dependence.

**Table 3: HT29 Cell Line Proliferation Inhibition IC₅₀ Values of AST-3424 as Monotherapy and in Combination Therapy with Palbociclib**

| **Compounds** | **IC₅₀ (nM)** | **Ratio single/combo** |
|---|---|---|
| **AST-3424** | **73.39** | **/** |
| **AST-3424 + 50 nM palbociclib** | **62.50** | **1.17** |
| **AST-3424 + 1000 nM palbociclib** | **38.13** | **1.92** |
| **AST-3424 + 5000 nM palbociclib** | **31.35** | **2.34** |
| **AST-3424 + 10000 nM palbociclib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with palbociclib. | | |

From further statistical analysis of the data in Fig. 5, we obtained inhibition rates of AST-3424 and palbociclib in combination therapy at several selected concentrations, at which AST-3424 and palbociclib separately as monotherapy achieved about 50% (IC₅₀), about 25% (IC₂₅), about 10% (IC₁₀) and about 0% (IC₀) inhibition of HT29 cells. The results are shown in Fig. 6.

Conclusions: the above results demonstrated that, in the p53-deficient (HT29) cell line, when AST-3424 at various concentrations were respectively used in combination therapy with the cell cycle inhibitor palbociclib at various concentrations, they exhibited significant additive cytotoxic effects, and such additive cytotoxic effects were still significant even when both AST-3424 and the cell cycle inhibitor palbociclib were used in combination therapy at low concentrations.

### 4. Results of H460 Cell Line Proliferation Inhibition Assay of AST-3424 as Monotherapy and in Combination Therapy with Adavosertib

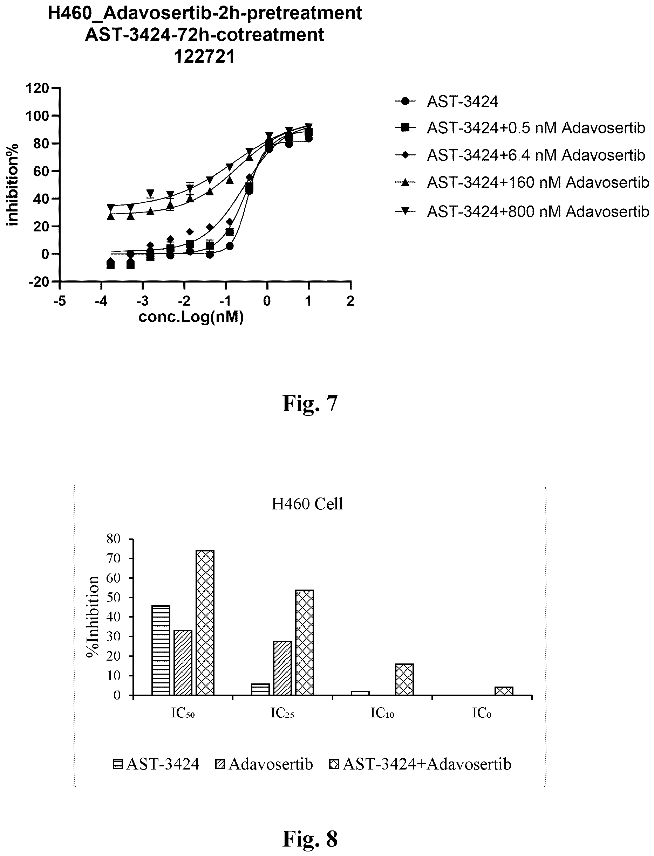

H460 cell line proliferation inhibition IC₅₀ values of AST-3424 as monotherapy and in combination therapy with adavosertib were derived by fitting the assay results of Fig. 7 and summarized in Table 4. The IC₅₀ values of AST-3424 in combination therapy with adavosertib at high concentrations were approximately twice higher those of AST-3424 as monotherapy. However, the IC₅₀ values of AST-3424 in combination therapy with adavosertib at other concentrations did not significantly differ from those of AST-3424 as monotherapy.

**Table 4: H460 Cell Line Proliferation Inhibition IC₅₀ Values of AST-3424 as Monotherapy and in Combination Therapy with Adavosertib**

| **Compounds** | **IC₅₀ (nM)** | **Ratio single/combo** |
|---|---|---|
| **AST-3424** | **0.34** | **/** |
| **AST-3424 + 0.5 nM adavosertib** | **0.31** | **1.10** |
| **AST-3424 + 6.4 nM adavosertib** | **0.27** | **1.26** |
| **AST-3424 + 160 nM adavosertib** | **0.19** | **1.79** |
| **AST-3424 + 800 nM adavosertib** | **0.15** | **2.27** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with adavosertib. | | |

From further statistical analysis of the data in Fig. 7, we obtained inhibition rates of AST-3424 and adavosertib in combination therapy at several selected concentrations, at which AST-3424 and adavosertib separately as monotherapy achieved about 50% (IC₅₀), about 25% (IC₂₅), about 10% (IC₁₀) and about 0% (IC₀) inhibition of H460 cells. The results are shown in Fig. 8.

Conclusions: the above results demonstrated that, in the wild-type p53-expressing (H460) cell line, when AST-3424 at various concentrations were respectively used in combination therapy with the cell cycle inhibitor adavosertib at various concentrations, the compounds used in combination therapy at high concentrations exhibited certain additive cytotoxic effects. However, such additive cytotoxic effects were not significant when both AST-3424 and the cell cycle inhibitor adavosertib were used in combination therapy at low concentrations.

### 5. Results of H460 Cell Line Proliferation Inhibition Assay of AST-3424 as Monotherapy and in Combination Therapy with AZD7762

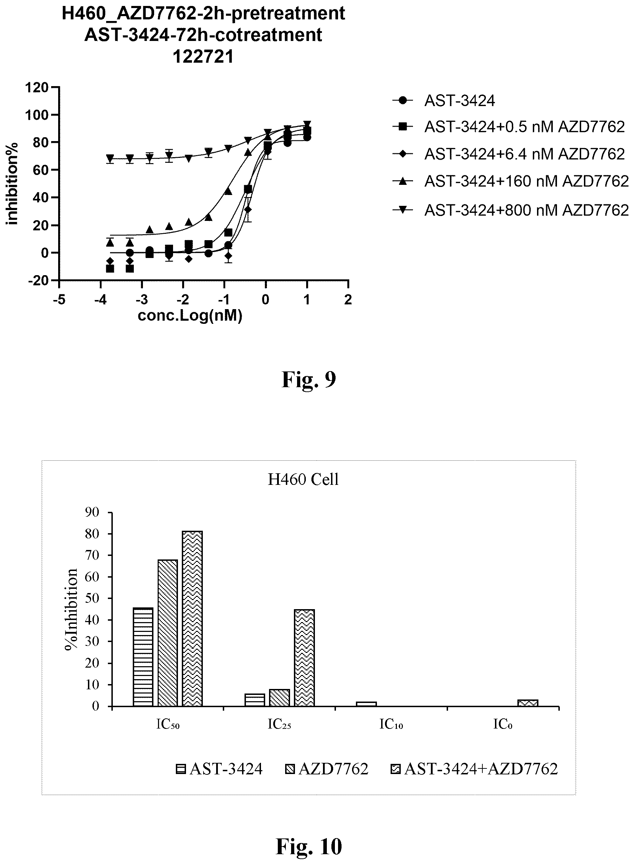

H460 cell line proliferation inhibition IC₅₀ values of AST-3424 as monotherapy and in combination therapy with AZD7762 were derived by fitting the assay results of Fig. 9 and summarized in Table 5. The IC₅₀ values of AST-3424 in combination therapy with AZD7762 at 160 nM were approximately twice those of AST-3424 as monotherapy. However, the IC₅₀ values of AST-3424 in combination therapy with AZD7762 at other concentrations did not significantly differ from those of AST-3424 as monotherapy.

**Table 5: H460 Cell Line Proliferation Inhibition IC₅₀ Values of AST-3424 as Monotherapy and in Combination Therapy with AZD7762**

| **Compounds** | **IC₅₀ (nM)** | **Ratio single/combo** |
|---|---|---|
| **AST-3424** | **0.34** | **/** |
| **AST-3424 + 0.5 nM AZD7762** | **0.33** | **1.03** |
| **AST-3424 + 6.4 nM AZD7762** | **0.47** | **0.73** |
| **AST-3424 + 160 nM AZD7762** | **0.16** | **2.13** |
| **AST-3424 + 800 nM AZD7762** | **NA** | **NA** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with AZD7762. | | |

From further statistical analysis of the data in Fig. 9, we obtained inhibition rates of AST-3424 and AZD7762 in combination therapy at several selected concentrations, at which AST-3424 and AZD7762 separately as monotherapy achieved about 50% (IC₅₀), about 25% (IC₂₅), about 10% (IC₁₀) and about 0% (IC₀) inhibition of H460 cells. The results are shown in Fig. 10.

Conclusions: the above results demonstrated that, in the wild-type p53-expressing (H460) cell line, when AST-3424 at various concentrations were respectively used in combination therapy with the cell cycle inhibitor AZD7762 at various concentrations, the compounds used in combination therapy at medium and high concentrations exhibited certain additive cytotoxic effects. However, such additive cytotoxic effects were not significant when both AST-3424 and the cell cycle inhibitor AZD7762 were used in combination therapy at low concentrations.

### 6. Results of H460 Cell Line Proliferation Inhibition Assay of AST-3424 as Monotherapy and in Combination Therapy with Palbociclib

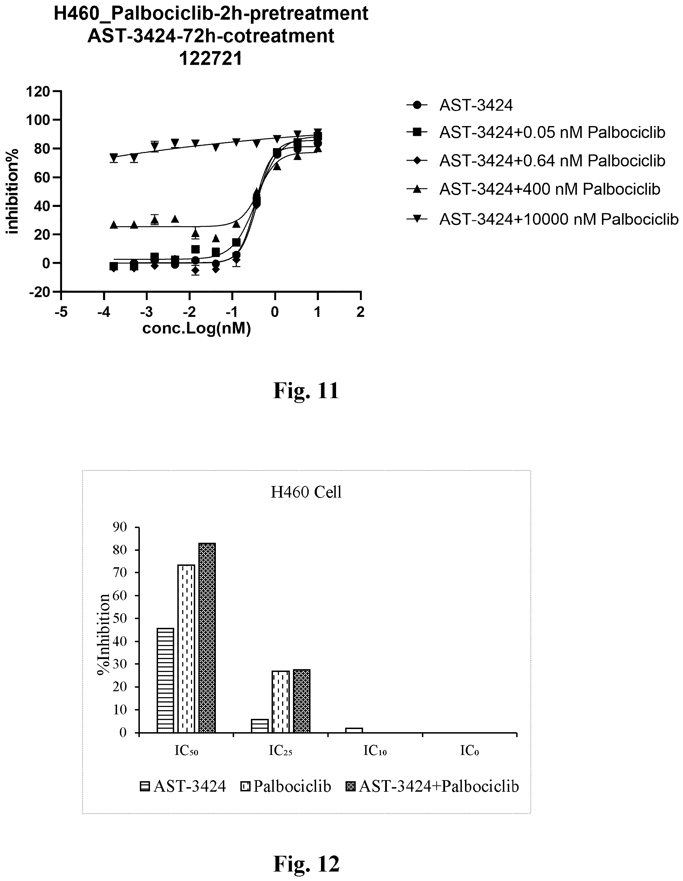

H460 cell line proliferation inhibition IC₅₀ values of AST-3424 as monotherapy and in combination therapy with palbociclib were derived by fitting the assay results of Fig. 11 and summarized in Table 6. The IC₅₀ values of AST-3424 in combination therapy with palbociclib at various concentrations did not significantly differ from those of AST-3424 as monotherapy.

**Table 6: H460 Cell Line Proliferation Inhibition IC₅₀ Values of AST-3424 as Monotherapy and in Combination Therapy with Palbociclib**

| **Compounds** | **IC₅₀ (nM)** | **Ratio Combo/single** |
|---|---|---|
| **AST-3424** | **0.34** | **/** |
| **AST-3424 + 0.05 nM palbociclib** | **0.38** | **1.12** |
| **AST-3424 + 0.64 nM palbociclib** | **0.38** | **1.12** |
| **AST-3424 + 400 nM palbociclib** | **0.42** | **1.24** |
| **AST-3424 + 10000 nM palbociclib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with palbociclib. | | |

From further statistical analysis of the data in Fig. 11, we obtained inhibition rates of AST-3424 and palbociclib in combination therapy at several selected concentrations, at which AST-3424 and palbociclib separately as monotherapy achieved about 50% (IC₅₀), about 25% (IC₂₅), about 10% (IC₁₀) and about 0% (IC₀) inhibition of H460 cells. The results are shown in Fig. 12.

Conclusions: the above results demonstrated that, in the wild-type p53-expressing (H460) cell line, when AST-3424 at various concentrations were respectively used in combination therapy with the cell cycle inhibitor palbociclib at various concentrations, the compounds used in combination therapy at all concentrations did not exhibit any additive cytotoxic effects.

### 7. Results of HT29 Cell Line Proliferation Inhibition Assay of AST as Monotherapy and in Combination Therapy with Adavosertib

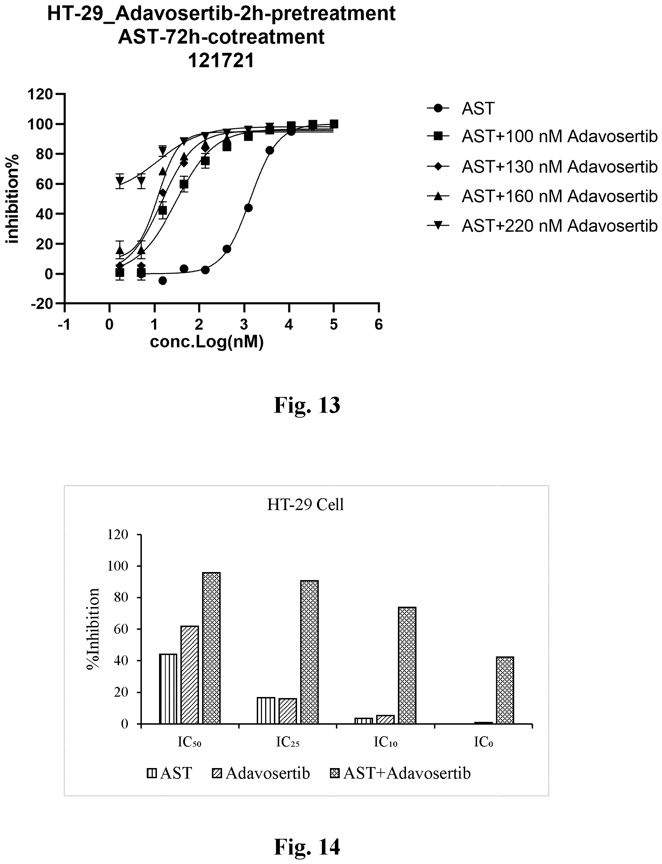

HT29 cell line proliferation inhibition IC₅₀ values of AST as monotherapy and in combination therapy with adavosertib were derived by fitting the assay results of Fig. 13 and summarized in Table 7. The IC₅₀ values of AST in combination therapy with adavosertib at different concentrations were significantly lower than those of AST as monotherapy and showed a certain degree of adavosertib dose dependence.

**Table 7: HT29 Cell Line Proliferation Inhibition IC₅₀ Values of AST as Monotherapy and in Combination Therapy with Adavosertib**

| **Compounds** | **IC₅₀ (nM)** | **Ratio single/combo** |
|---|---|---|
| **AST** | **1372.00** | **/** |
| **AST + 100 nM adavosertib** | **30.51** | **44.97** |
| **AST + 130 nM adavosertib** | **14.36** | **95.54** |
| **AST + 160 nM adavosertib** | **12.01** | **114.24** |
| **AST + 220 nM adavosertib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with adavosertib. | | |

From further statistical analysis of the data in Fig. 13, we obtained inhibition rates of AST and adavosertib in combination therapy at several selected concentrations, at which AST and adavosertib separately as monotherapy achieved about 50% (IC₅₀), about 25% (IC₂₅), about 10% (IC₁₀) and about 0% (IC₀) inhibition of HT29 cells. The results are shown in Fig. 14.

Conclusions: the above results demonstrated that, in the p53-deficient (HT29) cell line, when AST at various concentrations were respectively used in combination therapy with the cell cycle inhibitor adavosertib at various concentrations, all of them exhibited significant additive cytotoxic effects, and such additive cytotoxic effects were still significant even when both AST and the cell cycle inhibitor adavosertib were used in combination therapy at low concentrations.

### 8. Results of HT29 Cell Line Proliferation Inhibition Assay of AST as Monotherapy and in Combination Therapy with AZD7762

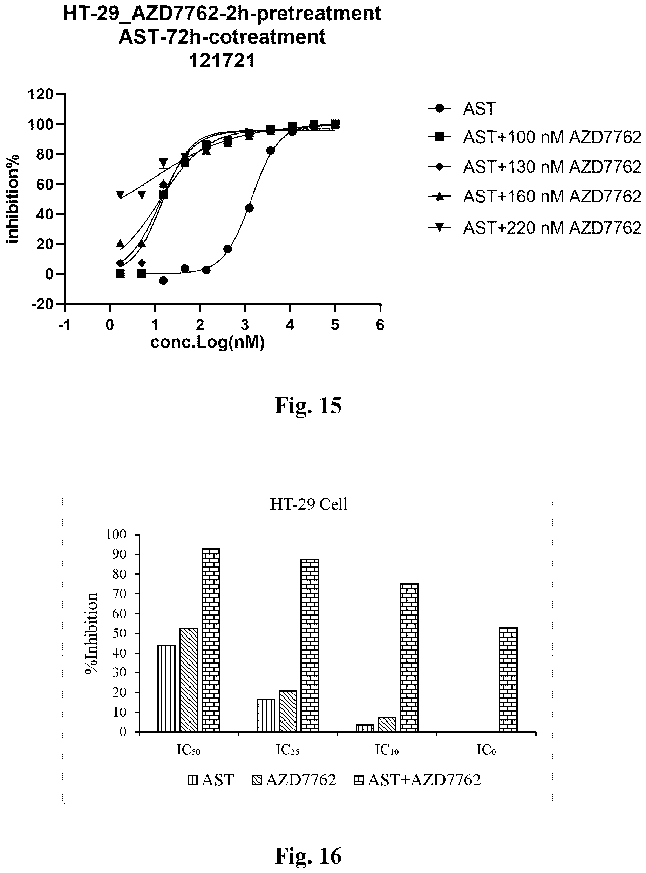

HT29 cell line proliferation inhibition IC₅₀ values of AST as monotherapy and in combination therapy with AZD7762 were derived by fitting the assay results of Fig. 15 and summarized in Table 8. The IC₅₀ values of AST in combination therapy with AZD7762 at different concentrations were significantly lower than those of AST as monotherapy.

**Table 8: HT29 Cell Line Proliferation Inhibition IC₅₀ Values of AST as Monotherapy and in Combination Therapy with AZD7762**

| **Compounds** | **IC₅₀ (nM)** | **Ratio single/combo** |
|---|---|---|
| **AST** | **1372.00** | **/** |
| **AST + 100 nM AZD7762** | **14.35** | **95.61** |
| **AST + 130 nM AZD7762** | **12.98** | **105.70** |
| **AST + 160 nM AZD7762** | **12.73** | **107.78** |
| **AST + 220 nM AZD7762** | **NA** | **NA** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with AZD7762. | | |

From further statistical analysis of the data in Fig. 15, we obtained inhibition rates of AST and AZD7762 in combination therapy at several selected concentrations, at which AST and AZD7762 separately as monotherapy achieved about 50% (IC₅₀), about 25% (IC₂₅), about 10% (IC₁₀) and about 0% (IC₀) inhibition of HT29 cells. The results are shown in Fig. 16.

Conclusions: the above results demonstrated that, in the p53-deficient (HT29) cell line, when AST at various concentrations were respectively used in combination therapy with the cell cycle inhibitor AZD7762 at various concentrations, all of them exhibited significant additive cytotoxic effects, and such additive cytotoxic effects were still significant even when both AST and the cell cycle inhibitor AZD7762 were used in combination therapy at low concentrations.

### 9. Results of HT29 Cell Line Proliferation Inhibition Assay of AST as Monotherapy and in Combination Therapy with Palbociclib

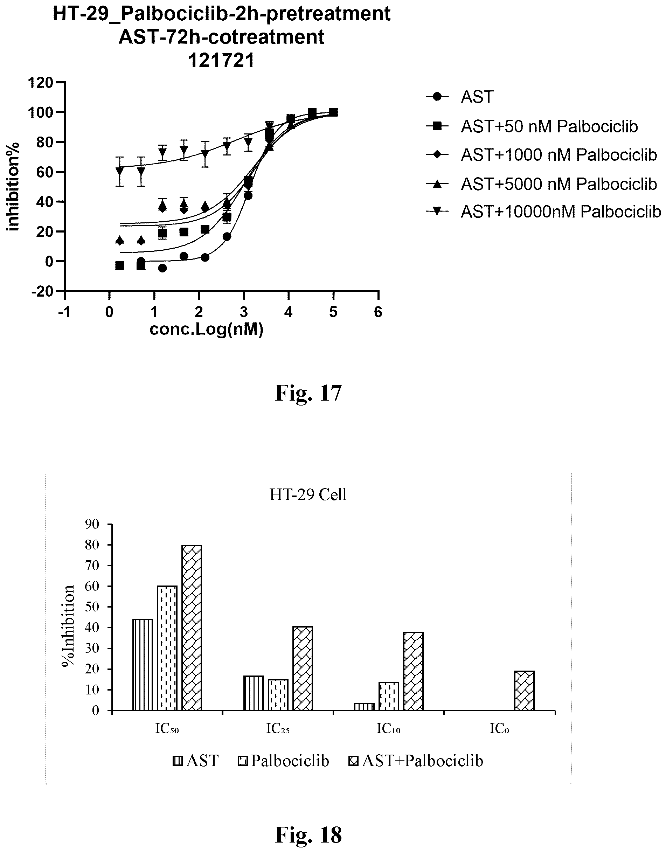

HT29 cell line proliferation inhibition IC₅₀ values of AST as monotherapy and in combination therapy with palbociclib were derived by fitting the assay results of Fig. 17 and summarized in Table 9. The IC₅₀ values of AST in combination therapy with palbociclib at different concentrations did not significantly differ from those of AST as monotherapy.

**Table 9: HT29 Cell Line Proliferation Inhibition IC₅₀ Values of AST as Monotherapy and in Combination Therapy with Palbociclib**

| **Compounds** | **IC₅₀ (nM)** | **Ratio combo/single** |
|---|---|---|
| **AST** | **1372.00** | **/** |
| **AST + 50 nM palbociclib** | **1056.00** | **0.77** |
| **AST + 1000 nM palbociclib** | **1739.00** | **1.27** |
| **AST + 5000 nM palbociclib** | **1570.00** | **1.14** |
| **AST + 10000nM palbociclib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with palbociclib. | | |

From further statistical analysis of the data in Fig. 17, we obtained inhibition rates of AST and palbociclib in combination therapy at several selected concentrations, at which AST and palbociclib separately as monotherapy achieved about 50% (IC₅₀), about 25% (IC₂₅), about 10% (IC₁₀) and about 0% (IC₀) inhibition of HT29 cells. The results are shown in Fig. 18.

Conclusions: the above results demonstrated that, in the p53-deficient (HT29) cell line, when AST at various concentrations were respectively used in combination therapy with the cell cycle inhibitor palbociclib at various concentrations, they exhibited certain additive cytotoxic effects when the drugs were used in combination therapy at low concentrations.

### 10. Results of H460 Cell Line Proliferation Inhibition Assay of AST as Monotherapy and in Combination Therapy with Adavosertib

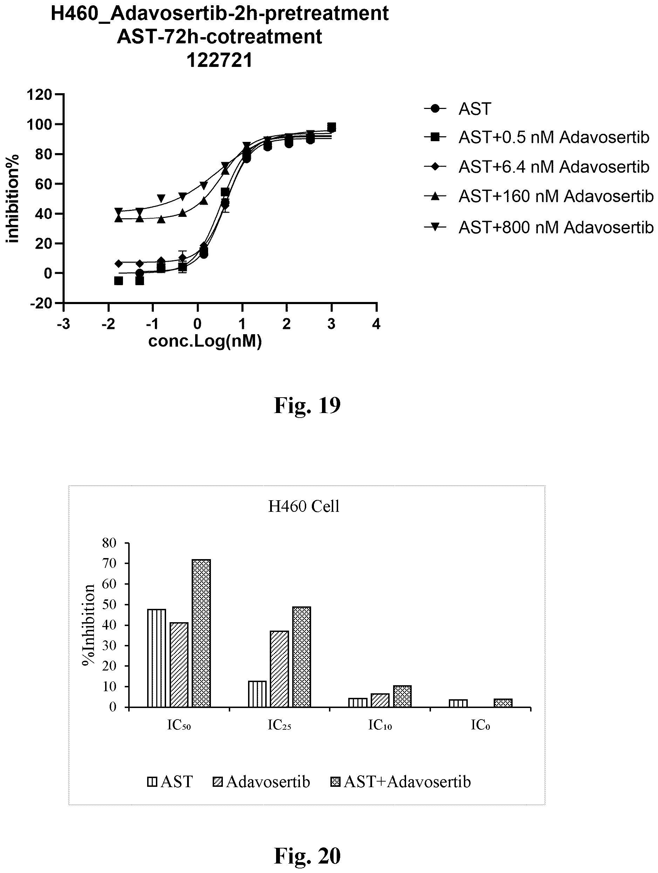

H460 cell line proliferation inhibition IC₅₀ values of AST as monotherapy and in combination therapy with adavosertib were derived by fitting the assay results of Fig. 19 and summarized in Table 10. The IC₅₀ values of AST in combination therapy with adavosertib at different concentrations did not significantly differ from those of AST as monotherapy.

**Table 10: H460 Cell Line Proliferation Inhibition IC₅₀ Values of AST as Monotherapy and in Combination Therapy with Adavosertib**

| **Compounds** | **IC₅₀ (nM)** | **Ratio single/combo** |
|---|---|---|
| **AST** | **4.08** | **/** |
| **AST + 0.5 nM adavosertib** | **3.29** | **1.24** |
| **AST + 6.4 nM adavosertib** | **4.53** | **0.90** |
| **AST + 160 nM adavosertib** | **3.44** | **1.19** |
| **AST + 800 nM adavosertib** | **2.89** | **1.41** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with adavosertib. | | |

From further statistical analysis of the data in Fig. 19, we obtained inhibition rates of AST and adavosertib in combination therapy at several selected concentrations, at which AST and adavosertib separately as monotherapy achieved about 50% (IC₅₀), about 25% (IC₂₅), about 10% (IC₁₀) and about 0% (IC₀) inhibition of H460 cells. The results are shown in Fig. 20.

Conclusions: the above results demonstrated that, in the wild-type p53-expressing (H460) cell line, when AST at various concentrations were respectively used in combination therapy with the cell cycle inhibitor adavosertib, all of them did not exhibit any additive toxic effects.

### 11. Results of H460 Cell Line Proliferation Inhibition Assay of AST as Monotherapy and in Combination Therapy with AZD7762

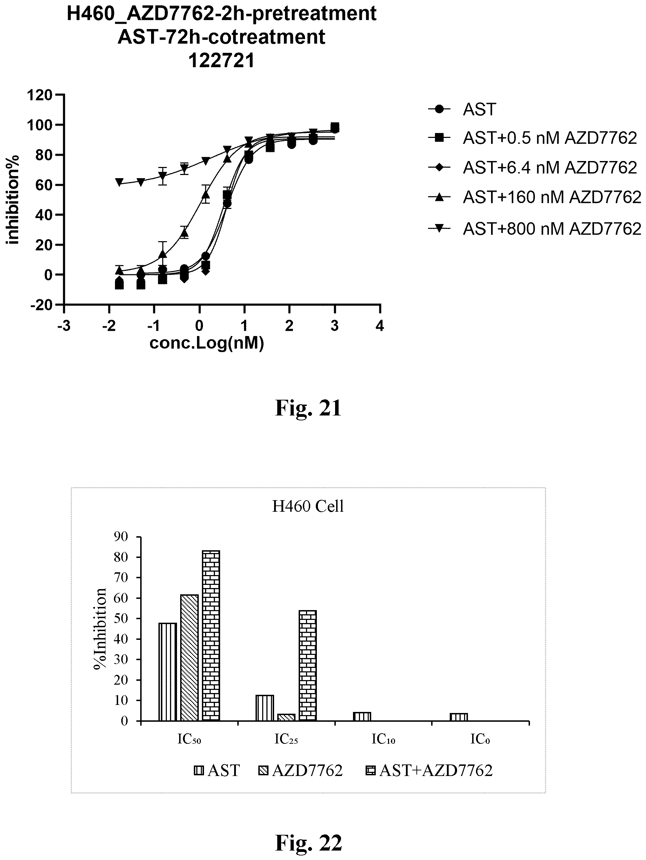

H460 cell line proliferation inhibition IC₅₀ values of AST as monotherapy and in combination therapy with AZD7762 were derived by fitting the assay results of Fig. 21 and summarized in Table 11. The IC₅₀ values of AST in combination therapy with AZD7762 at 160 nM were approximately thrice higher those of AST as monotherapy. However, the IC₅₀ values of AST in combination therapy with AZD7762 at other concentrations did not significantly differ from those of AST as monotherapy.

**Table 11: H460 Cell Line Proliferation Inhibition IC₅₀ Values of AST as Monotherapy and in Combination Therapy with AZD7762**

| **Compounds** | **IC₅₀ (nM)** | **Ratio single/combo** |
|---|---|---|
| **AST** | **4.08** | **/** |
| **AST + 0.5 nM AZD7762** | **3.40** | **1.20** |
| **AST + 6.4 nM AZD7762** | **4.08** | **1.00** |
| **AST + 160 nM AZD7762** | **1.07** | **3.81** |
| **AST + 800 nM AZD7762** | **NA** | **NA** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with AZD7762. | | |

From further statistical analysis of the data in Fig. 21, we obtained inhibition rates of AST and AZD7762 in combination therapy at several selected concentrations, at which AST and AZD7762 separately as monotherapy achieved about 50% (IC₅₀), about 25% (IC₂₅), about 10% (IC₁₀) and about 0% (IC₀) inhibition of H460 cells. The results are shown in Fig. 22.

Conclusions: the above results demonstrated that, in the wild-type p53-expressing (H460) cell line, when AST at various concentrations were respectively used in combination therapy with the cell cycle inhibitor AZD7762 at various concentrations, they exhibited certain additive cytotoxic effects when the concentration of the cell cycle inhibitor AZD7762 was160 nM, However, when the drugs were used in combination therapy at other concentrations, they did not exhibit any additive cytotoxic effects.

### 12. Results of H460 Cell Line Proliferation Inhibition Assay of AST as Monotherapy and in Combination Therapy with Palbociclib

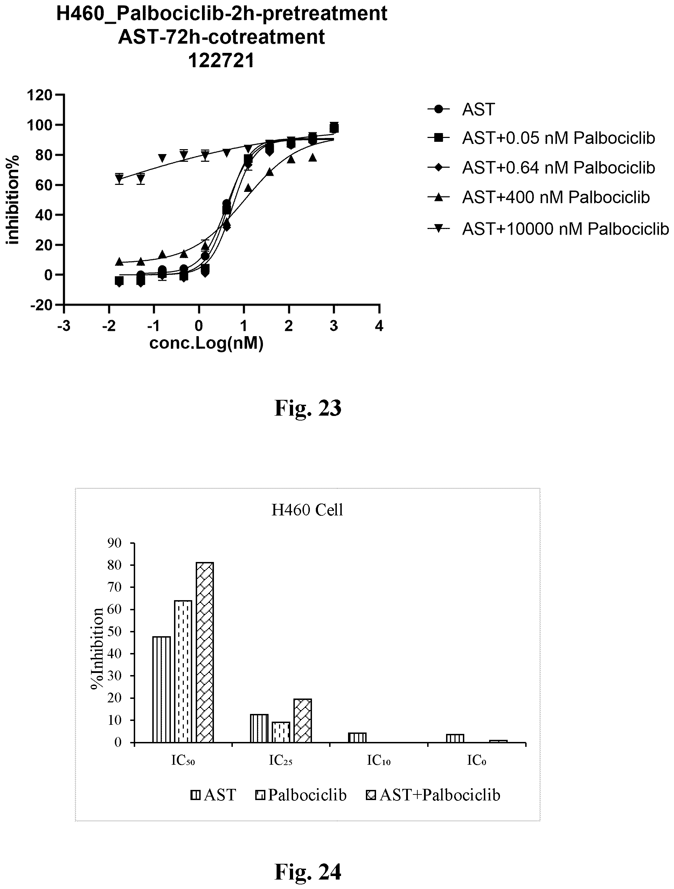

H460 cell line proliferation inhibition IC₅₀ values of AST as monotherapy and in combination therapy with palbociclib were derived by fitting the assay results of Fig. 23 and summarized in Table 12. The IC₅₀ values of AST in combination therapy with palbociclib at various concentrations did not significantly differ from, and were higher than, those of AST as monotherapy.

**Table 12: H460 Cell Line Proliferation Inhibition IC₅₀ Values of AST as Monotherapy and in Combination Therapy with Palbociclib**

| **Compounds** | **IC₅₀ (nM)** | **Ratio combo/single** |
|---|---|---|
| **AST** | **4.08** | **/** |
| **AST + 0.05 nM palbociclib** | **4.39** | **1.08** |
| **AST + 0.64 nM palbociclib** | ***5.50*** | **1.35** |
| **AST + 400 nM palbociclib** | **10.41** | **2.55** |
| **AST + 10000 nM palbociclib** | **NA** | **NA** |

| | | |
|---|---|---|
| Notes: the "Compounds" column lists the compounds as monotherapy and in combination therapy; and the "Ratio, single/combo" column lists the IC₅₀ ratios of AST as monotherapy to AST in combination therapy with palbociclib. | | |

From further statistical analysis of the data in Fig. 23, we obtained inhibition rates of AST and palbociclib in combination therapy at several selected concentrations, at which AST and palbociclib separately as monotherapy achieved about 50% (IC₅₀), about 25% (IC₂₅), about 10% (IC₁₀) and about 0% (IC₀) inhibition of H460 cells. The results are shown in Fig. 24.

Conclusions: the above results demonstrated that, in the wild-type p53-expressing (H460) cell line, when AST at various concentrations were respectively used in combination therapy with the cell cycle inhibitor palbociclib, all of them did not exhibit any additive cytotoxic effects.

### 13. Results of HT29/H460 Cell Line Proliferation Inhibition Assays of AST-3424 as Monotherapy and in Combination Therapy with Ceralasertib

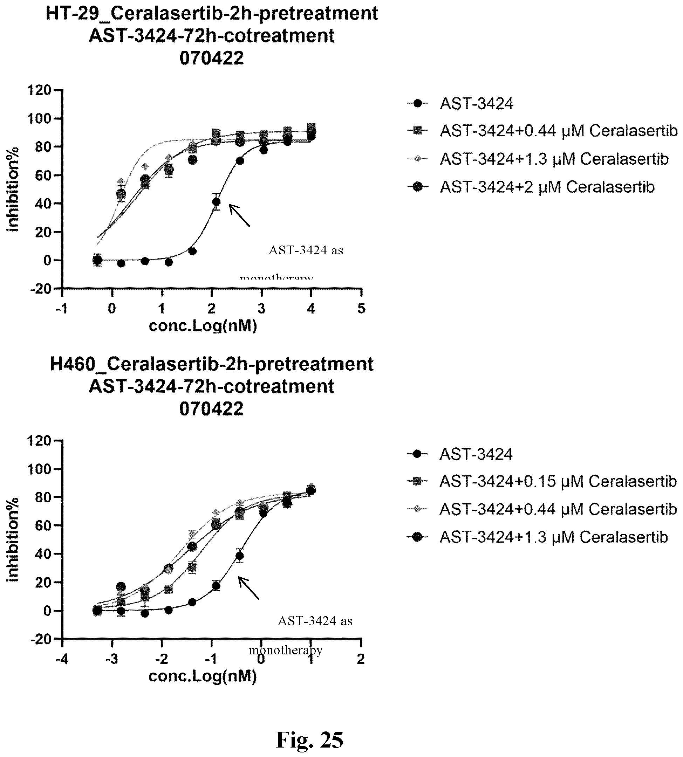

In a first assay, the HT29/H460 cell line proliferation inhibition curves of AST-3424 as monotherapy and in combination therapy with ceralasertib in Fig. 25 were plotted. HT29/H460 cell line proliferation inhibition IC₅₀ values of AST-3424 as monotherapy and in combination therapy with ceralasertib were derived by fitting the assay results of Fig. 25 and summarized in Table 13 below.

**Table 13: HT29/H460 Cell Line Proliferation Inhibition IC₅₀ Values of AST-3424 as Monotherapy and in Combination Therapy with Ceralasertib (First Assay)**

| **HT29** | | | **H460** | | |
|---|---|---|---|---|---|
| **Compounds** | **IC₅₀ (nM)** | **Ratio combo/single** | **Compounds** | **IC₅₀ (nM)** | **Ratio combo/ single** |
| **AST-3424** | **128.60** | **/** | **AST-3424** | **0.40** | **/** |
| **AST3424 + 0.04 µM ceralasertib** | **3.26** | **39.45** | **AST3424 + 0.15 µM ceralasertib** | **0.06** | **6.67** |
| **AST-3424 + 1.3 µM ceralasertib** | **1.42** | **90.56** | **AST-3424 + 0.44 µM ceralasertib** | **0.02** | **20.00** |
| **AST-3424 + 2 µM ceralasertib** | **2.41** | **53.36** | **AST-3424 + 1.3 µM ceralasertib** | **0.03** | **13.33** |

| | | | | | |
|---|---|---|---|---|---|
| Note: "Ratio" denotes a ratio. | | | | | |

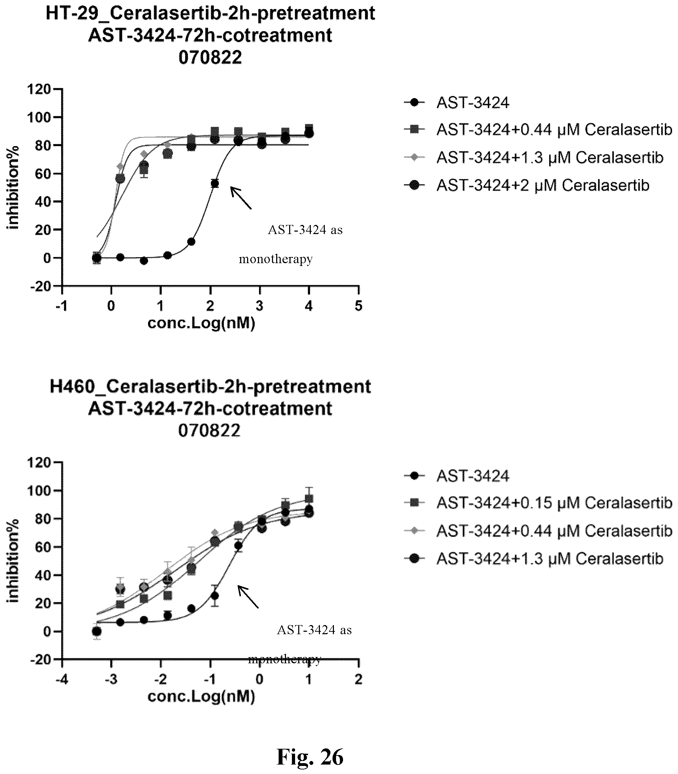

In a second duplicate assay, the HT29/H460 cell line proliferation inhibition curves of AST-3424 as monotherapy and in combination therapy with ceralasertib in Fig. 26 were plotted. HT29/H460 cell line proliferation inhibition IC₅₀ values of AST-3424 as monotherapy and in combination therapy with ceralasertib were derived by fitting the assay results of Fig. 26 and summarized in Table 14 below.

**Table 14: HT29/H460 Cell Line Proliferation Inhibition IC₅₀ Values of AST-3424 as Monotherapy and in Combination Therapy with Ceralasertib (Second Assay)**

| **HT29** | | | **H460** | | |
|---|---|---|---|---|---|
| **Compounds** | **IC₅₀ (nM)** | **Ratio combo/single** | **Compounds** | **IC₅₀ (nM)** | **Ratio combo /single** |
| **AST-3424** | **98.99** | **/** | **AST-3424** | **0.24** | **/** |
| **AST3424 + 0.04 µM ceralasertib** | **1.62** | **61.10** | **AST3424 + 0.15 µM ceralasertib** | **0.06** | **4.00** |
| **AST-3424 + 1.3 µM ceralasertib** | **1.21** | **81.81** | **AST-3424 + 0.44 µM ceralasertib** | **0.01** | **24.00** |
| **AST-3424 + 2 µM ceralasertib** | **1.23** | **80.48** | **AST-3424 + 1.3 µM ceralasertib** | **0.02** | **12.00** |

| | | | | | |
|---|---|---|---|---|---|
| Note: "Ratio" denotes a ratio. | | | | | |

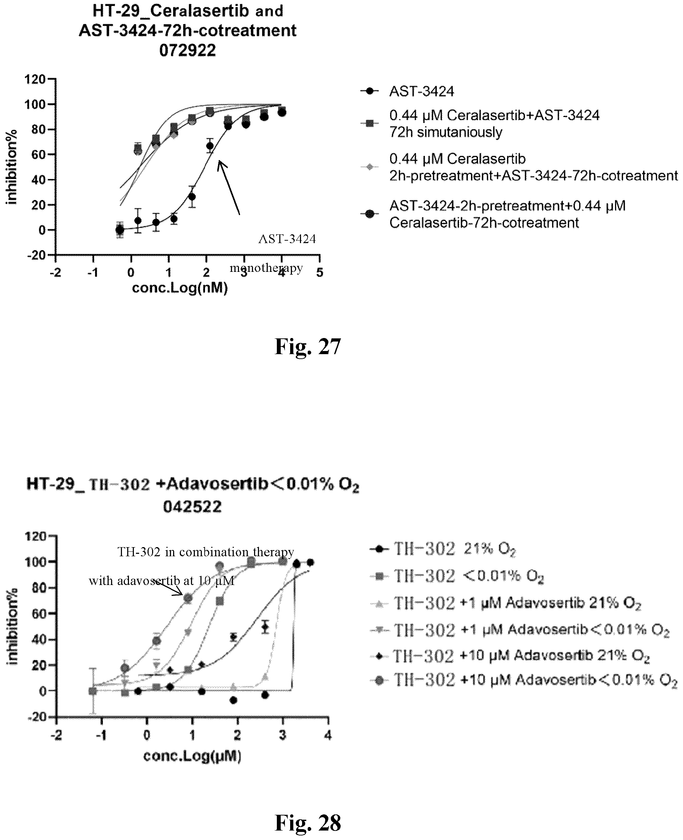

In order to further investigate the influence of the order of adding drugs on the efficacy of combination therapy, ceralasertib was added before AST-3424, and proliferation inhibition curves were plotted in Fig. 27. HT29/H460 cell line proliferation inhibition IC₅₀ values of AST-3424 as monotherapy and in combination therapy with ceralasertib were derived by fitting the assay results of Fig. 27 and summarized in Table 15 below.

**Table 15: HT29 Cell Line Proliferation Inhibition IC₅₀ Values of AST-3424 as Monotherapy and AST-3424 and Ceralasertib in Combination Therapy Added in Different Orders**

| **Compounds** | **IC₅₀ (nM)** | **Ratio single/combo** |
|---|---|---|
| **AST-3424** | **88.63** | |
| **0.44 µM ceralasertib + AST-3424 72 h, simultaneously** | **1.72** | **51.53** |
| **0.44 µM ceralasertib 2h-pretreatment + AST-3424-72h-cotreatment** | **2.36** | **37.56** |
| **AST3424-2h-pretreatment + 0.44 µM ceralasertib-72h-cotreatment** | **1.69** | **52.44** |

| | | |
|---|---|---|
| Notes: the "Compounds" column lists the compounds as monotherapy and in combination therapy; and the "Ratio, single/combo" column lists the IC₅₀ ratios of AST-3424 as monotherapy to AST-3424 in combination therapy with ceralasertib. | | |

### Conclusions:

When AST-3424 and ceralasertib (ATR inhibitor) were used in combination therapy, they exhibited a significant additive cytotoxic effect on the proliferation of H460 (wild-type p53-expressing) cells.

When AST-3424 was used in combination therapy with ceralasertib (ATR inhibitor) at various concentrations, they exhibited very significant additive effects on the proliferation of HT29 (p53-deficient) cells, and these additive cytotoxic effects were more significant than those on the H460 cell line.

When the orders of adding drugs were different, the additive cytotoxic effects of AST-3424 and ceralasertib used in combination therapy did not show any differences.

### Conclusions on in Vitro Cell Assays of Compounds AST and AST-3424:

(1) in the p53-deficient (HT29) cell line, AST and AST-3424 each exhibited significant additive cytotoxic effects when they were used in combination therapy with any of the following cell cycle inhibitors: adavosertib, AZD7762, palbociclib and ceralasertib. And such the additive cytotoxic effects were still significant even when both AST or AST-3424 and the cell cycle inhibitor were used in combination therapy at low concentrations.
(2) in wild-type p53-expressing (H460) cells, AST and AST-3424 each exhibited certain additive cytotoxic effects when they were used in combination therapy with any of the following cell cycle inhibitors at a medium or high concentration: adavosertib, AZD7762 and ceralasertib. However, such additive cytotoxic effects were not significant when both AST or AST-3424 and the cell cycle inhibitor were used in combination therapy at low concentrations.
(3) in wild-type p53-expressing (H460) cells, AST-3424 or AST, when used in combination therapy with palbociclib, did not exhibit any significant additive cytotoxic effects.

Further assays were conducted under different conditions, namely, under hypoxia and normoxia, according to the method described below to investigate HT29/H460 cell line proliferation inhibition of TH-302 as monotherapy or in combination therapy with adavosertib/ceralasertib:
1) An HT29 cell suspension was added to two types of 24-well plates at 495 µL per well so that each well contained 5×10⁴ cells. 24-well plates with glass inserts were used in assays under hypoxia, and ordinary plastic 24-well plates were used in assays under normoxia.
2) The cells were incubated overnight at 37 °C in an incubator with 5% CO₂.
3) Treatment with Compounds

### Hypoxia (O₂<0.01%):

A hypoxia workstation was tuned to create a hypoxic environment, and an oxygen indicator was used to confirm the hypoxia in the workstation.

After cell plating for 24 hours, the 24-well plates with glass inserts were transferred into the hypoxia workstation.

The 24-well plates were placed on a shaker, uncovered and shaken to allow air exchange for 5 min.

For each well for combination therapy, 5 or 10 µL of medium was removed and 5 µL of adavosertib (to a final concentration of 1 or 10 µM) or ceralasertib (to a final concentration of 200 or 2000 µM), followed by incubation for 2 h.

To each well, 5 or 10 µL of the compound was added at a 100× concentration. Three replicate wells were set up for each group.

Gentle shaking was carried out until homogeneous mixing of the compound, and the 24-well plates were incubated for 3 hours in the hypoxia workstation with their lids being half opened.

### Normoxia (O₂ 21%):

24 hours after plating of the cells, for each well for combination therapy, 5 or 10 µL of medium was removed and 5 µL of adavosertib (to a final concentration of 1 or 10 µM) or ceralasertib (to a final concentration of 200 or 2000 µM), followed by incubation for 2 h.

To each well, 5 or 10 µL of the compound was added at a 100× concentration. Three replicate wells were set up for each group.

Gentle shaking was carried out until homogeneous mixing of the compound, and the 24-well plates were incubated for 3 hours at 37 °C in a standard incubator with 5% CO₂.
4) After 3 hours of cotreatment with the compounds, all the 24-well plates were washed twice with medium, with 500 µL for each well.
5) 500 µL of medium was added to each well.
6) The cells were incubated at 37 °C for 72 hours in an incubator with 5% CO₂.
7) For each well, 300 µL of medium was removed and discarded, and 50 µL of CTG was added, followed by shaking for 2 min for homogenization and placement in dark at room temperature for 15 min.
8) 100 µL of medium was transferred from each well in the 24-well plates to a blank 96-well plate.
9) Chemiluminescence was read for 1000 ms on a multimode microplate reader.
10) IC₅₀ (half-maximal inhibitory concentration) values of the compounds were calculated using the GraphPad Prism 5 software.

In the H460 cell proliferation inhibition assays, adavosertib was added to final concentrations of 3 µM and 30 µM, and ceralasertib was added to final concentrations of 2 µM, 20 µM and 1000 µM.

### 14. Results of HT29/H460 Cell Line Proliferation Inhibition Assays of TH-302 as Monotherapy and in Combination Therapy with Adavosertib/Ceralasertib

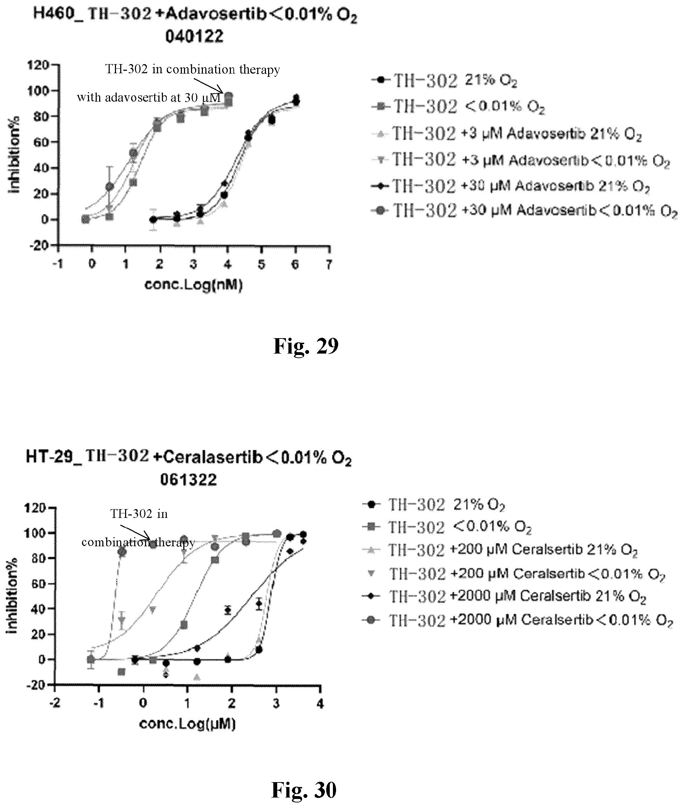

According to the method discussed above, IC₅₀ values of TH-302 as monotherapy or in combination therapy with adavosertib under normoxia and hypoxia were derived for HT29 and H460 cell lines and summarized in Tables 16 and 17, and corresponding proliferation inhibition curves are shown in Figs. 28 and 29.

**Table 16: Results of HT29 Cell Line Proliferation Inhibition Assays of TH-302 as Monotherapy and in Combination Therapy with Adavosertib under Normoxia and Hypoxia**

| Compounds | IC₅₀ (µM) | Normoxia /Hypoxia Ratio | Ratio single/combo under Normoxia | Ratio single/combo under Hypoxia |
|---|---|---|---|---|
| TH-302, normoxia | ~ 1736 | / | / | / |
| TH-302, hypoxia | 23.35 | 74.35 | / | / |
| TH-302 + 1 µM adavosertib, normoxia | 720.40 | / | 2.41 | / |
| TH-302 + 1 µM adavosertib, hypoxia | 9.38 | 76.80 | / | 2.49 |
| TH-302 + 10 µM adavosertib, normoxia | 264.10 | / | 6.57 | / |
| TH-302 + 10 µM adavosertib, hypoxia | 2.69 | 98.18 | / | 8.68 |

| | | | | |
|---|---|---|---|---|
| Notes: the "Compounds" column lists the compounds as monotherapy and in combination therapy; and "Ratio" denotes a ratio. | | | | |

**Table 17: Results of H460 Cell Line Proliferation Inhibition Assays of TH-302 as Monotherapy and in Combination Therapy with Adavosertib**

| Compounds | IC₅₀ (µM) | Normoxia /Hypoxia Ratio | Ratio single/combo under Normoxia | Ratio single/combo under Hypoxia |
|---|---|---|---|---|
| TH-302, normoxia | 20992.0 0 | / | / | / |
| TH-302, hypoxia | 25.45 | 824.83 | / | / |
| TH-302 + 3 µM adavosertib, normoxia | 23560.0 0 | / | 1.12 | / |
| TH-302 + 3 µM adavosertib, hypoxia | 16.27 | 1448.06 | / | 1.56 |
| TH-302 + 30 µM adavosertib, normoxia | 18055.0 0 | / | / | / |
| TH-302 + 30 µM adavosertib, hypoxia | 11.89 | 1518.50 | / | 2.14 |

| | | | | |
|---|---|---|---|---|
| Notes: the "Compounds" column lists the compounds as monotherapy and in combination therapy; and "Ratio" denotes a ratio. | | | | |

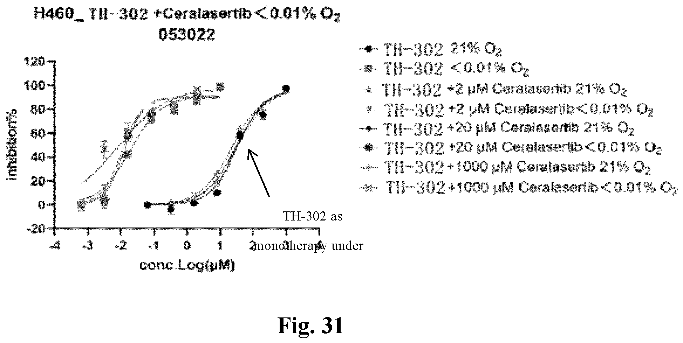

According to the method discussed above, IC₅₀ values of TH-302 as monotherapy or in combination therapy with ceralasertib under normoxia and hypoxia were derived for HT29 and H460 cell lines and summarized in Tables 18 and 19, and corresponding proliferation inhibition curves are shown in Figs. 30 and 31.

**Table 18: Results of HT29 Cell Line Proliferation Inhibition Assays of TH-302 as Monotherapy and in Combination Therapy with Ceralasertib**

| Compounds | IC₅₀ (µM) | Normoxia /Hypoxia Ratio | Ratio single/combo under Normoxia | Ratio single/combo under Hypoxia |
|---|---|---|---|---|
| TH-302, normoxia | 737.60 | / | / | / |
| TH-302, hypoxia | 14.29 | 51.62 | / | / |
| TH-302 + 200 µM ceralsertib, normoxia | 592.50 | / | 1.24 | / |
| TH-302 + 200 µM ceralsertib, hypoxia | 2.01 | 294.78 | / | 7.11 |
| TH-302 + 2000 µM ceralsertib, normoxia | 265.90 | / | 2.77 | / |
| TH-302 + 2000 µM ceralsertib, hypoxia | <0.32 | >821.56 | / | >44.66 |

| | | | | |
|---|---|---|---|---|
| Notes: the "Compounds" column lists the compounds as monotherapy and in combination therapy; and "Ratio" denotes a ratio. | | | | |

**Table 19: Results of H460 Cell Line Proliferation Inhibition Assays of TH-302 as Monotherapy and in Combination Therapy with Ceralasertib**

| Compounds | IC₅₀ (µM) | Normoxia /Hypoxia Ratio | Ratio combo/single under Normoxia | Ratio single/combo under Normoxia | Ratio single/combo under Hypoxia |
|---|---|---|---|---|---|
| TH-302, normoxia | 32.15 | / | / | / | / |
| TH-302, hypoxia | 0.0185 | 1737.84 | / | / | / |
| TH-302 + 2 µM ceralasertib, normoxia | 34.46 | / | 1.07 | / | / |
| TH-302 + 2 µM ceralasertib, hypoxia | 0.0108 | 3190.74 | / | / | 1.71 |
| TH-302 + 20 µM ceralasertib, normoxia | 30.56 | / | / | 1.05 | |
| TH-302 + 20 µM ceralasertib, hypoxia | 0.0124 | 2464.52 | / | / | 1.49 |
| TH-302 + 1000 µM ceralasertib, normoxia | 23.56 | / | / | 1.36 | / |
| TH-302 + 1000 µM ceralasertib, hypoxia | <0.003 | >7853.33 | / | / | >6.17 |

| | | | | | |
|---|---|---|---|---|---|
| Notes: the "Compounds" column lists the compounds as monotherapy and in combination therapy; and "Ratio" denotes a ratio. | | | | | |

### Conclusions on in Vitro Cell Assays of Compound TH-302

(1) in the p53-deficient (HT29) cell line, TH-302 used in combination therapy with the G2/M cell cycle regulator adavosertib did not exhibit any significant additive cytotoxic effects under normoxia.
(2) in the the p53-deficient (HT29) cell line, TH-302 used in combination therapy with the G2/M cell cycle regulator adavosertib at a high concentration exhibited a significant additive cytotoxic effect under hypoxia.
(3) in the wild-type p53-expressing (H460) cells, TH-302 used in combination therapy with the G2/M cell cycle regulator adavosertib did not exhibit any significant additive cytotoxic effects, whether under normoxia or under hypoxia.
(4) in the p53-deficient (HT29) cell line, TH-302 used in combination therapy with the ATR kinase inhibitor ceralasertib did not exhibit any significant additive cytotoxic effects under normoxia.
(5) in the p53-deficient (HT29) cell line, TH-302 used in combination therapy with the ATR kinase inhibitor ceralasertib at a high concentration exhibited a significant additive cytotoxic effect under hypoxia.
(6) in the wild-type p53-expressing (H460) cells, TH-302 used in combination therapy with the ATR kinase inhibitor ceralasertib did not exhibit any significant additive cytotoxic effects, whether under normoxia or under hypoxia.

In the above embodiments, the compounds AST-3424, AST and TH-302 are all DNA alkylating agent prodrugs, which specifically release the small-molecule DNA alkylating agent AST-2660 (or 2660) or Br-IPM in particular environments.

For example, the mechanisms of AST-3424 and TH-302 are as shown below:

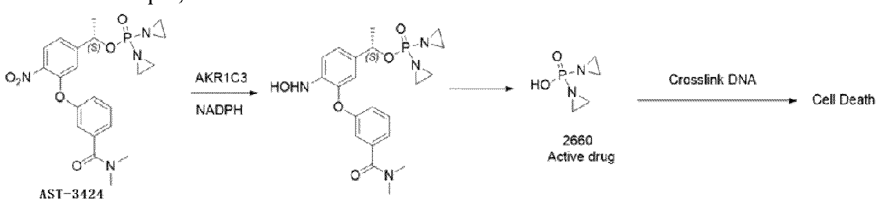

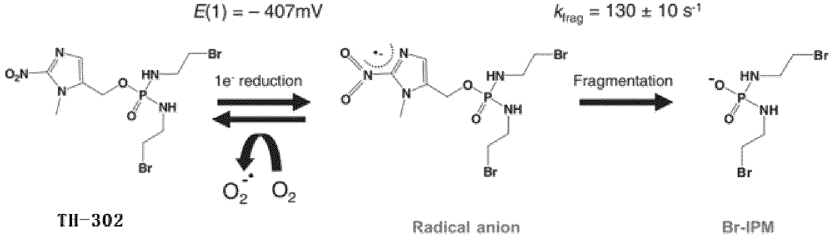

The DNA alkylating agents eventually released by those compounds are non-cell-cycle-specific. That is, they act on double-stranded DNA at various stages of the cell cycle. Alkylating agents can form very strong bonds between double-stranded DNA molecules, making them unable to undergo transcription, translation, replication or other processes. That is, they can exert DNA damage and proliferation inhibition effects on cells at various cell cycle phases.

The compound adavosertib is a selective inhibitor of the Wee1 kinase, a crucial kinase for the G2/M cell cycle checkpoint and DNA damage repair. Cyclin-dependent kinases (CDKs) regulate the cell cycle by phosphorylating specific substrates, and their activity depends on that of the complexes resulting from their binding with cyclins. Moreover, their activity is regulated by phosphorylation, and the phosphorylation of CDK1 further controls the G2/M checkpoint. Wee1 maintains CDK1 inactive by phosphorylating Tyr15 in CDK1, thereby inhibiting the activity of the CDK1-cyclin B complex. In this way, it arrests cell division at the G2/M checkpoint and negatively regulates the cell cycle. Its biological significance is to repair DNA damage that failed to be repaired in time to prevent cells from progressing to the mitosis phase with such DNA damage. That is, adavosertib can regulate CDK1 phosphorylation by inhibiting the Wee1kinase, thus regulating the progression of the cell cycle. In other words, adavosertib is an inhibitor that indirectly acts on CDK and can be considered as a CDK inhibitor in a broader sense. Palbociclib is an orally active selective inhibitor of CDK4 and CDK6. Therefore, adavosertib and palbociclib are both cell-cycle-specific CDK inhibitors that act on specific cell cycle stages.

According to a considerable body of literature, the p53 protein plays an important regulatory role in DNA repair at the G1/S cell cycle phase, and p53-deficient cells can be inhibited from DNA repair at the G1/S phase. Therefore, exposure of such cells to an inhibitor capable of inducing G2/M cell cycle arrest can significantly inhibit DNA repair at the G2/M cell cycle phase.

The assays described herein reveal that adavosertib at a medium or high concentration shows a significant additive cytotoxic effect on tumor cell lines when used in combination therapy with AST or AST-3424. Moreover, a significant additive cytotoxic effect can be achieved on p53-deficient tumor cell lines, even at a low concentration of adavosertib. Palbociclib shows a significant additive cytotoxic effect only on p53-deficient tumor cell lines, when used in combination therapy with AST or AST-3424. Adavosertib at a high concentration shows a significant additive cytotoxic effect only on p53-deficient tumor cell lines, when used in combination therapy with TH-302.

Based on these assay findings, coupled with the facts that AST, AST-3424 and TH-302 are all DNA alkylating agents of general structural formulae (1) to (9), which are prodrug compounds ultimately acting on cross-linking of double-stranded DNA, and that adavosertib and palbociclib are both CDK inhibitors acting on specific cell cycle stages, one of skill in the art can infer that such an alkylating agent prodrug compound and a CDK inhibitor acting on a specific cell cycle stage have an additive cytotoxic effect on tumor cell lines. That is, the two drugs, when used in combination therapy to treat a cancer/tumor patient, can provide a better therapeutic effect. Moreover, p53 deficiency can enhance the additive/combination therapy's effect.

AZD7762 is an effective ATP-competitive inhibitor of cell cycle checkpoint kinases (CHKs). This CHK inhibitor can eliminate DNA damage-induced S and G2 checkpoints. AZD7762 is a cell-cycle-specific CHK inhibitor and acts on a specific cell cycle phase.

The assays described herein reveal that AZD7762 at a medium or high concentration exhibits a significant additive cytotoxic effect on tumor cell lines when used in combination therapy with AST or AST-3424. Moreover, a significant additive cytotoxic effect can be achieved on p53-deficient tumor cell lines, even at a low concentration of AZD7762.

Based on these assay findings, coupled with the facts that AST and AST-3424 are both DNA alkylating agents of general structural formulae (1) to (9), which are prodrug compounds ultimately acting on cross-linking of double-stranded DNA, and that AZD7762 is a CHK inhibitor acting on a specific cell cycle stage, one of skill in the art can infer that such an alkylating agent prodrug compound and a CHK inhibitor acting on a specific cell cycle stage have an additive cytotoxic effect on tumor cell lines. That is, the two drugs, when used in combination therapy to treat a cancer/tumor patient, can provide a better therapeutic effect. Moreover, p53 deficiency can enhance the additive/combination therapy's effect.

Ceralasertib (AZD6738) is an effective inhibitor of the ATR kinase. It is cell-cycle-specific and acts on a specific cell cycle phase.

The assays described herein reveal that ceralasertib at a medium or high concentration exhibits a significant additive cytotoxic effect on tumor cell lines when used in combination therapy with AST or AST-3424. Moreover, a significant additive cytotoxic effect can be achieved on p53-deficient tumor cell lines, even at a low concentration of AZD7762. Ceralasertib at a high concentration shows a significant additive cytotoxic effect only on p53-deficient tumor cell lines when used in combination therapy with TH-302.

Based on these assay findings, coupled with the facts that AST, AST-3424 and TH-302 are all DNA alkylating agents of general structural formulae (1) to (9), which are prodrug compounds ultimately acting on cross-linking of double-stranded DNA, and that ceralasertib is an ATR inhibitor acting on a specific cell cycle stage, one of skill in the art can infer that such an alkylating agent prodrug compound and an ATR inhibitor acting on a specific cell cycle stage have an additive cytotoxic effect on tumor cell lines. That is, the two drugs, when used in combination therapy to treat a cancer/tumor patient, can provide a better therapeutic effect. Moreover, p53 deficiency can enhance the additive/combination therapy's effect.

It can be further inferred that a non-cell-cycle-specific alkylating agent prodrug compound and a cell cycle inhibitor acting on a specific cell cycle stage have an additive cytotoxic effect on tumor cell lines, when used in combination therapy. That is, the two drugs, when used in combination therapy to treat a cancer/tumor patient, can provide a better therapeutic effect. Moreover, p53 deficiency can enhance the additive/combination therapy's effect. That is, p53-deficient patients would therapeutically benefit more from such combination therapy.

## Claims

1. A method of treatment, the method comprising the steps of treating a cancer/tumor patient by using a drug containing an alkylating agent prodrug compound or salt, ester, solvate or isotopomer thereof in combination therapy with a drug containing a cell cycle inhibitor compound or salt, ester, solvate or isotopomer thereof to treat a cancer/tumor patient.

2. The method of treatment according to claim 1, wherein the cell cycle inhibitor is selected from a CDK inhibitor, a Wee inhibitor, a CHK inhibitor or an ATR inhibitor.

3. The method of treatment according to claim 2, wherein the alkylating agent prodrug compound is selected from an AKR1C3 enzyme-activated alkylating agent prodrug compound or a hypoxia-activated alkylating agent prodrug compound, and wherein
preferably, the AKR1C3 enzyme-activated alkylating agent prodrug compound is used in combination therapy with the CDK inhibitor, the Wee inhibitor, the CHK inhibitor or the ATR inhibitor,
and the hypoxia-activated alkylating agent prodrug compound is used in combination therapy with the CDK inhibitor, the Wee inhibitor or the ATR inhibitor.

4. The method of treatment according to claim 1, wherein the alkylating agent prodrug compound comprises structures represented by the chemical Formulae (1) to (9): 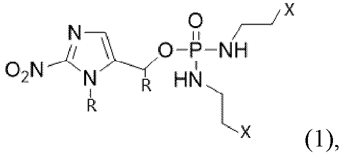
wherein each R is independently selected from H, -CH₃, -CH₂CH₃ and -CF₃, and each X is independently selected from leaving functional groups including Cl, Br, MsO and TsO; 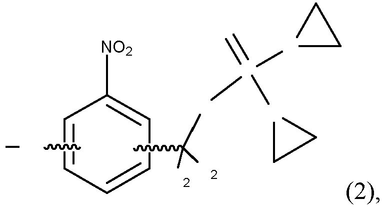
wherein R₁, R₂, R₃ and Cx are defined as in claims of Pat. App. No. PCT/CN2020/114519, published as Pub. No. WO2021120717A1; 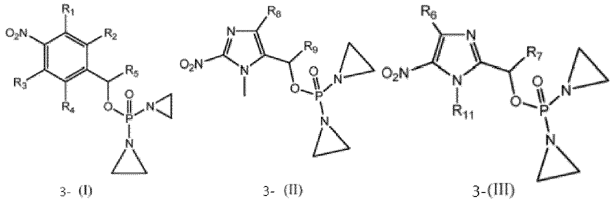 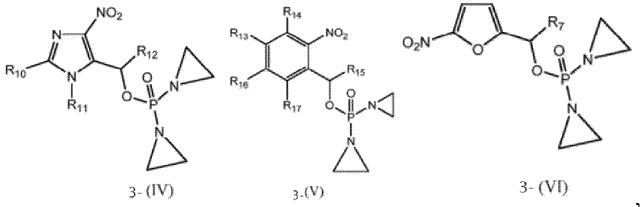
wherein R₁, R₂, R₃, R₄, R₅, R₆, R₇, R₈, R₉, R₁₀, R₁₁, R₁₂, R₁₃, R₁₄, R₁₅, R₁₆ and R₁₇ are defined as in claims of Pat. App. No. PCT/US2016/039092, published as Pub. No. WO2016210175A1 (or Chinese Pat. App. No. 2016800368985, published as Pub. No. CN108024974A); 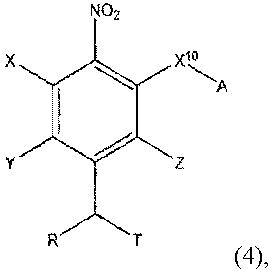
wherein X, Y, Z, R, T, A and X¹⁰ are defined as in claims of Pat. App. No. PCT/US2016/021581, published as Pub. No. WO2016145092A1 (or Chinese Pat. App. No. 2016800150788, published as Pub. No. CN107530556A); 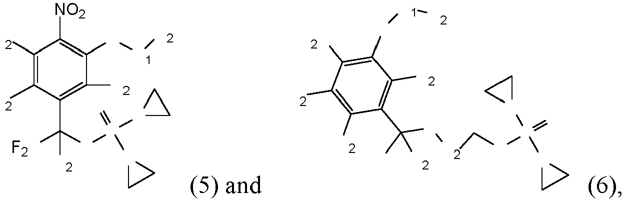
wherein R₁, R₂, R₃, R₄, R₅, R₈, R₉ and R₁₀ are defined as in claims of Pat. App. No. PCT/CN2020/089692, published as Pub. No. WO2020228685A1; 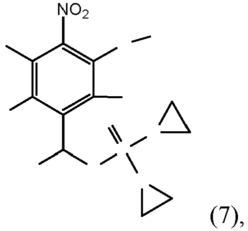
wherein:
A is a substituted or unsubstituted C₆-C₁₀ aryl, biaryl or substituted biaryl, 5-15 membered heteroaryl or -N=CR¹R², wherein each of the substituted groups is substituted with a substituent selected from the group consisting of a halo group, -CN, -NO₂, -O-(CH₂)-O-, -CO₂H or salt thereof, -OR¹⁰⁰, -CO₂R¹⁰⁰, -CONR¹⁰¹R¹⁰², -NR¹⁰¹R¹⁰², - NR¹⁰⁰SO₂R¹⁰⁰, -SO₂R¹⁰⁰, -SO₂NR¹⁰¹R¹⁰², C₁-C₆ alkyl and C₃-C₁₀ heterocycle,
wherein R¹⁰⁰, R¹⁰¹ and R¹⁰² are each independently hydrogen, a C₁-C₈ alkyl or C₆-C₁₂ aryl, or R¹⁰¹ and R¹⁰², together with the nitrogen atom to which they are attached, form a 5-7 membered heterocycle,
wherein the alkyl and aryl are each substituted with 1-3 halo groups or 1-3 C₁-C₆ alkyls, and
R¹ and R² are each independently a phenyl or methyl;
X, Y and Z are each independently hydrogen or a halo group; and
R is hydrogen, a C₁-C₆ alkyl or haloalkyl; 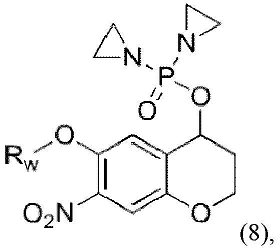
where Rw is defined as in claims of Pat. App. No. PCT/CN2020/120281, published as Pub. No. WO2021068952A1; and 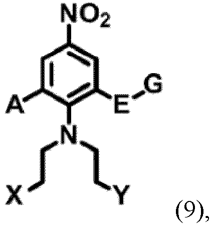
where A, E, G, X and Y are defined as in claims of Pat. App. No. PCT/NZ2019/050030, published as Pub. No. WO2019190331A1 (or Chinese Pat. App. No. 2019800234236, published as Pub. No. CN111918864A).

5. The method of treatment according to claim 1 or 2, wherein
the cancer/tumor patient or a biological sample thereof has been detected with loss or damage of a specific gene,
the specific gene is selected from genes involved in cell cycle checkpoint regulation, preferably from p53, p21, CCNB1, WIP1, 14-3-3 sigma protein and cdc2/cycB.

6. The method of treatment according to claim 3, wherein
the CDK inhibitor is selected from palbociclib, ribociclib, abemaciclib, trilaciclib, dalpiciclib, adavosertib, Ro-3306, dinaciclib, cirtuvivint, rintodestrant, DS96432529, THZ1, THZ531, seliciclib, flavopiridol, AZD4573, SR-4835, simurosertib, fadraciclib, NVP-2, SNS-032, (E/Z)-zotiraciclib, AZD-5438, AT7519, mevociclib, kenpaullone, YKL-5-124 TFA, NG 52, GSK-3 Inhibitor IX, OTS964, samuraciclib, flavopiridol, KB-0742 dihydrochloride, ( + )-enitociclib, AUZ 454, SY-5609, SEL120-34A monohydrochloride, CCT-251921, MBQ-167, XL413 hydrochloride, BI-1347, THAL-SNS-032, JNJ-7706621, TG003, LDC4297, BMS-265246, roniciclib, CGP60474, (R)-CR8 trihydrochloride, R547, milciclib, T025, AS2863619, Senexin A, BSJ-4-116, CLK-IN-T3, CDK12-IN-3, CVT-313, atuveciclib, PHA-793887, indirubin-3'-monoxime, YKL-5-124, PHA-767491 hydrochloride, KH-CB19, cucurbitacin E, purvalanol A, BSJ-03-204, ON123300, CDK5 inhibitor 20-223, riviciclib, FN-1501, CP-10, THZ2, abemaciclib metabolite M2, BS-181, CDK12-IN-E9, samuraciclib, RGB-286638, CDK2-IN-4, LDC000067, ML167, purvalanol B, NU6300, CLK1-IN-1, FMF-04-159-2, CKI-7, CDKI-73, MSC2530818, BSJ-04-132, NU6102, voruciclib and olomoucine;
the Wee inhibitor is selected from Wee1-IN-5, Wee1-IN-3, Wee1-IN-4, LEB-03-146, LEB-03-144, adavosertib, LEB-03-153, LEB-03-145, PD407824, PD0166285, PD0166285 dihydrochloride, pomalidomide-C3-adavosertib, DB0614 and FMF-06-098-1;
the CHK inhibitor is selected from AZD7762, prexasertib, SCH900776, GDC-0425, CHK1-IN-6, CCT245737, BML-277, CCT241533, PD407824, CHIR-124, CCT244747, PF477736, GDC-0575, SB-218078, MRT00033659, ANI-7, SAR-020106, CCT241533, CHK1-IN-4, VER-00158411, CHK1-IN-3, CHK1-IN-5, CHK1-IN-2 and CHK-IN-1; and
the ATR inhibitor is selected from ceralasertib, berzosertib, gartisertib, BAY1895344, BAY-937, AZ20, ETP-46464, dactolisib, VE-821, M1774, ATRN-199, RP-3500 and ART-0380.

7. The method of treatment according to claim 4, wherein
the hypoxia-activated alkylating agent prodrug compound of formula (1) is selected from the compounds having structures represented by the following formulae: 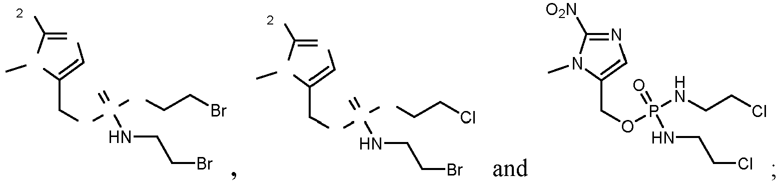
the hypoxia-activated alkylating agent prodrug compound of formula (2) is selected from the compounds having structures represented by the following formulae: 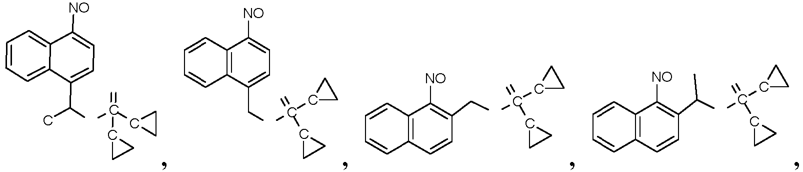 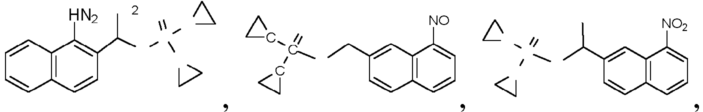 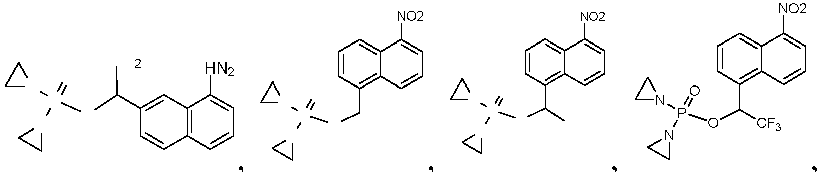 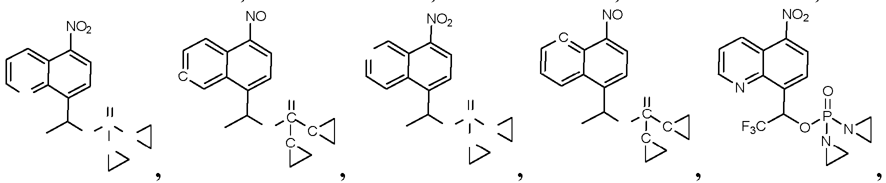 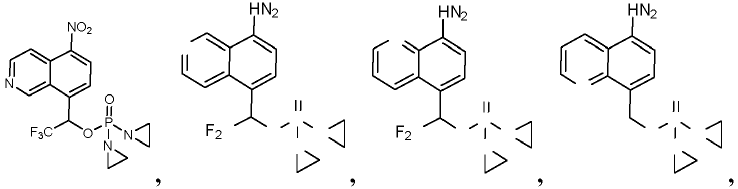 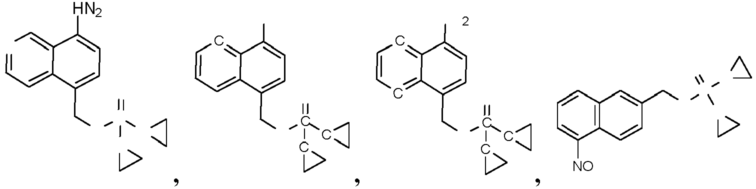 and 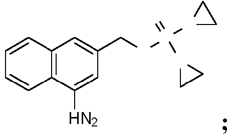
the hypoxia-activated alkylating agent prodrug compound of formula (3) is selected from the compounds having structures represented by the following formulae: 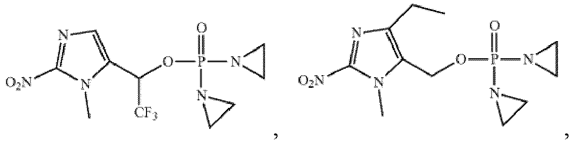 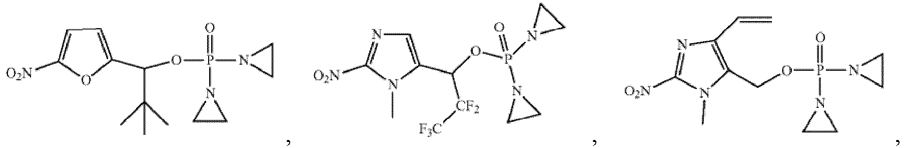 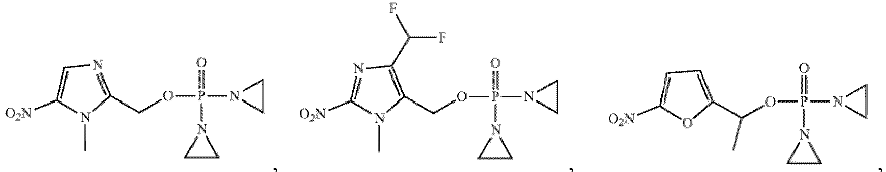 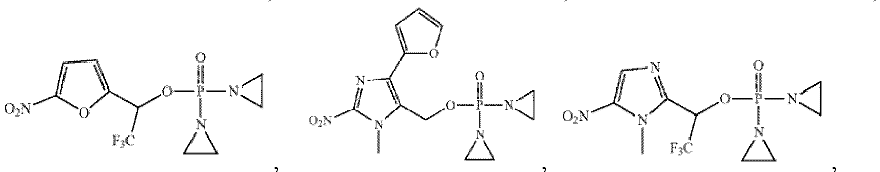 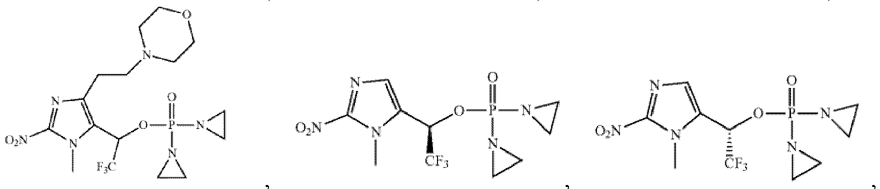 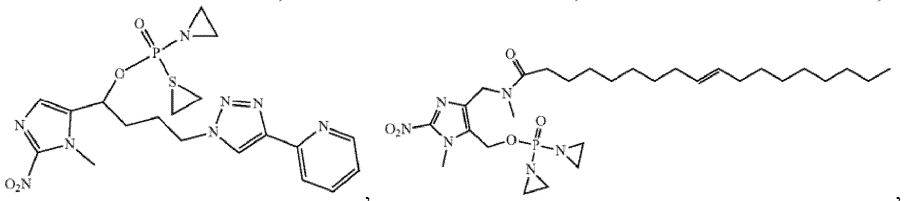 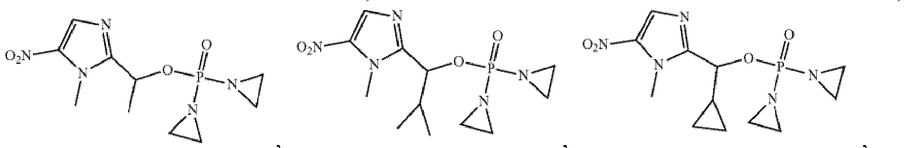 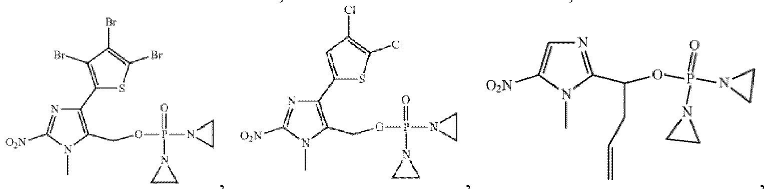 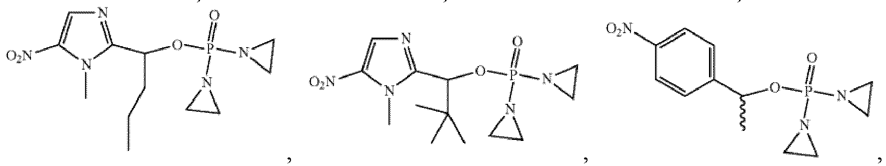 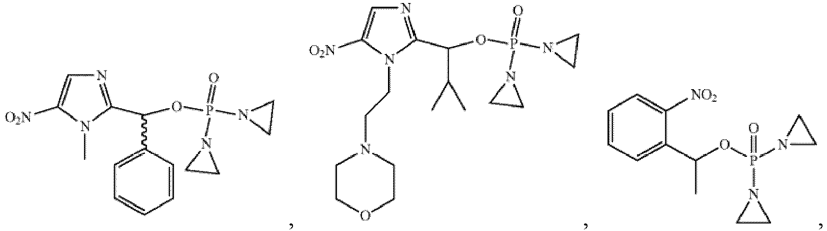 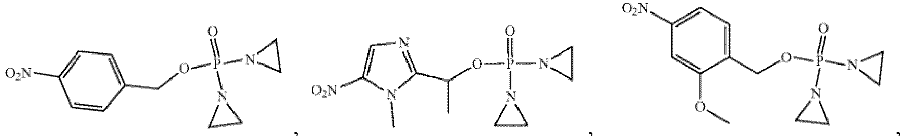 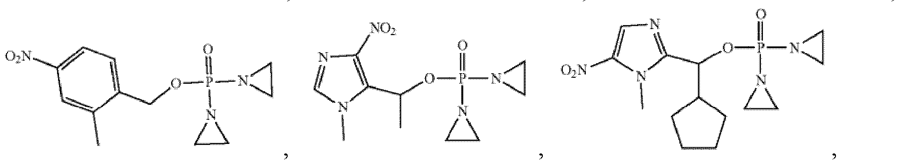 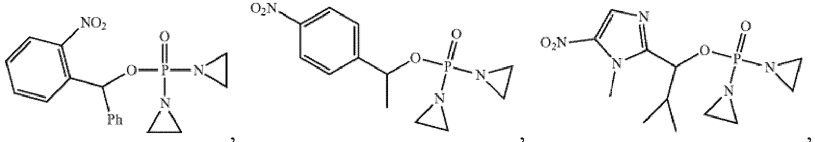 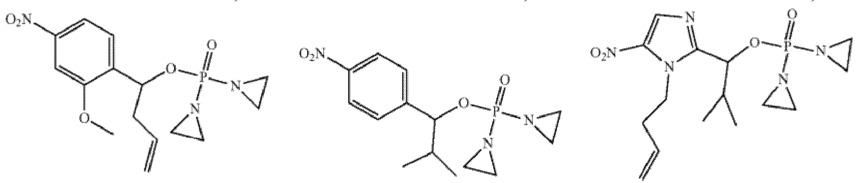 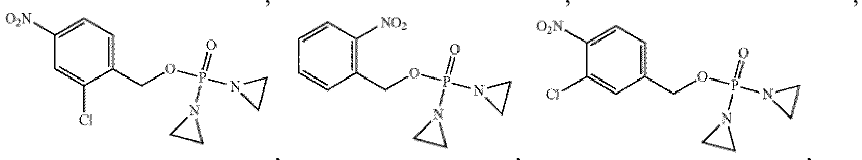 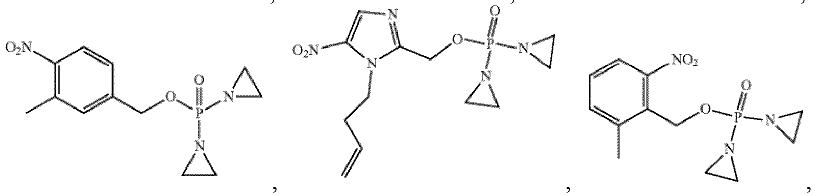 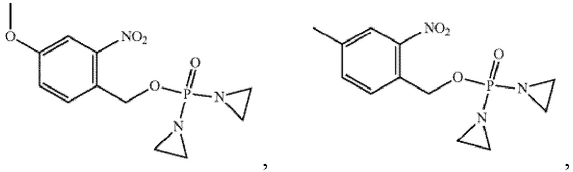 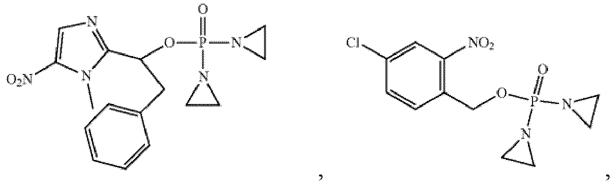 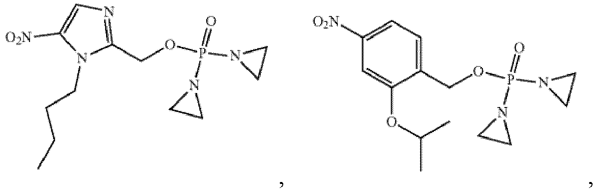 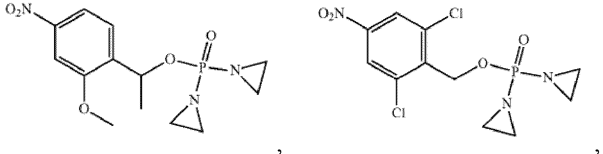 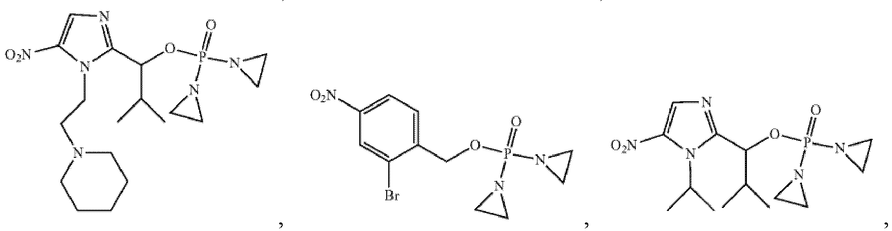 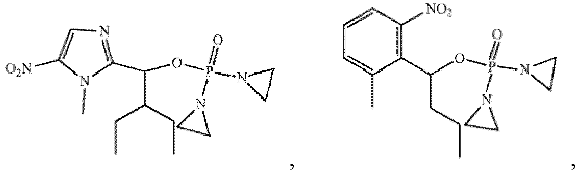 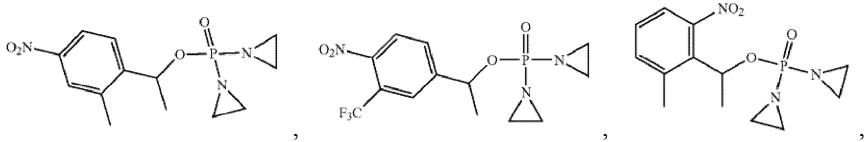 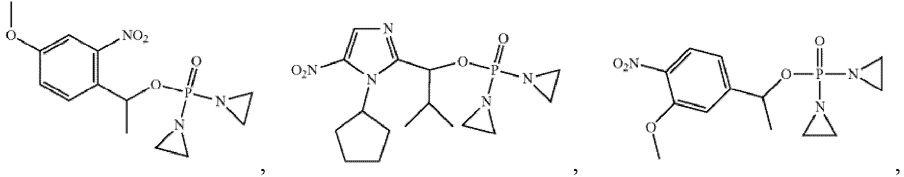 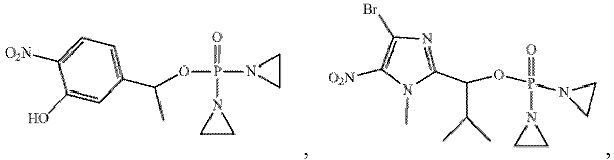 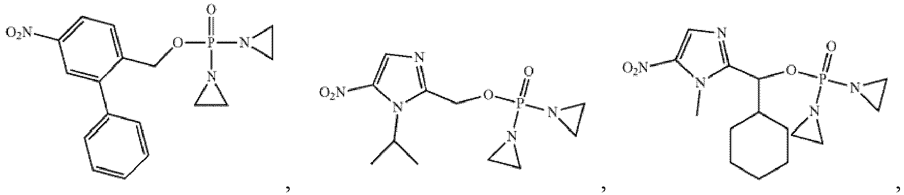 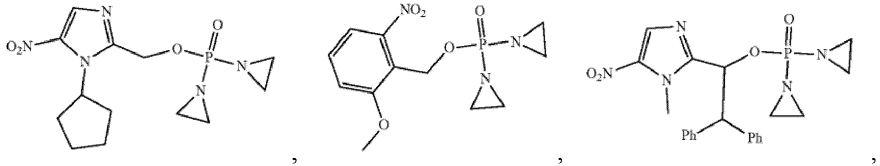 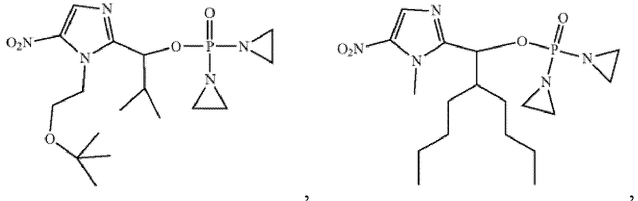 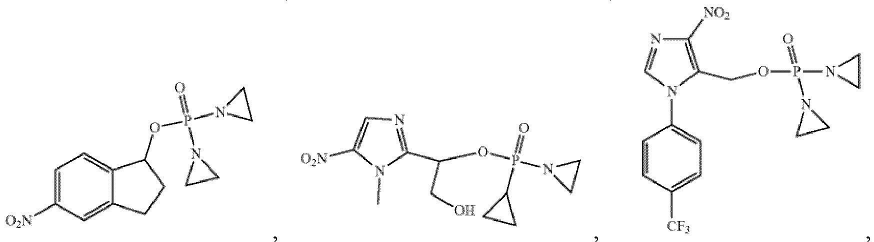 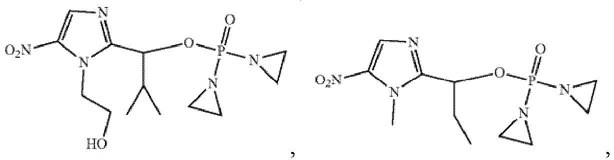 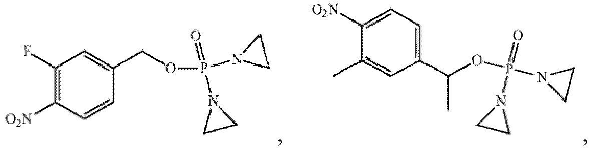 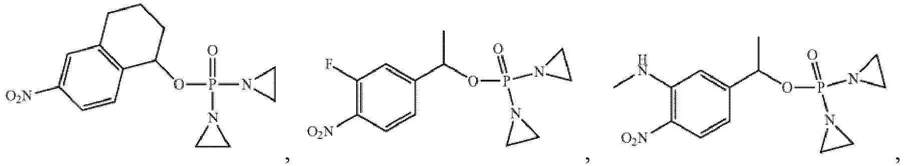 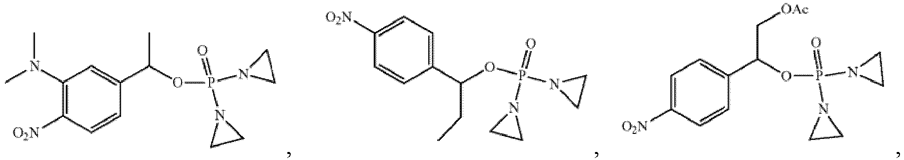 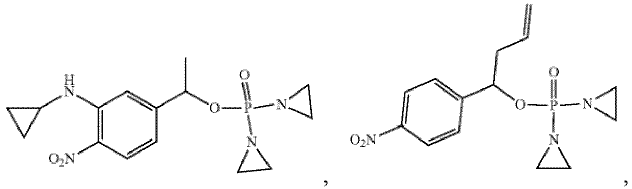 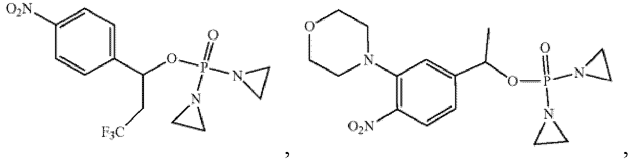 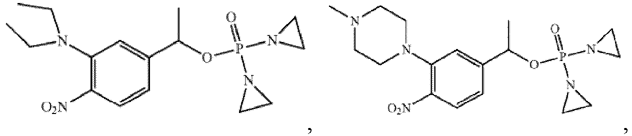 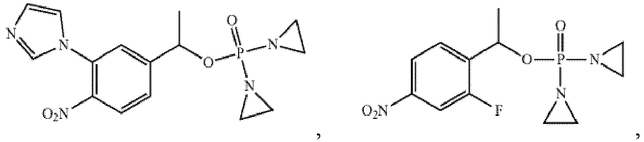 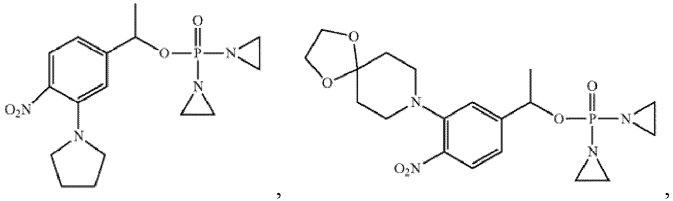 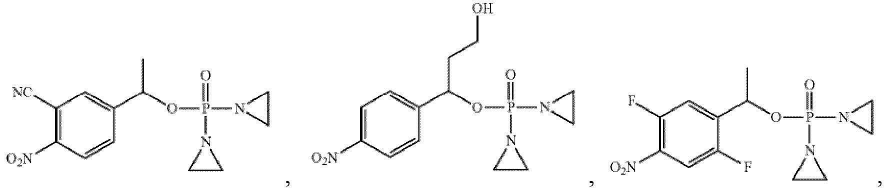 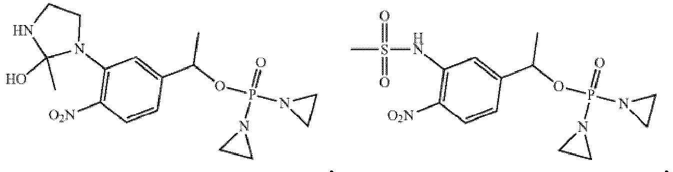 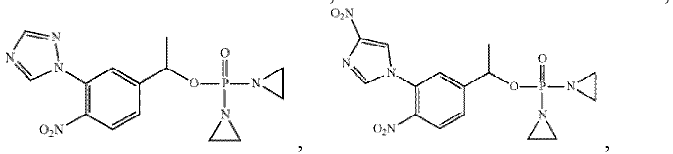 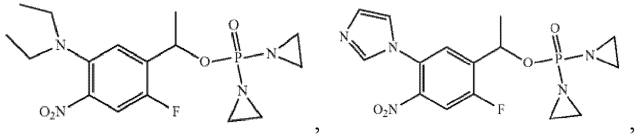 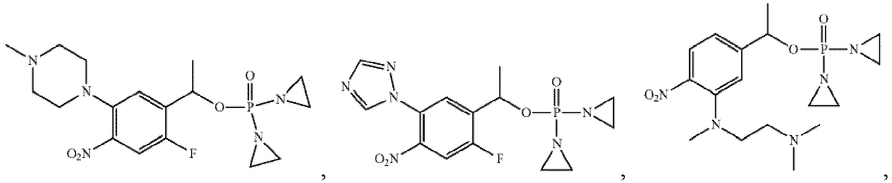 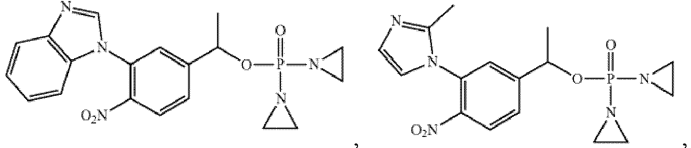 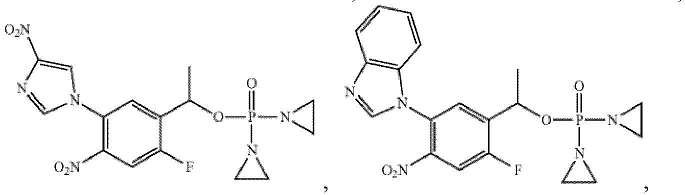 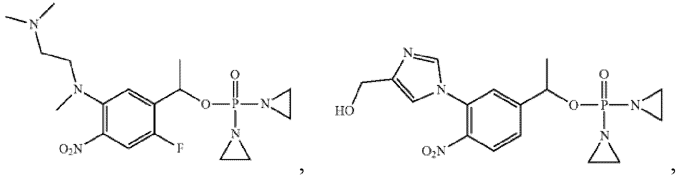 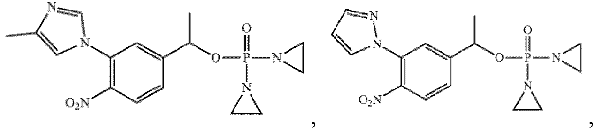 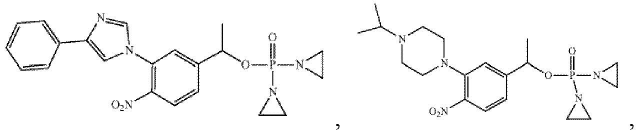 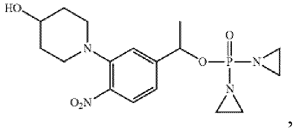 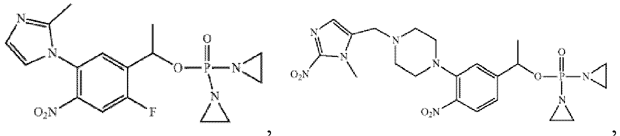 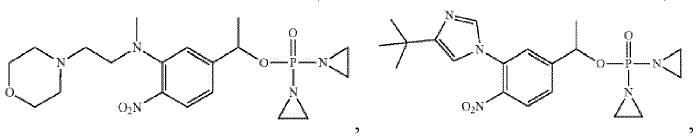 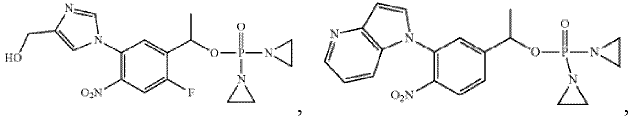 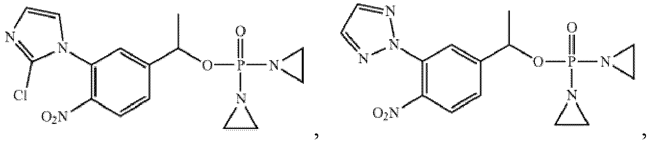 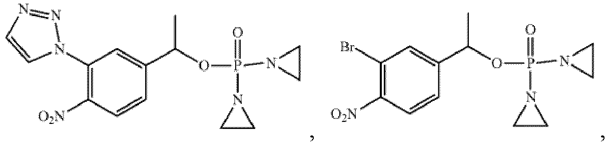 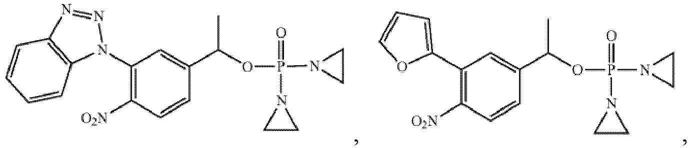 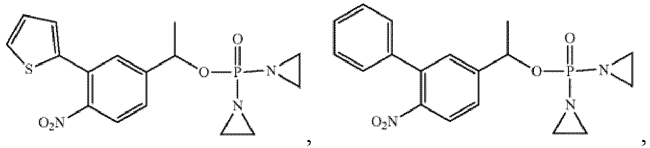 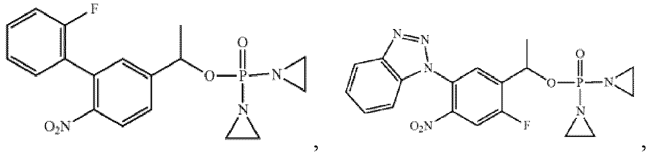 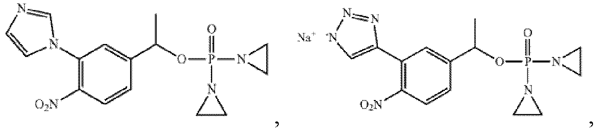 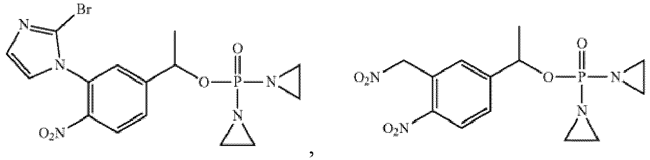 and 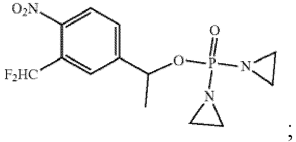
the AKR1C3 enzyme-activated alkylating agent prodrug compound of formula (4) is selected from the compounds having structures represented by the following formulae: 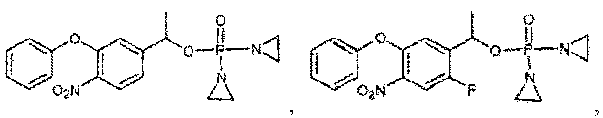 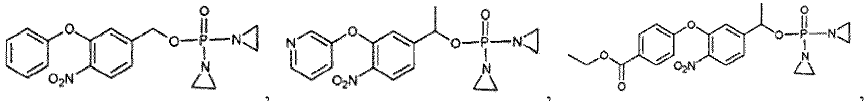 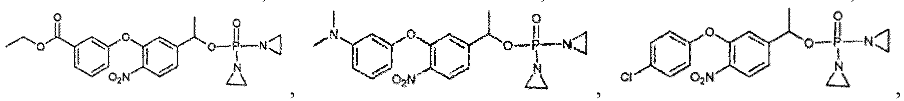 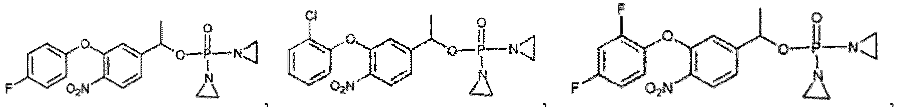 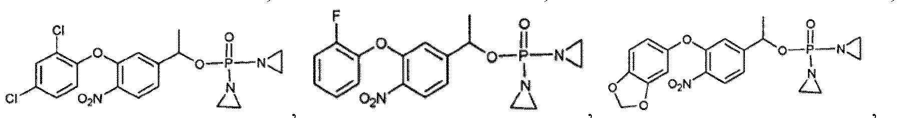 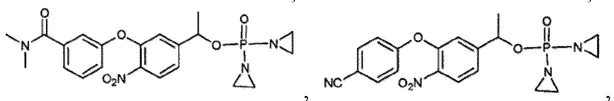 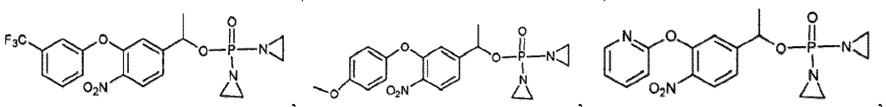 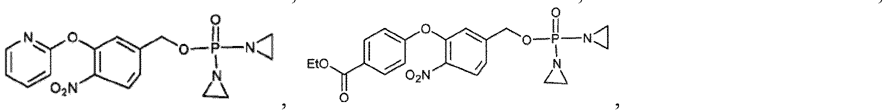 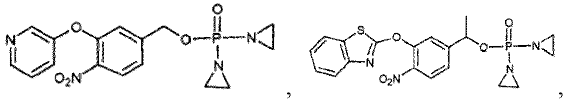 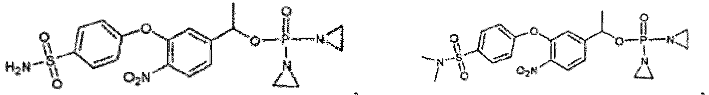 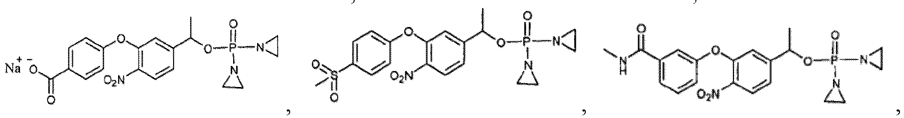 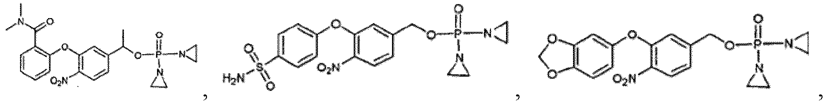 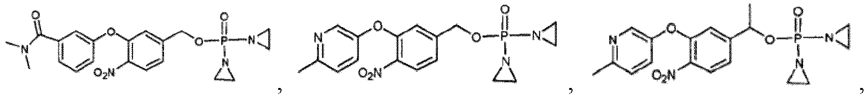 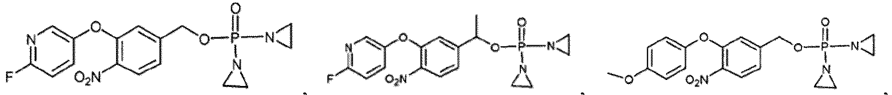 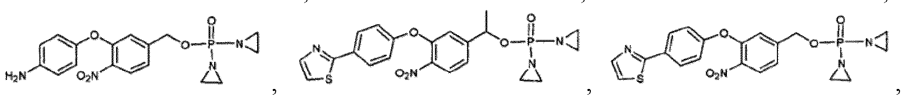 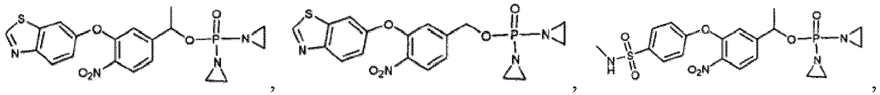 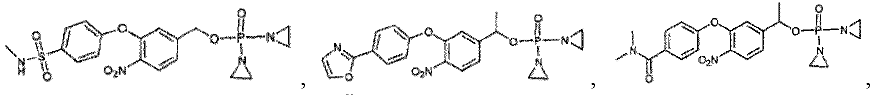 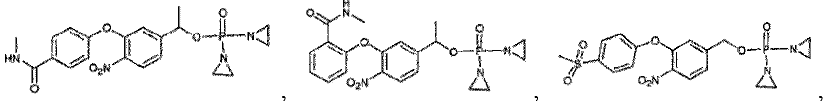 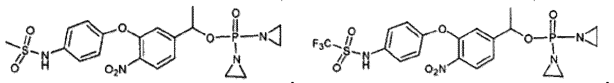 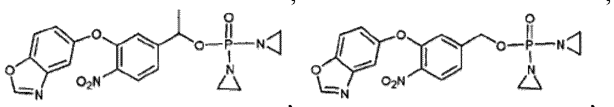 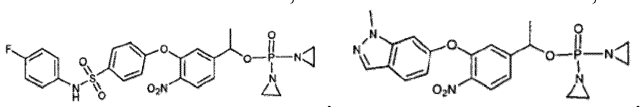 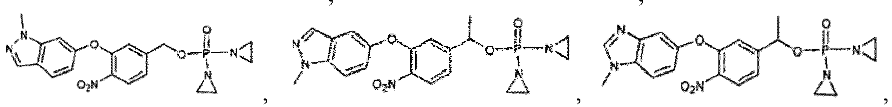 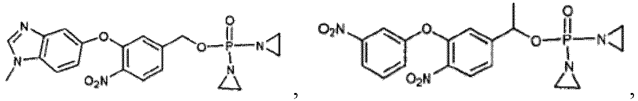 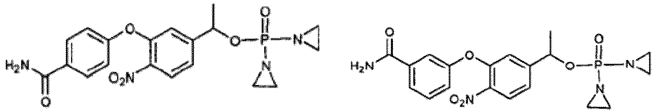 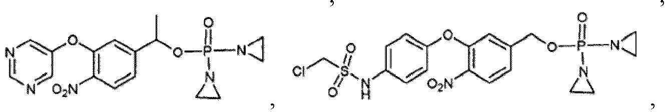 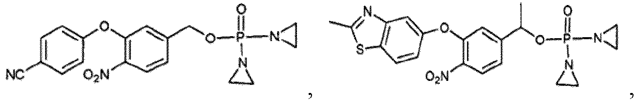 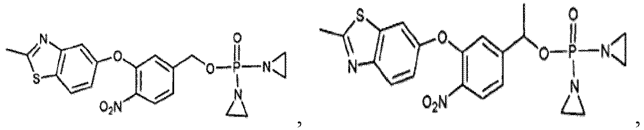 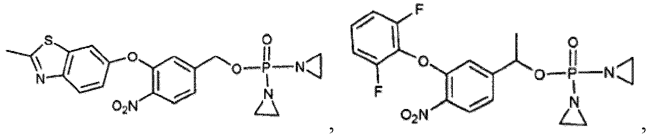 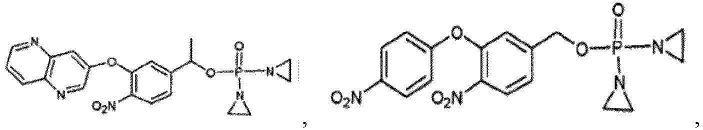 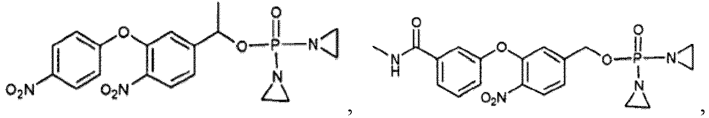 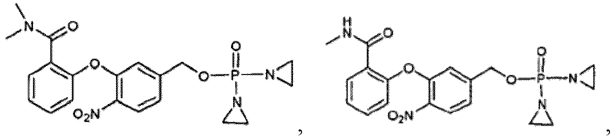 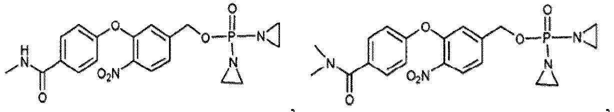 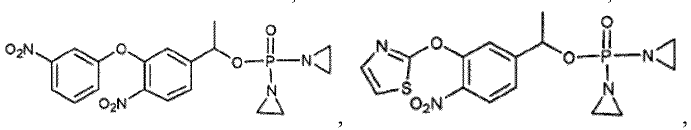 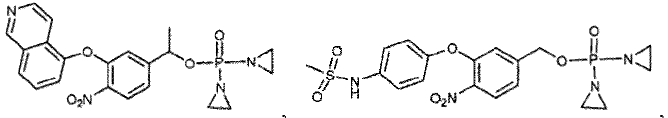 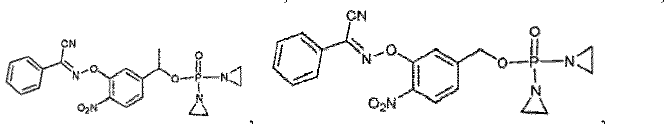 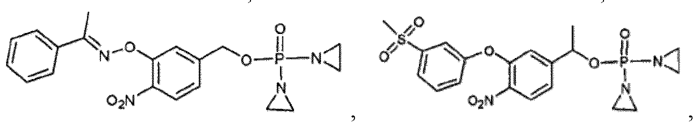 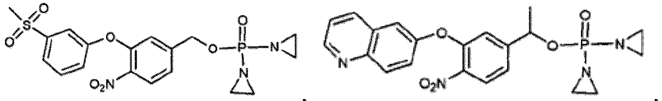 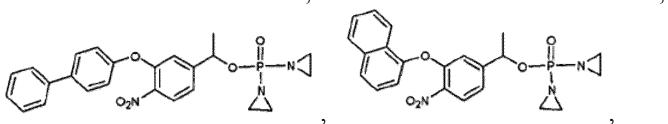 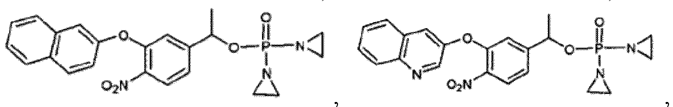 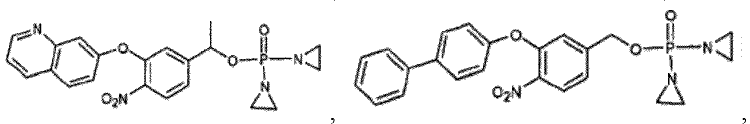 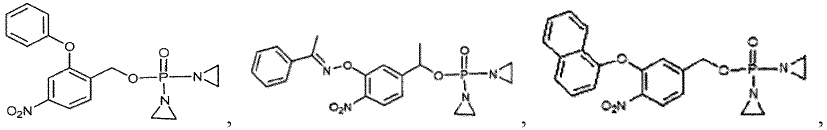 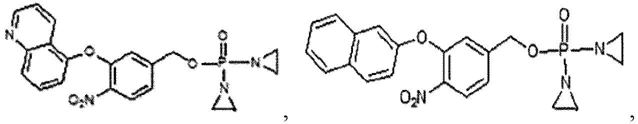 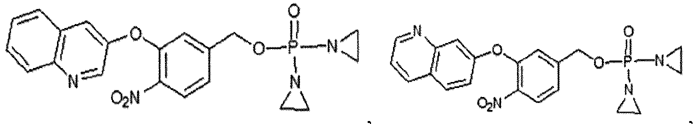 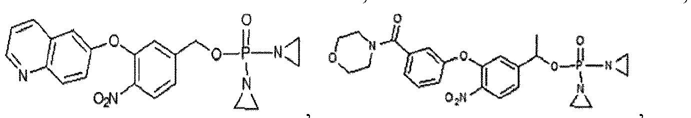 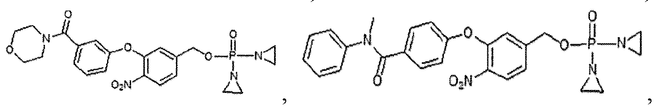 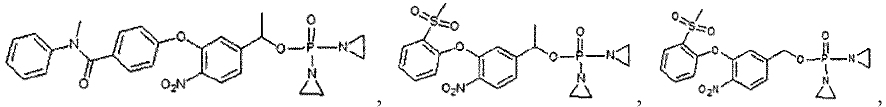 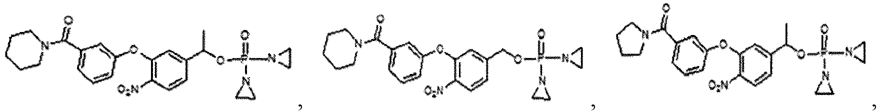 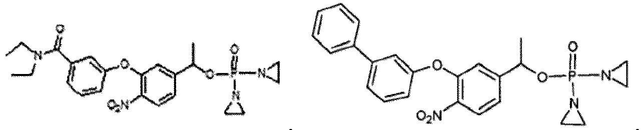 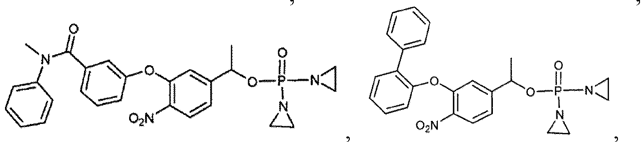 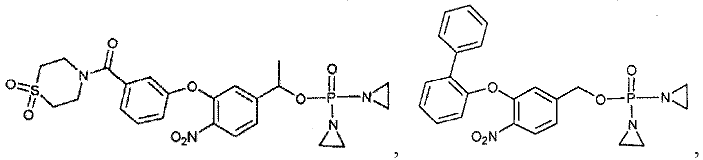 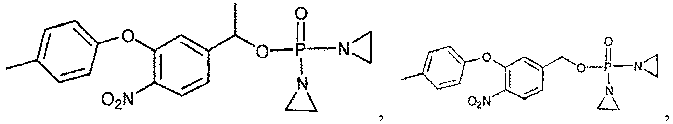 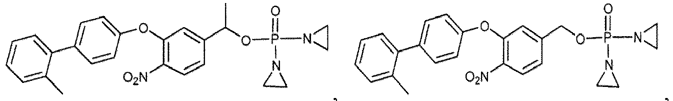 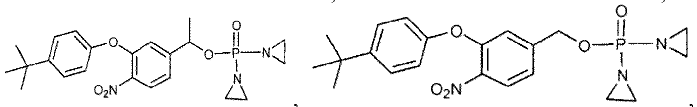 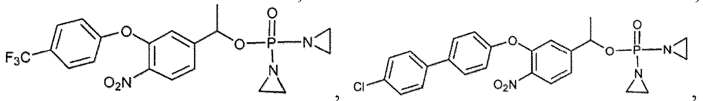 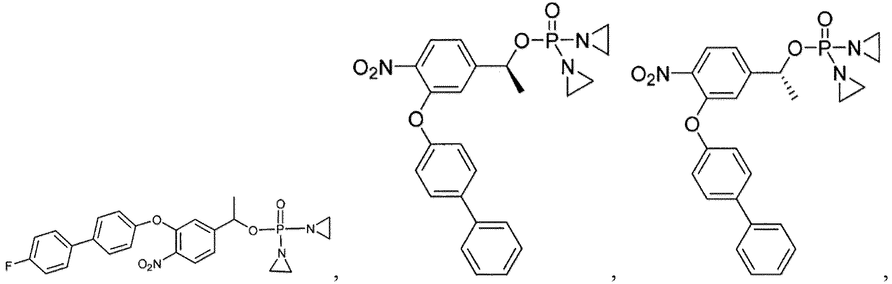 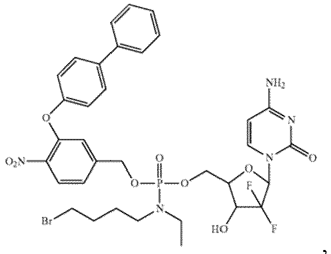 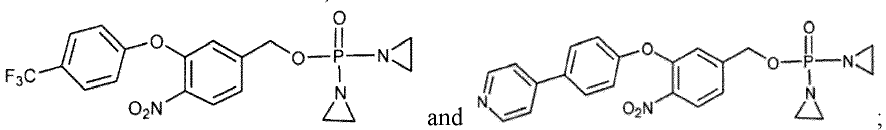
the AKR1C3 enzyme-activated prodrug compound of formula (6) is selected from the compounds having structures represented by the following formulae: 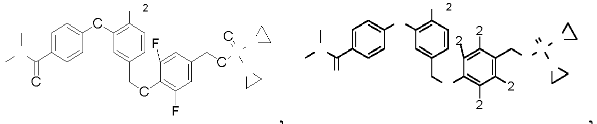 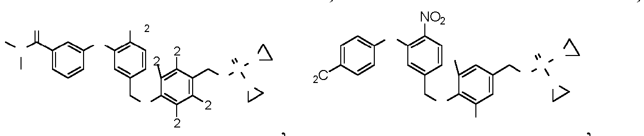 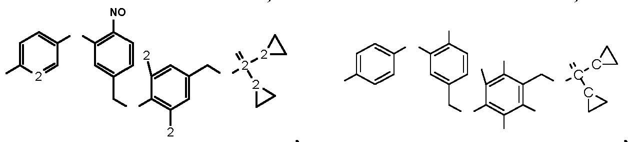 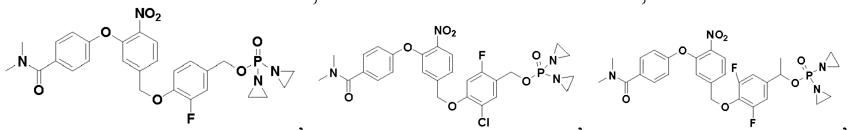 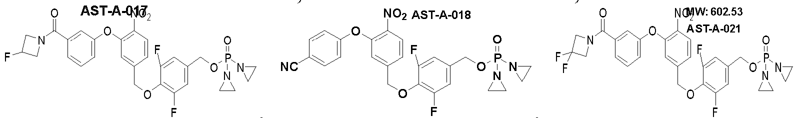 and 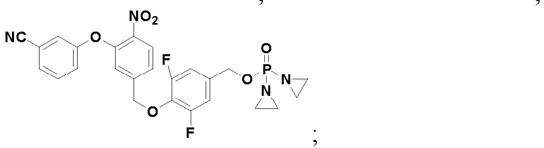
the AKR1C3 enzyme-activated prodrug compound of formula (5) is selected from the compounds having structures represented by the following formulae: 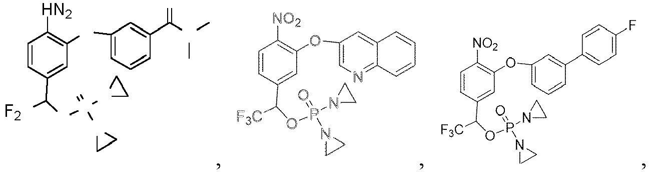 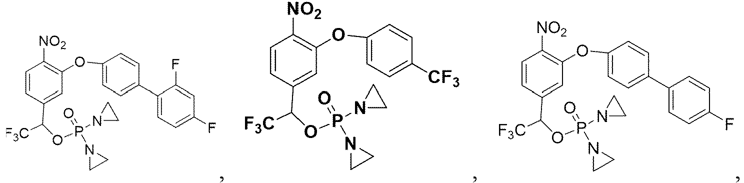 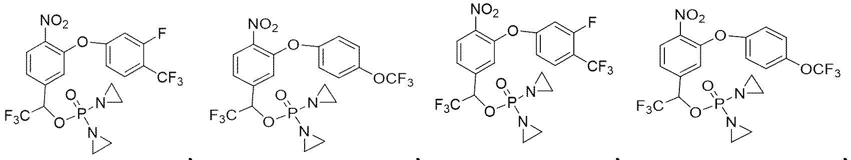 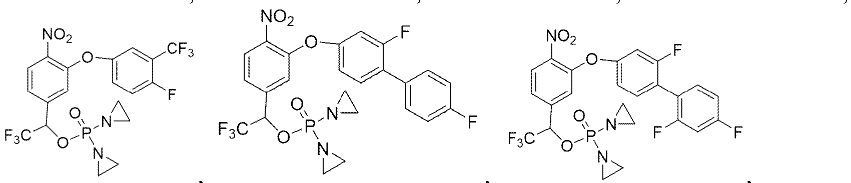 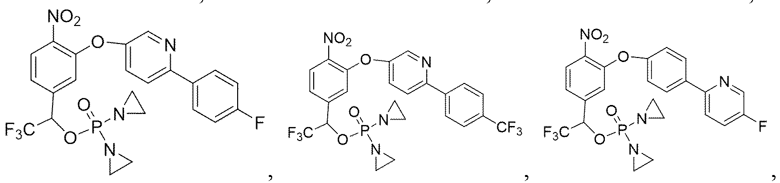 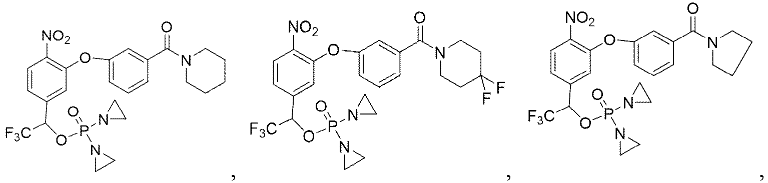 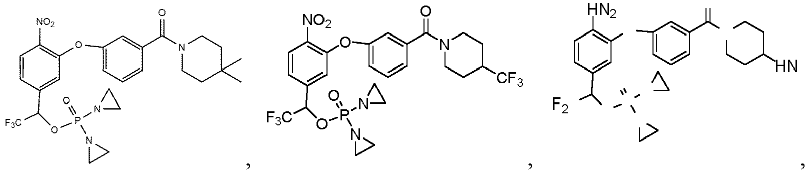 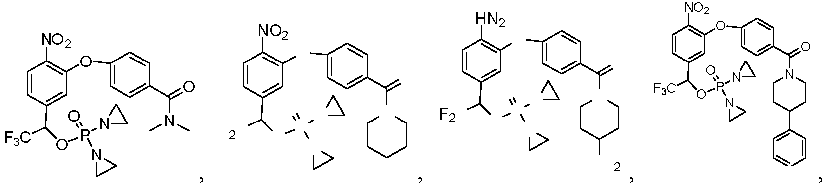 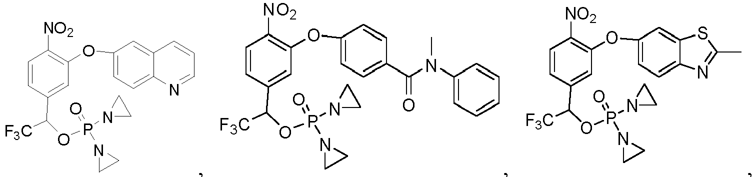 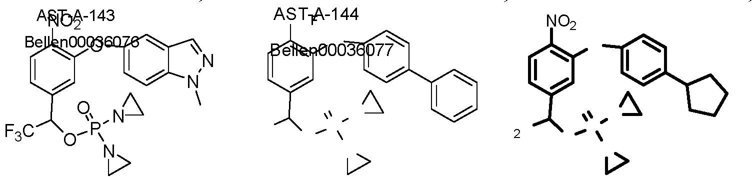 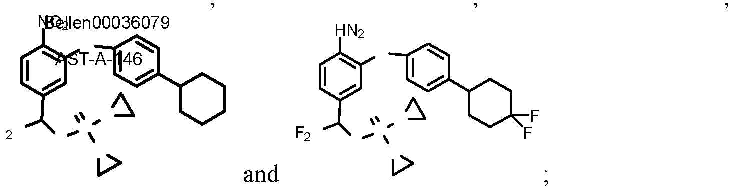
the AKR1C3 enzyme-activated prodrug compound of formula (7) is selected from the compounds having structures represented by the following formulae: 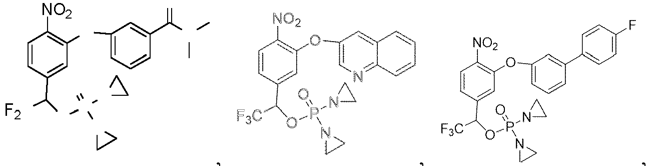 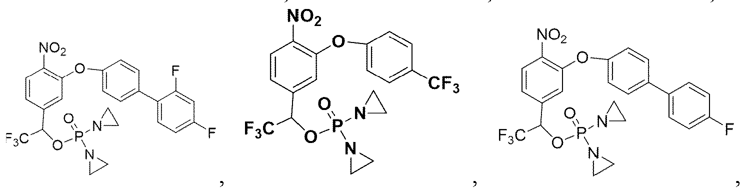 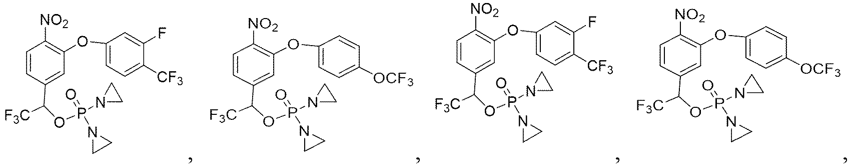 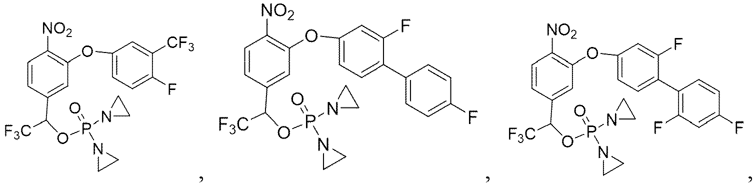 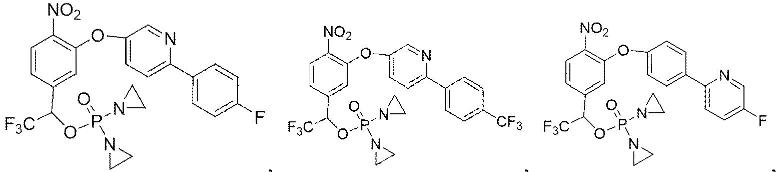 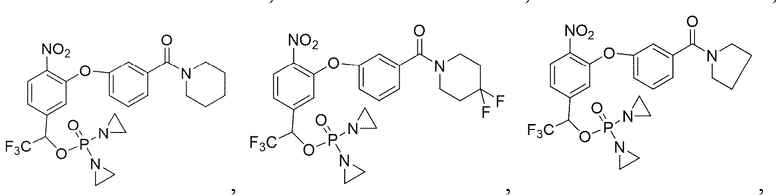 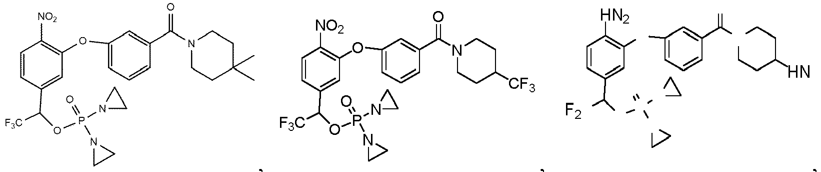 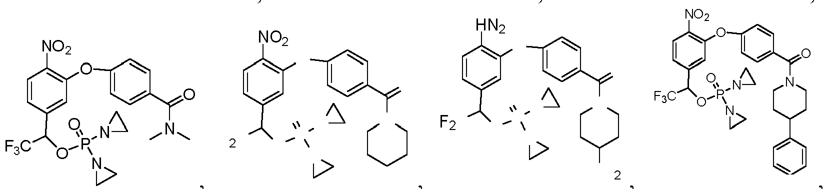 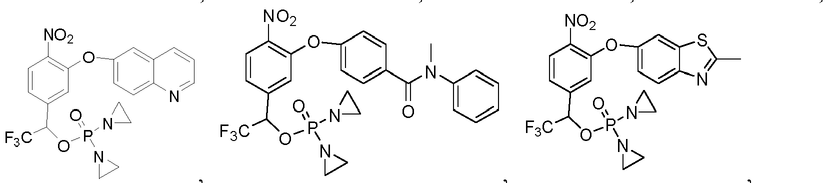 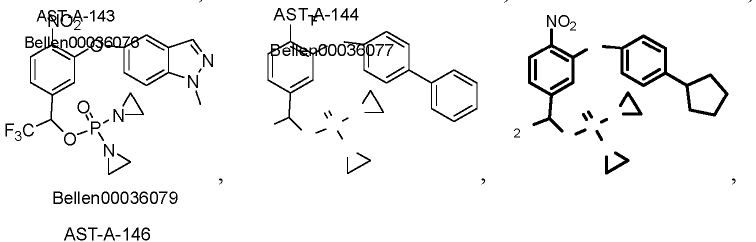 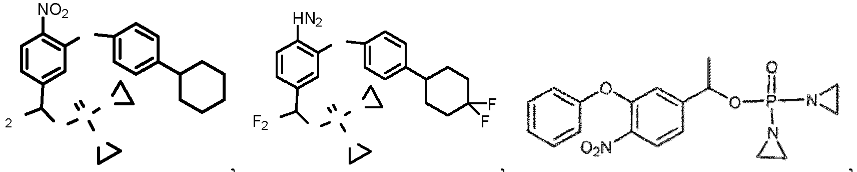 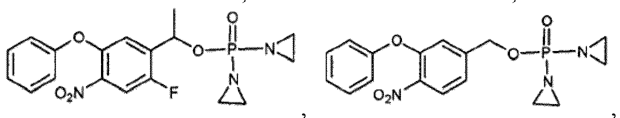 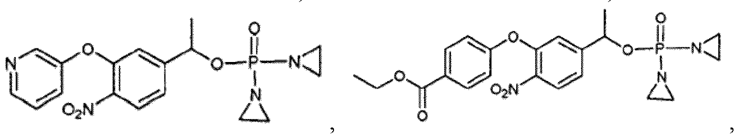 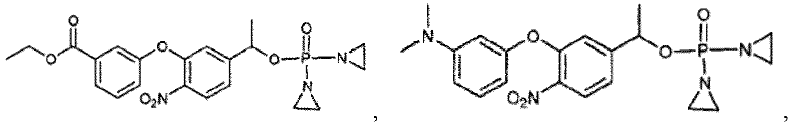 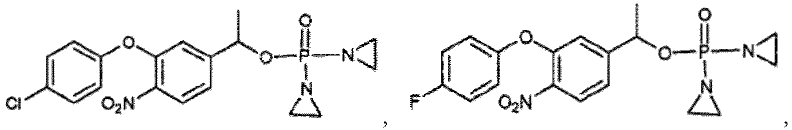 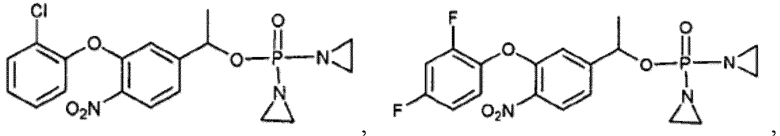 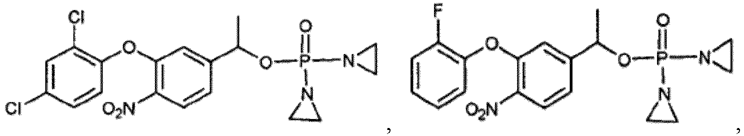 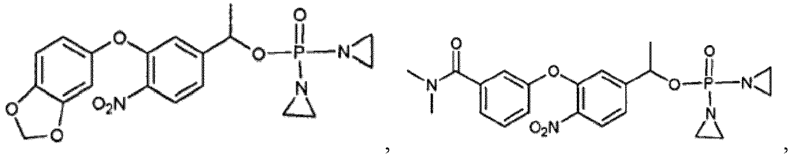 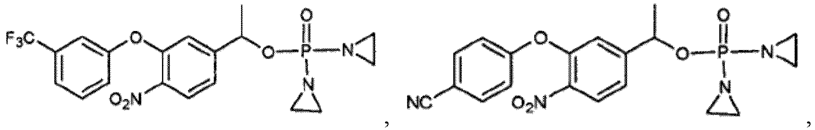 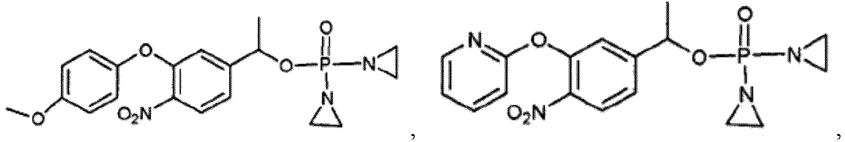 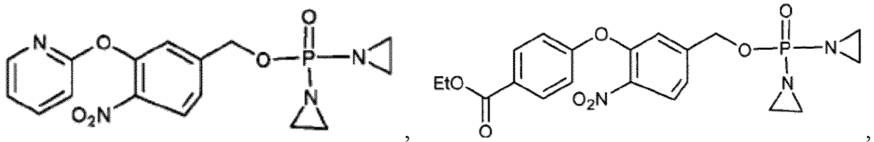 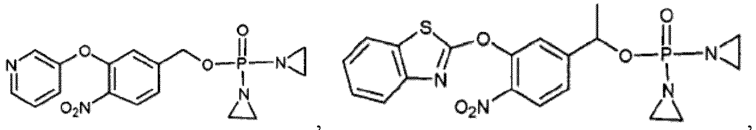 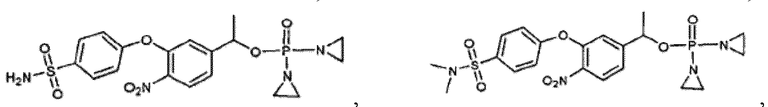 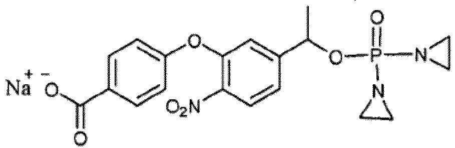 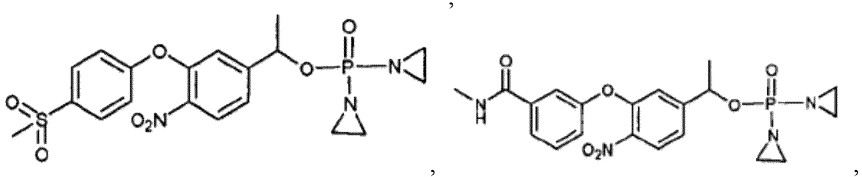 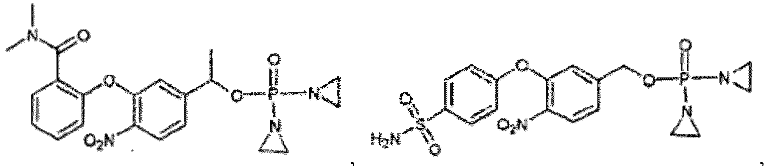 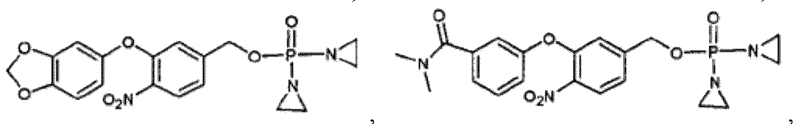 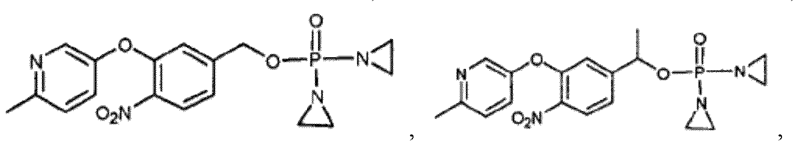 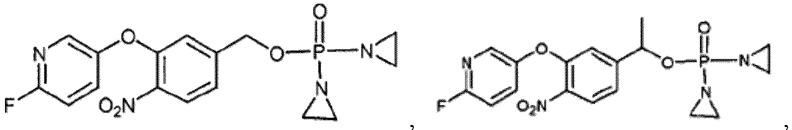 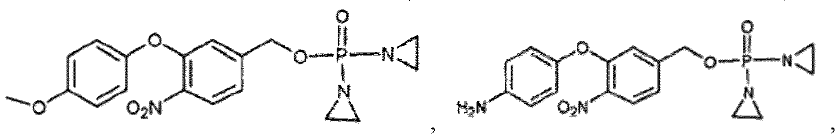 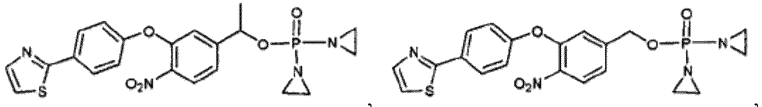 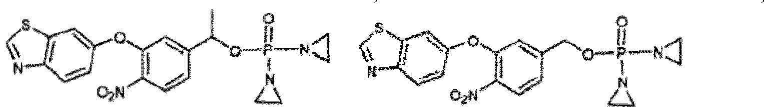 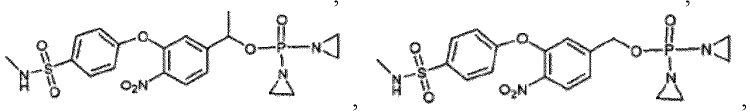 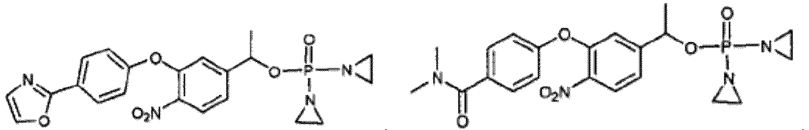 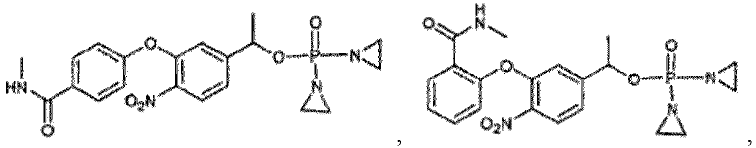 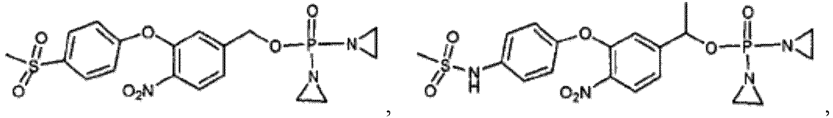 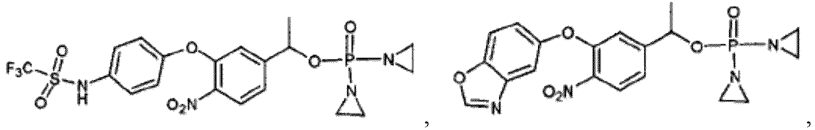 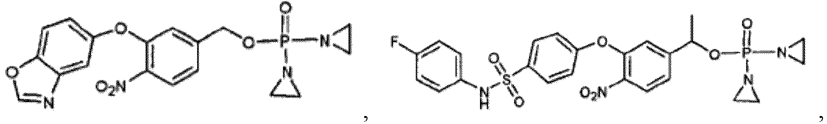 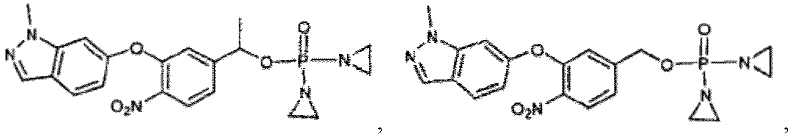 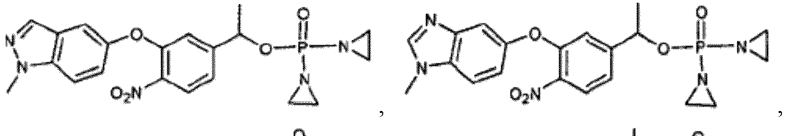 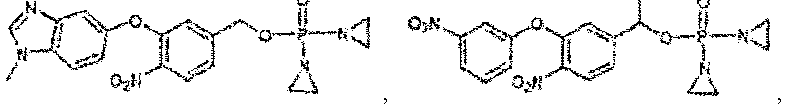 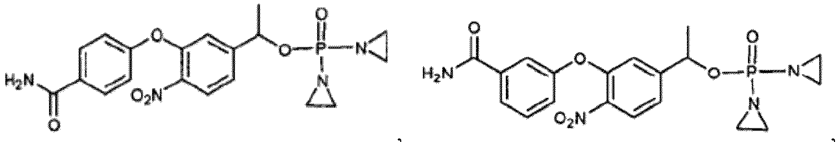 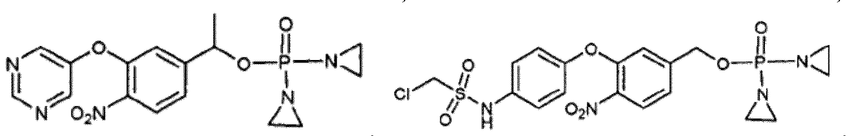 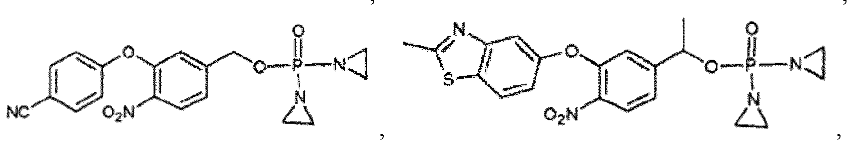 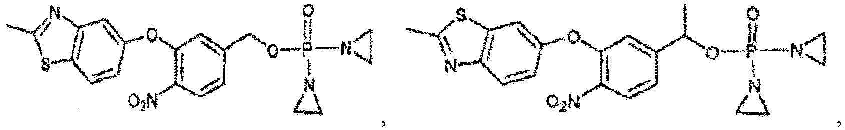 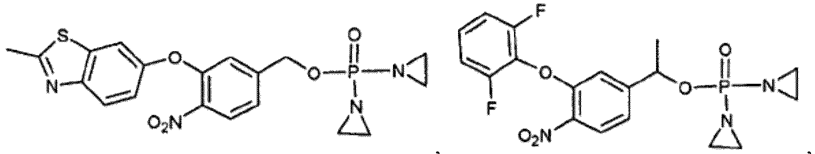 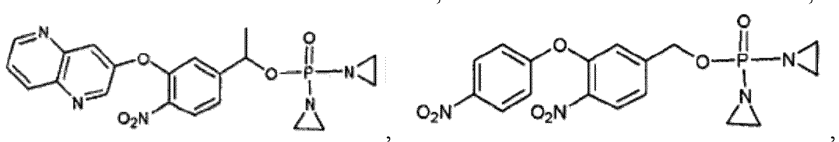 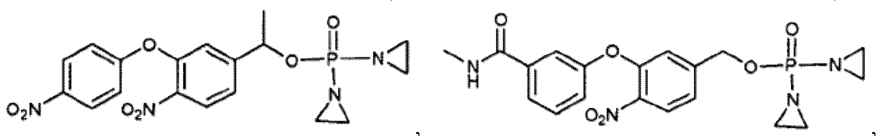 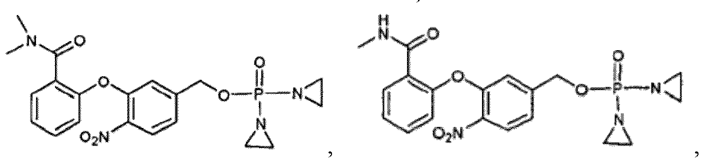 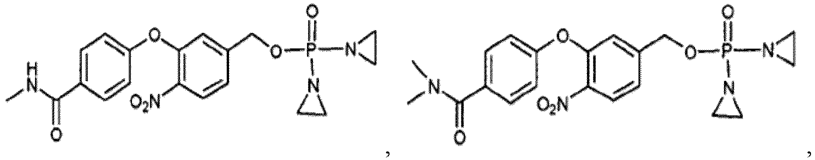 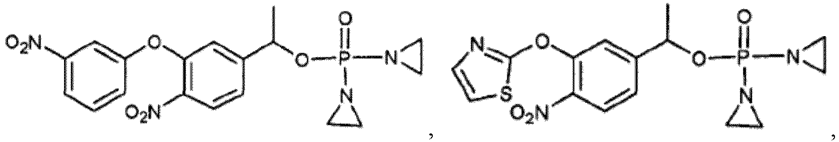 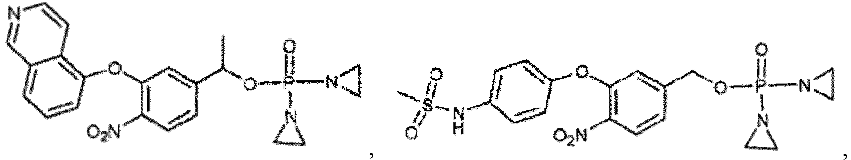 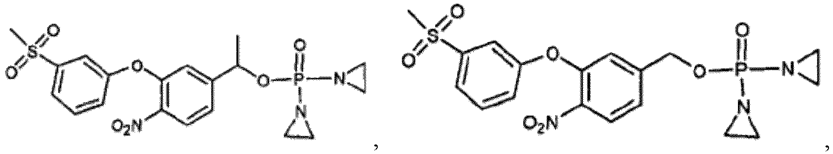 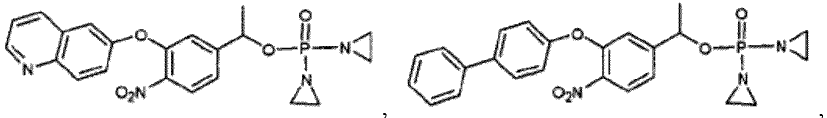 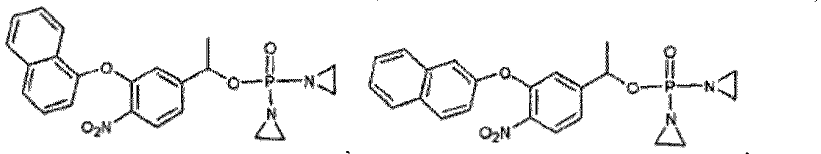 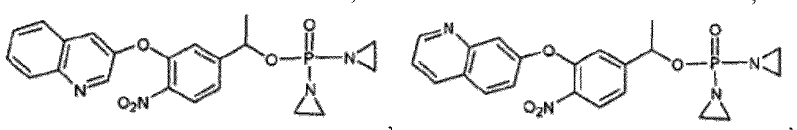 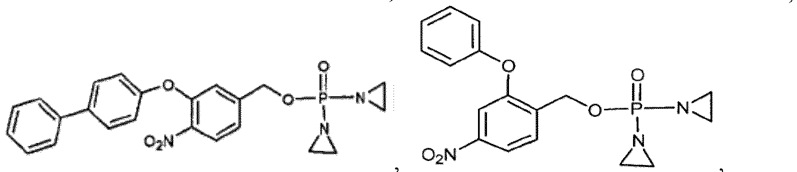 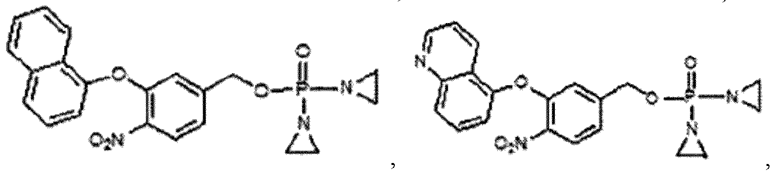 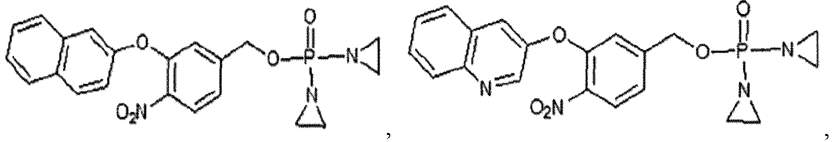 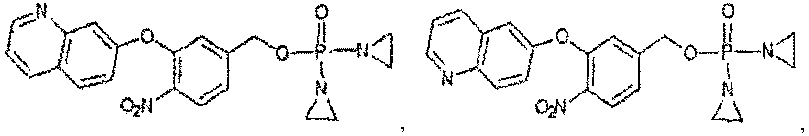 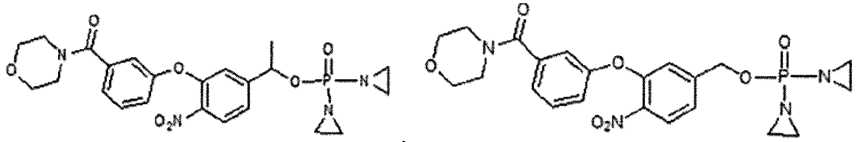 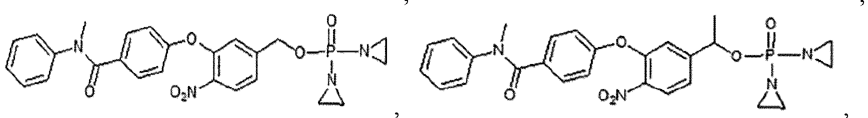 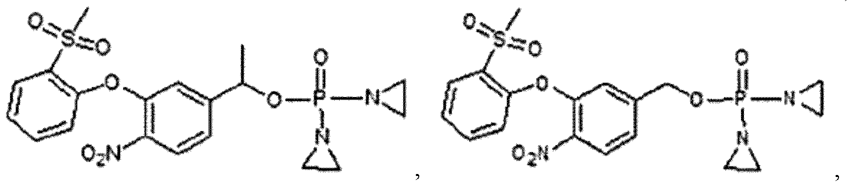 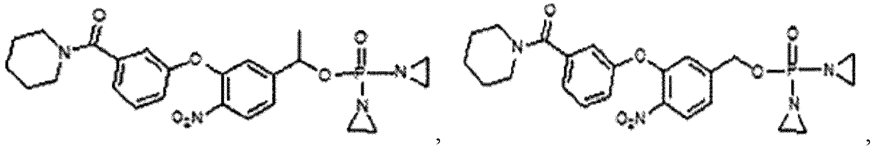 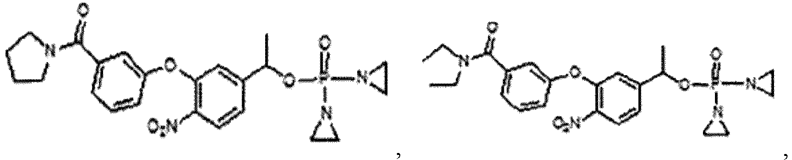 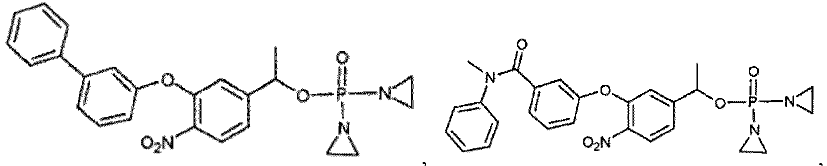 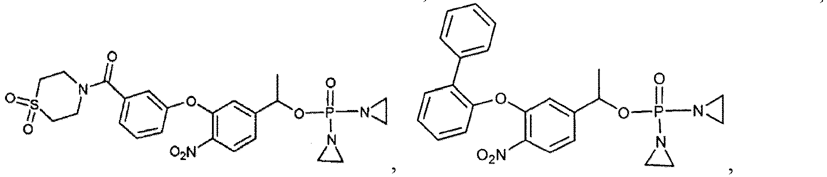 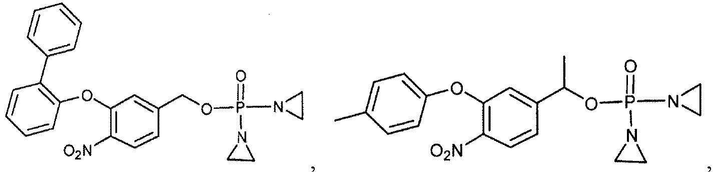 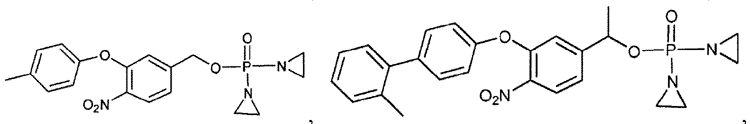 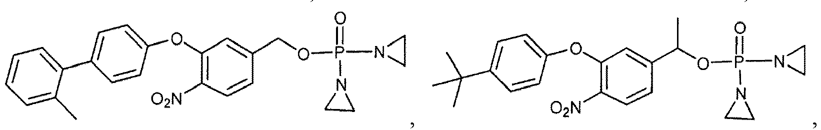 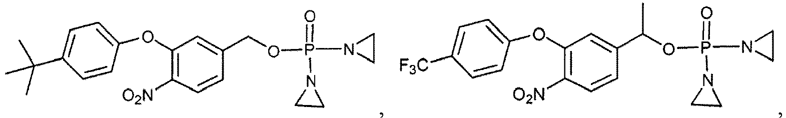 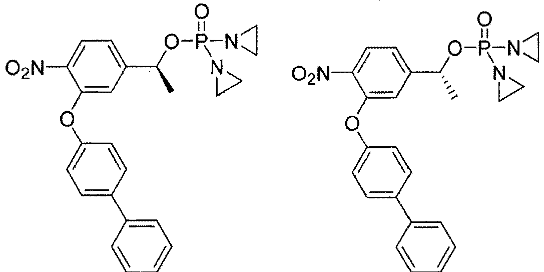 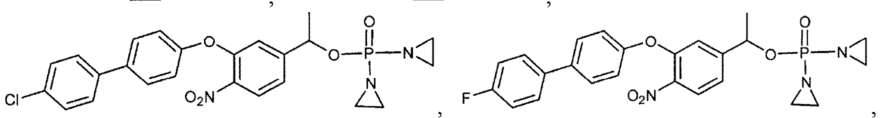 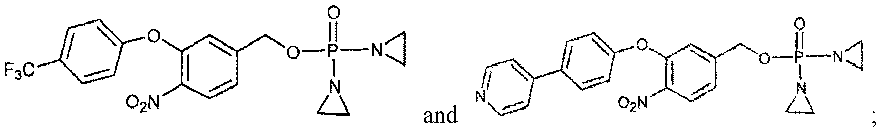
the AKR1C3 enzyme-activated prodrug compound of formula (8) is selected from the compounds having structures represented by the following formulae: 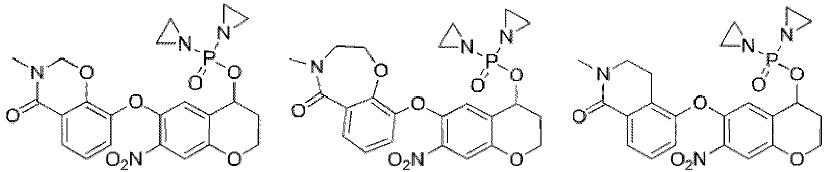 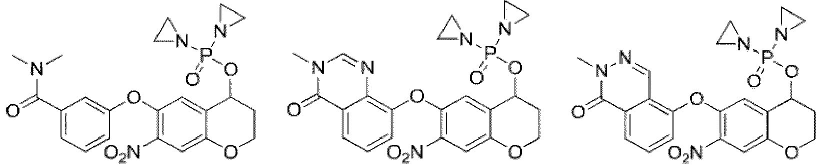 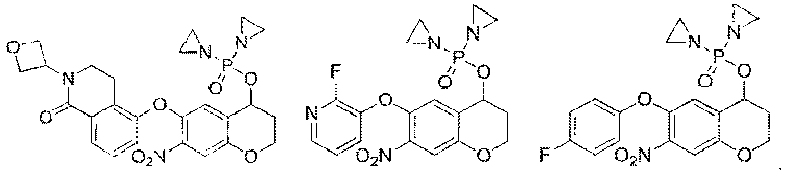 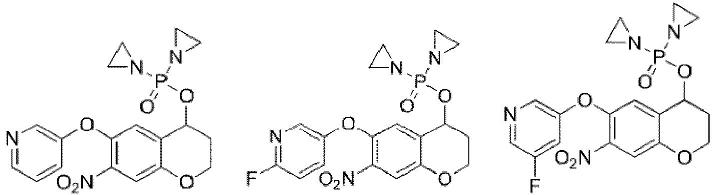 ; and
the AKR1C3 enzyme-activated prodrug compound of formula (9) is selected from the compounds having structures represented by the following formulae:

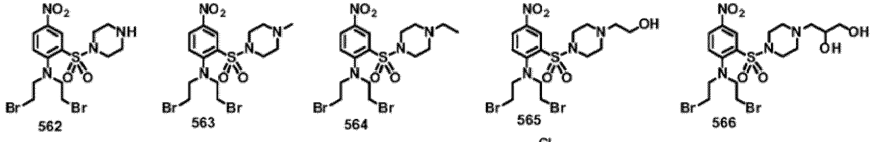

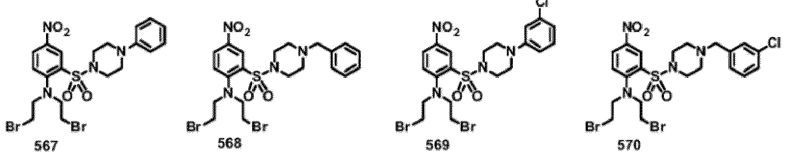

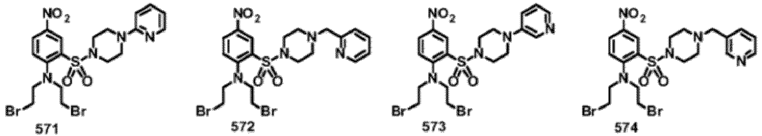

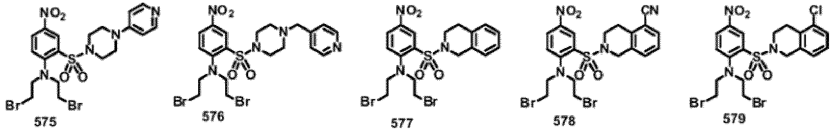

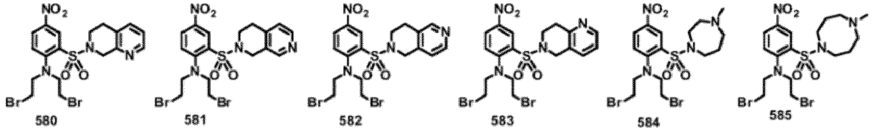

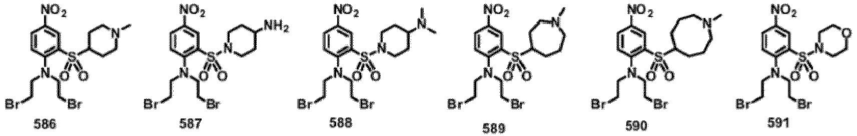

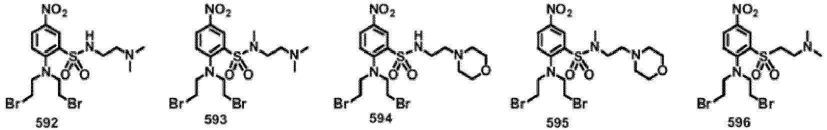

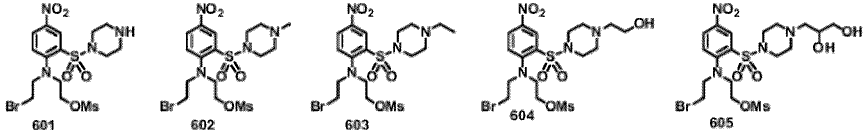

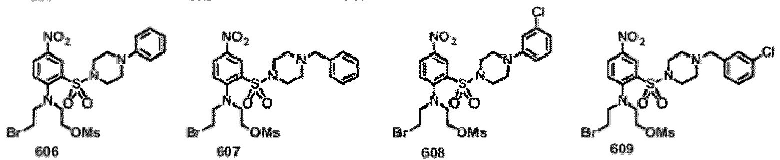

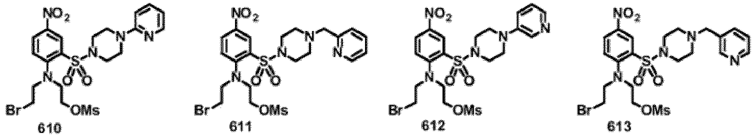

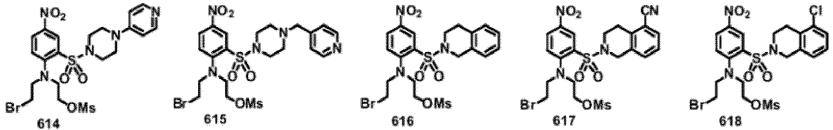

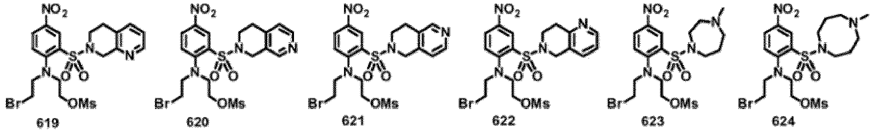

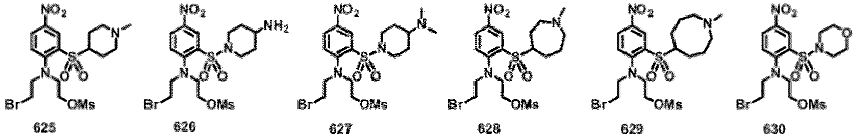

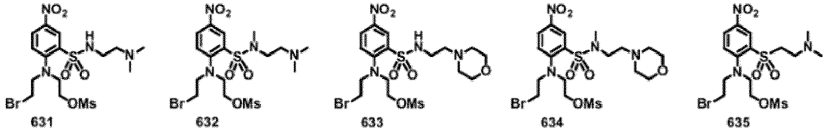

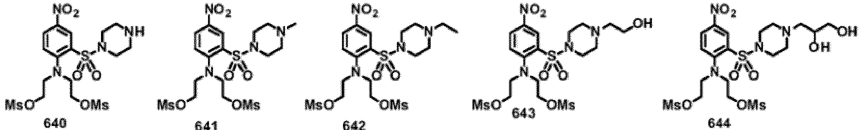

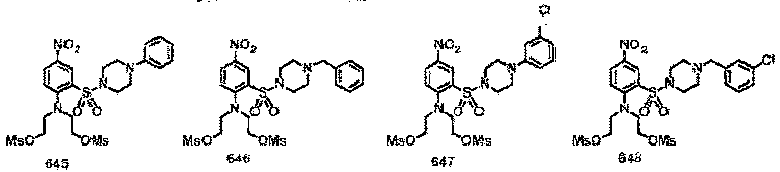

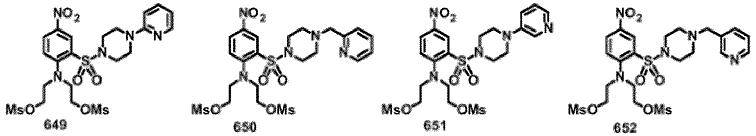

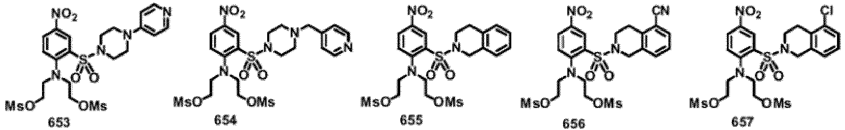

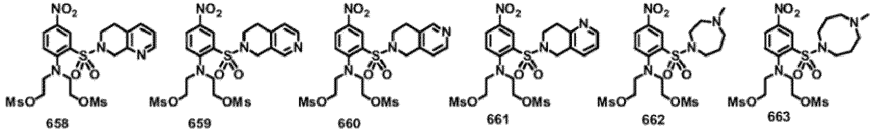

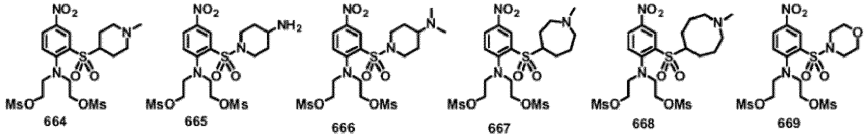

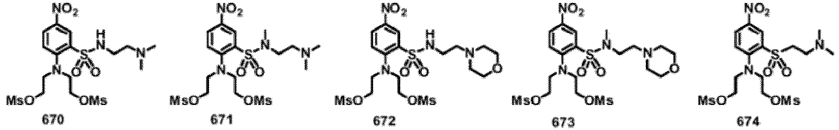

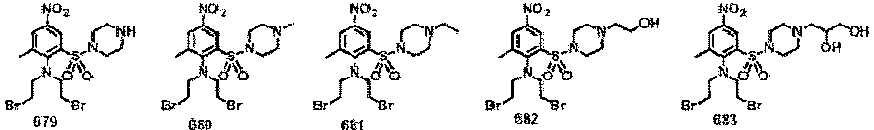

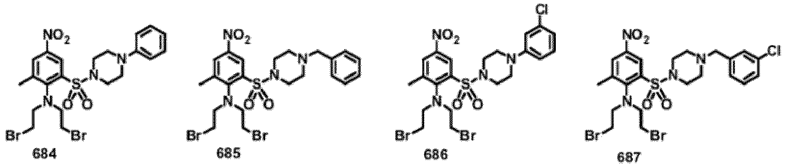

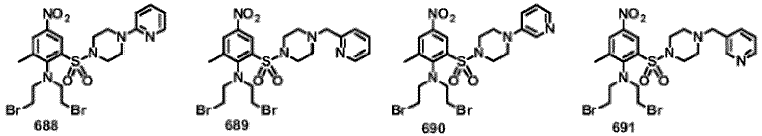

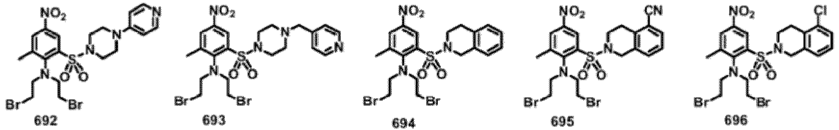

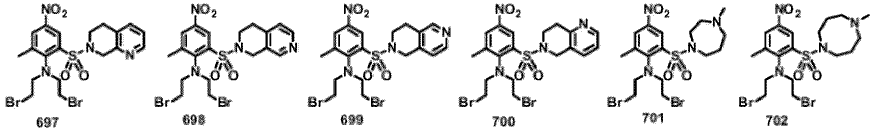

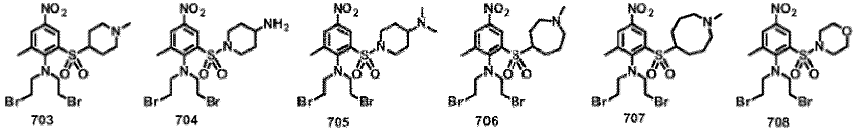

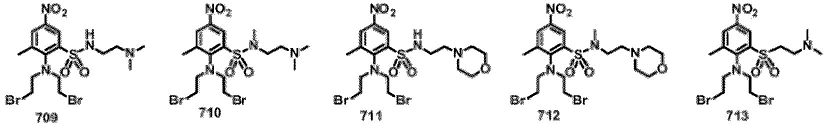

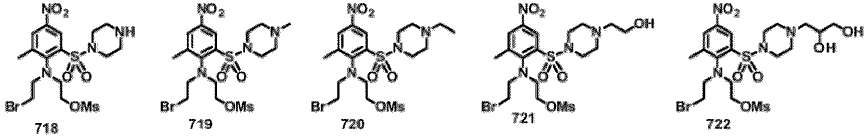

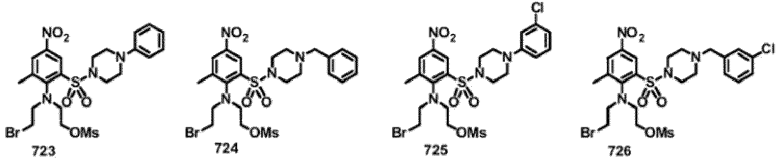

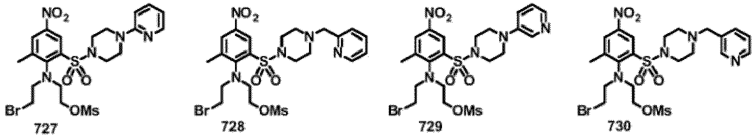

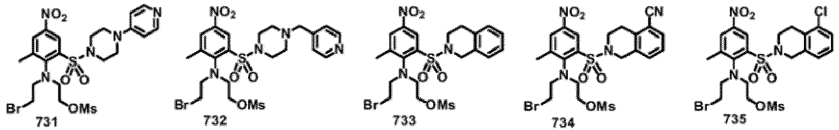

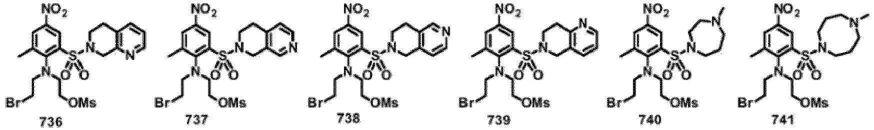

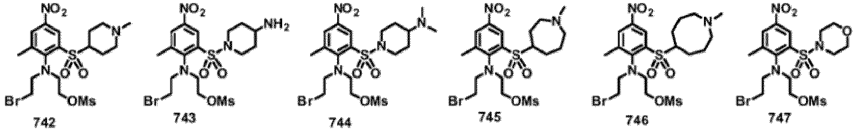

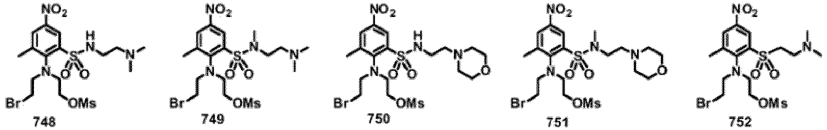

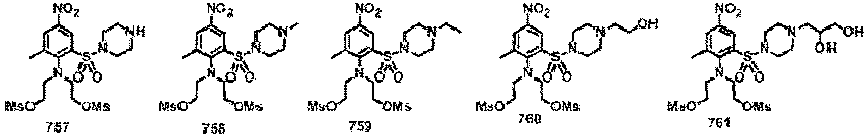

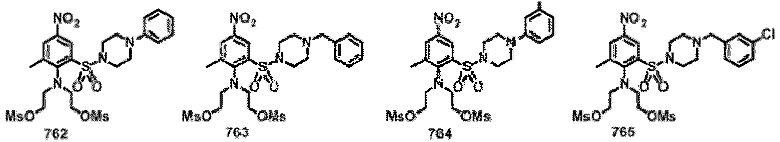

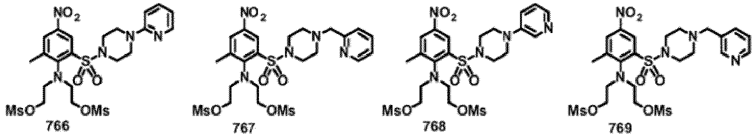

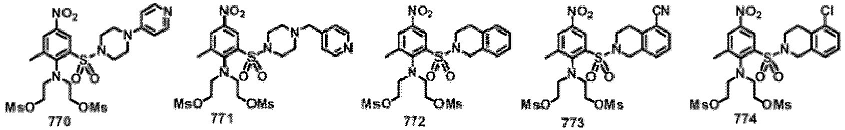

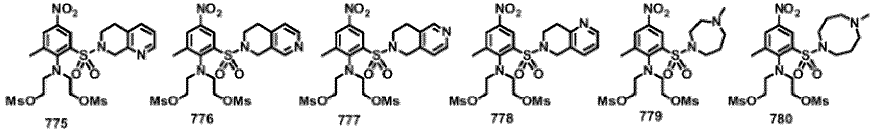

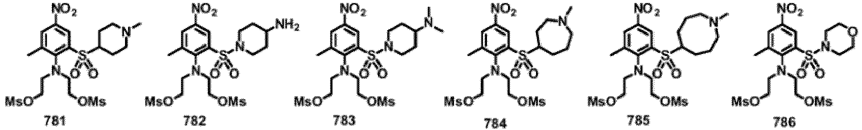

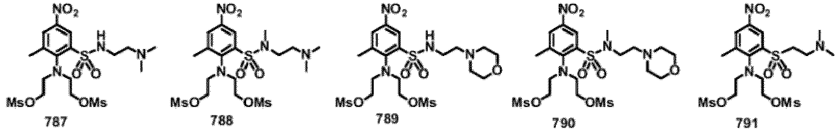

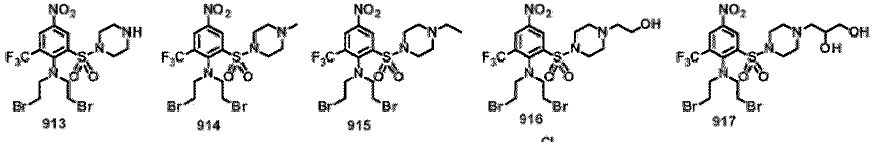

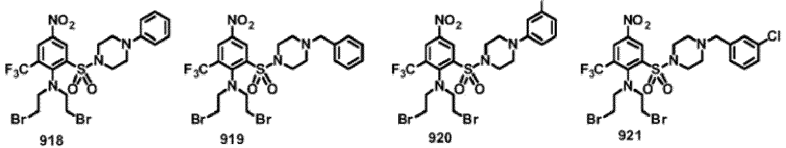

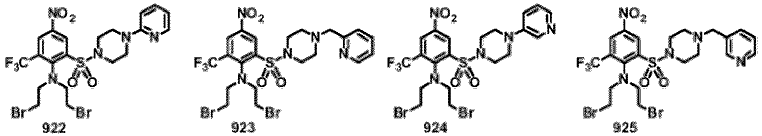

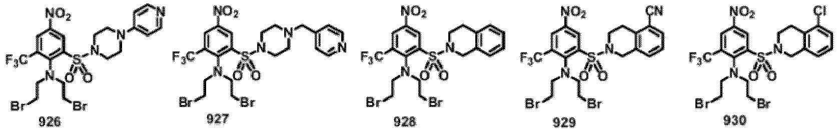

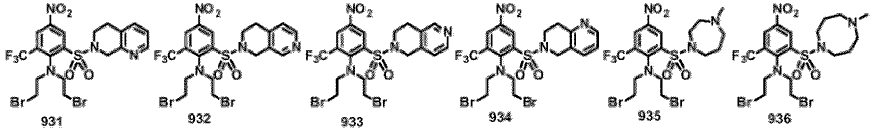

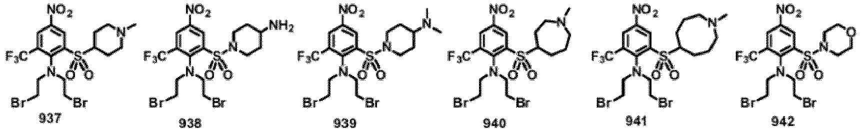

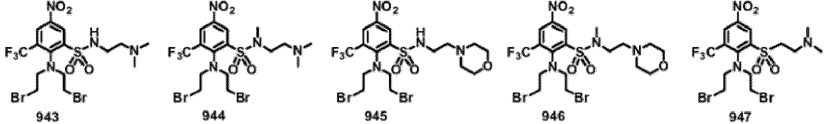

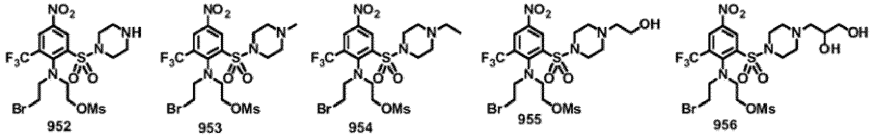

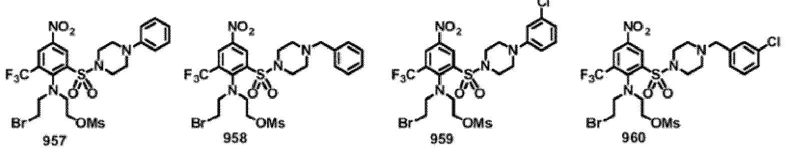

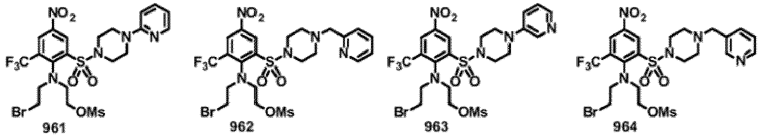

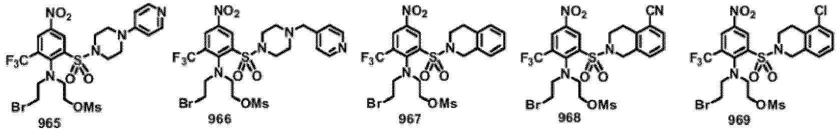

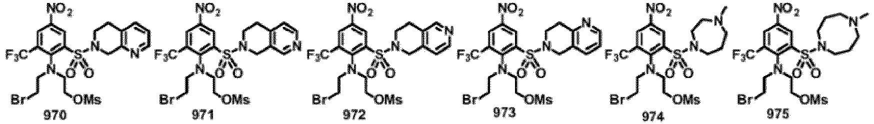

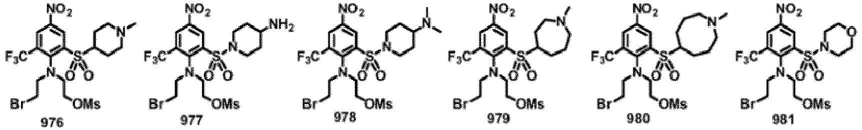

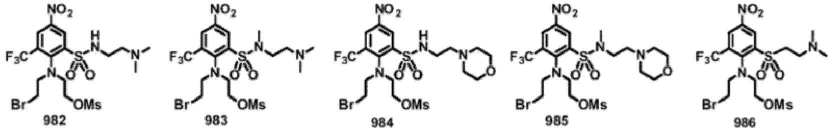

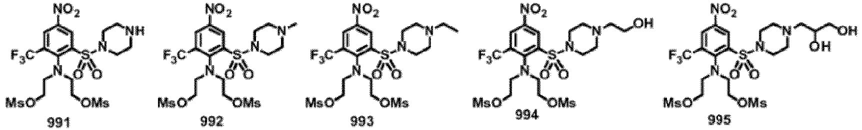

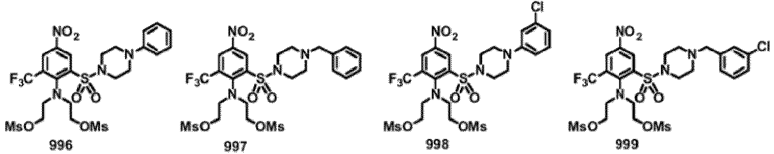

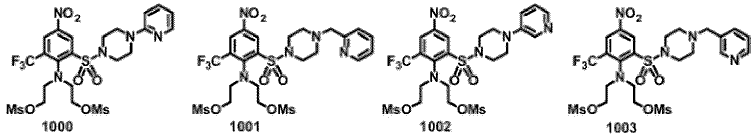

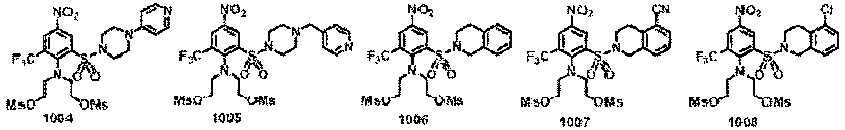

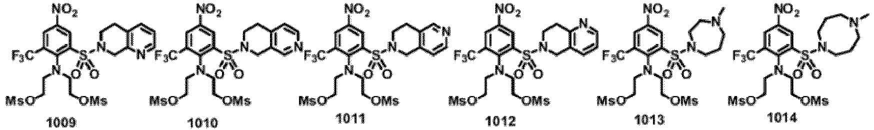

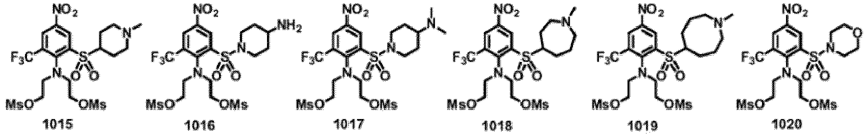

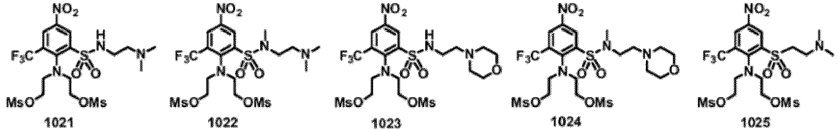

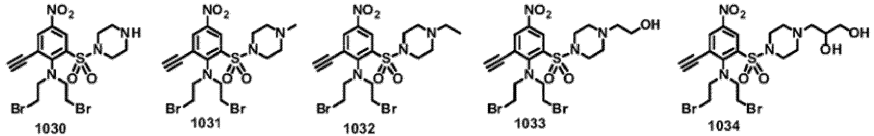

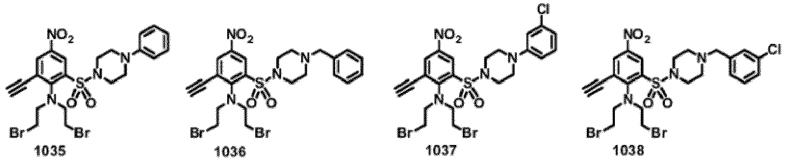

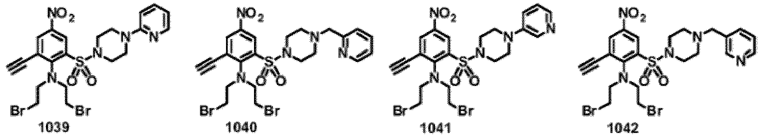

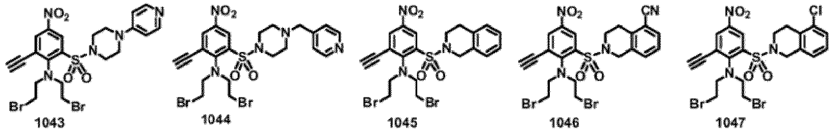

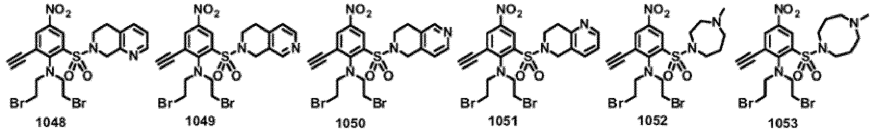

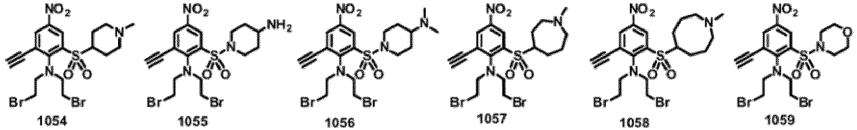

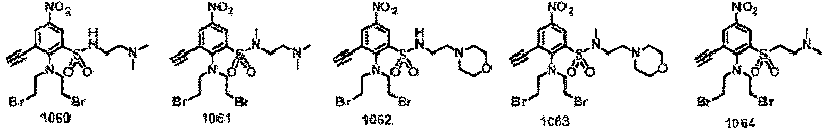

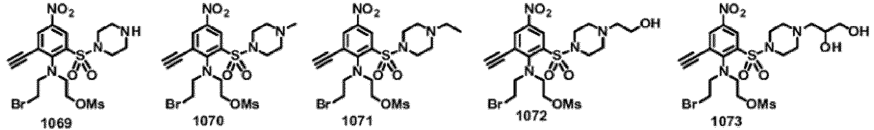

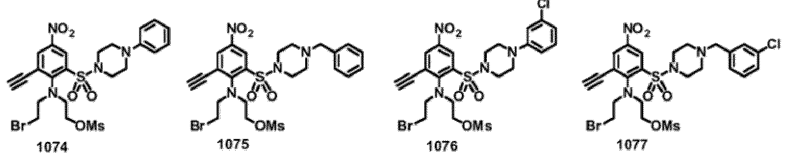

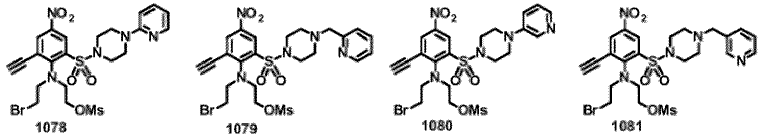

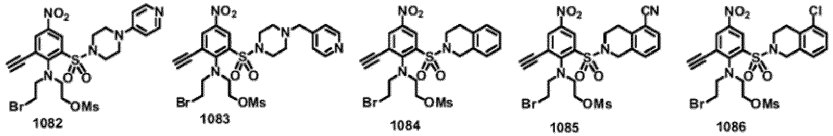

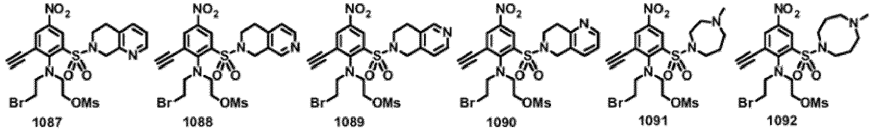

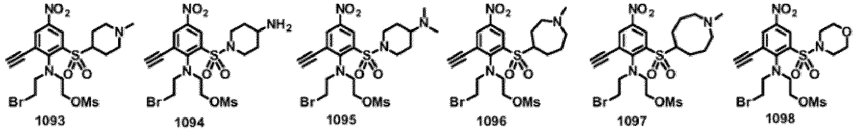

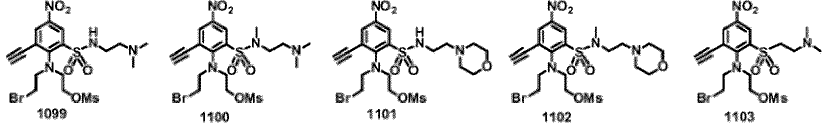

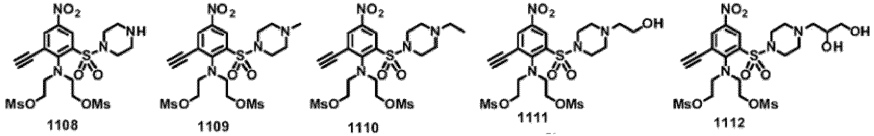

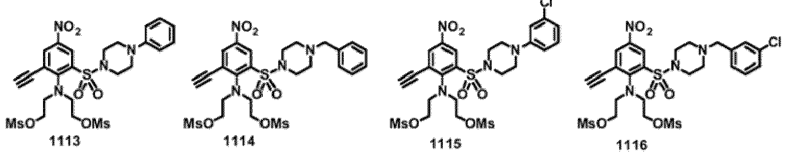

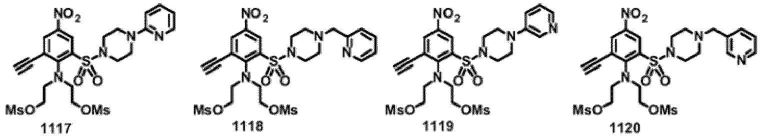

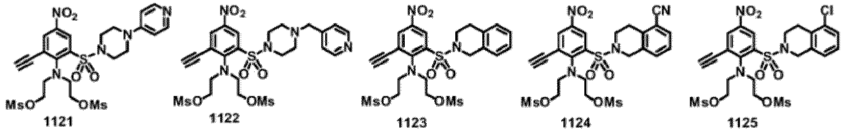

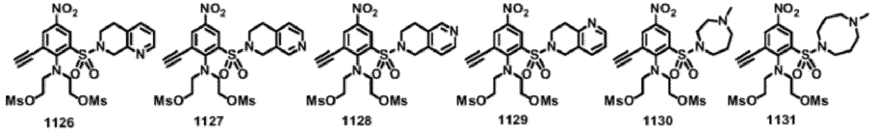

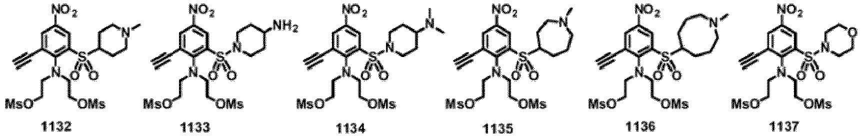

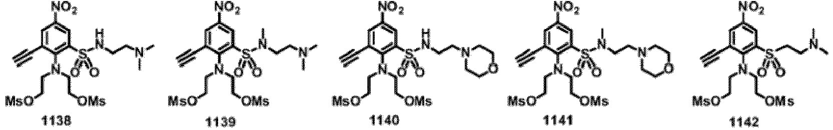

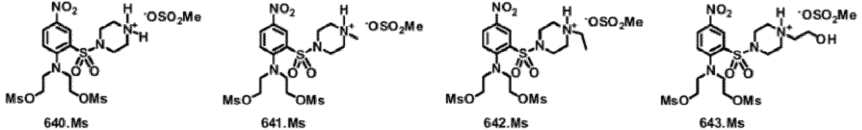

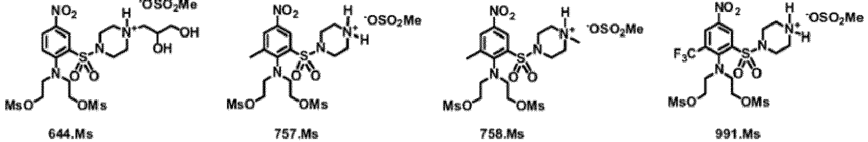

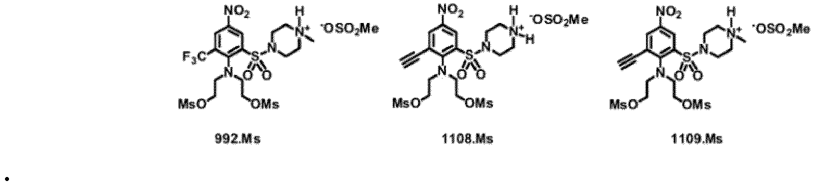

8. A pharmaceutical composition comprising an alkylating agent prodrug compound or salt, ester, solvate or isotopomer thereof and a cell cycle inhibitor compound or salt, ester, solvate or isotopomer thereof, wherein the pharmaceutical composition is used to treat a cancer/tumor patient.

9. The pharmaceutical composition of claim 8, wherein:
the alkylating agent prodrug compound is selected from an AKR1C3 enzyme-activated alkylating agent prodrug compound or a hypoxia-activated alkylating agent prodrug compound, preferably from an AKR1C3 enzyme-activated DNA alkylating agent prodrug compound or a hypoxia-activated DNA alkylating agent prodrug compound; or
the cell cycle inhibitor is selected from a CDK inhibitor, a Wee inhibitor, a CHK inhibitor and an ATR inhibitor; or
the alkylating agent prodrug compound comprises the chemical Formulae (1) to (9) as defined in claim 4; or
the cancer/tumor patient or a biological sample thereof has been detected with loss or damage of a specific gene, wherein the specific gene is selected from genes involved in cell cycle checkpoint regulation, preferably from p53, p21, CCNB1, WIP1, 14-3-3 sigma protein and cdc2/cycB; or
the CDK inhibitor is selected from palbociclib, ribociclib, abemaciclib, trilaciclib, dalpiciclib, adavosertib, Ro-3306, dinaciclib, cirtuvivint, rintodestrant, DS96432529, THZ1, THZ531, seliciclib, flavopiridol, AZD4573, SR-4835, simurosertib, fadraciclib, NVP-2, SNS-032, (E/Z)-zotiraciclib, AZD-5438, AT7519, mevociclib, kenpaullone, YKL-5-124 TFA, NG 52, GSK-3 Inhibitor IX, OTS964, samuraciclib, flavopiridol, KB-0742 dihydrochloride, ( + )-enitociclib, AUZ 454, SY-5609, SEL120-34A monohydrochloride, CCT-251921, MBQ-167, XL413 hydrochloride, BI-1347, THAL-SNS-032, JNJ-7706621, TG003, LDC4297, BMS-265246, roniciclib, CGP60474, (R)-CR8 trihydrochloride, R547, milciclib, T025, AS2863619, Senexin A, BSJ-4-116, CLK-IN-T3, CDK12-IN-3, CVT-313, atuveciclib, PHA-793887, indirubin-3'-monoxime, YKL-5-124, PHA-767491 hydrochloride, KH-CB19, cucurbitacin E, purvalanol A, BSJ-03-204, ON123300, CDK5 inhibitor 20-223, riviciclib, FN-1501, CP-10, THZ2, abemaciclib metabolite M2, BS-181, CDK12-IN-E9, samuraciclib, RGB-286638, CDK2-IN-4, LDC000067, ML167, purvalanol B, NU6300, CLK1-IN-1, FMF-04-159-2, CKI-7, CDKI-73, MSC2530818, BSJ-04-132, NU6102, voruciclib and olomoucine,
the Wee inhibitor is selected from Wee1-IN-5, Wee1-IN-3, Wee1-IN-4, LEB-03-146, LEB-03-144, adavosertib, LEB-03-153, LEB-03-145, PD407824, PD0166285, PD0166285 dihydrochloride, pomalidomide-C3-adavosertib, DB0614 and FMF-06-098-1,
the CHK inhibitor is selected from AZD7762, prexasertib, SCH900776, GDC-0425, CHK1-IN-6, CCT245737, BML-277, CCT241533, PD407824, CHIR-124, CCT244747, PF477736, GDC-0575, SB-218078, MRT00033659, ANI-7, SAR-020106, CCT241533, CHK1-IN-4, VER-00158411, CHK1-IN-3, CHK1-IN-5, CHK1-IN-2 and CHK-IN-1, and
the ATR inhibitor is selected from ceralasertib, berzosertib, gartisertib, BAY1895344, BAY-937, AZ20, ETP-46464, dactolisib, VE-821, M1774, ATRN-199, RP-3500 and ART-0380; or
the alkylating agent prodrug compound is selected from the compounds as defined in claim 7.

10. Use of an alkylating agent prodrug compound or salt, ester, solvate or isotopomer thereof for preparing a drug for cancer/tumor treatment in combination therapy with a cell cycle inhibitor compound or salt, ester, solvate or isotopomer thereof.

11. The use of claim 10, wherein:
the alkylating agent prodrug compound is selected from an AKR1C3 enzyme-activated alkylating agent prodrug compound or a hypoxia-activated alkylating agent prodrug compound, preferably from an AKR1C3 enzyme-activated DNA alkylating agent prodrug compound or a hypoxia-activated DNA alkylating agent prodrug compound; or
the cell cycle inhibitor is selected from a CDK inhibitor, a Wee inhibitor, a CHK inhibitor and an ATR inhibitor; or
the alkylating agent prodrug compound comprises the chemical formulae (1) to (9) as defined in claim 4; or
the cancer/tumor patient or a biological sample thereof has been detected with loss or damage of a specific gene, wherein the specific gene is selected from genes involved in cell cycle checkpoint regulation, preferably from p53, p21, CCNB1, WIP1, 14-3-3 sigma protein and cdc2/cycB; or
the CDK inhibitor is selected from palbociclib, ribociclib, abemaciclib, trilaciclib, dalpiciclib, adavosertib, Ro-3306, dinaciclib, cirtuvivint, rintodestrant, DS96432529, THZ1, THZ531, seliciclib, flavopiridol, AZD4573, SR-4835, simurosertib, fadraciclib, NVP-2, SNS-032, (E/Z)-zotiraciclib, AZD-5438, AT7519, mevociclib, kenpaullone, YKL-5-124 TFA, NG 52, GSK-3 Inhibitor IX, OTS964, samuraciclib, flavopiridol, KB-0742 dihydrochloride, ( + )-enitociclib, AUZ 454, SY-5609, SEL120-34A monohydrochloride, CCT-251921, MBQ-167, XL413 hydrochloride, BI-1347, THAL-SNS-032, JNJ-7706621, TG003, LDC4297, BMS-265246, roniciclib, CGP60474, (R)-CR8 trihydrochloride, R547, milciclib, T025, AS2863619, Senexin A, BSJ-4-116, CLK-IN-T3, CDK12-IN-3, CVT-313, atuveciclib, PHA-793887, indirubin-3'-monoxime, YKL-5-124, PHA-767491 hydrochloride, KH-CB19, cucurbitacin E, purvalanol A, BSJ-03-204, ON123300, CDK5 inhibitor 20-223, riviciclib, FN-1501, CP-10, THZ2, abemaciclib metabolite M2, BS-181, CDK12-IN-E9, samuraciclib, RGB-286638, CDK2-IN-4, LDC000067, ML167, purvalanol B, NU6300, CLK1-IN-1, FMF-04-159-2, CKI-7, CDKI-73, MSC2530818, BSJ-04-132, NU6102, voruciclib and olomoucine,
the Wee inhibitor is selected from Wee1-IN-5, Wee1-IN-3, Wee1-IN-4, LEB-03-146, LEB-03-144, adavosertib, LEB-03-153, LEB-03-145, PD407824, PD0166285, PD0166285 dihydrochloride, pomalidomide-C3-adavosertib, DB0614 and FMF-06-098-1,
the CHK inhibitor is selected from AZD7762, prexasertib, SCH900776, GDC-0425, CHK1-IN-6, CCT245737, BML-277, CCT241533, PD407824, CHIR-124, CCT244747, PF477736, GDC-0575, SB-218078, MRT00033659, ANI-7, SAR-020106, CCT241533, CHK1-IN-4, VER-00158411, CHK1-IN-3, CHK1-IN-5, CHK1-IN-2 and CHK-IN-1, and
the ATR inhibitor is selected from ceralasertib, berzosertib, gartisertib, BAY1895344, BAY-937, AZ20, ETP-46464, dactolisib, VE-821, M1774, ATRN-199, RP-3500 and ART-0380; or
the alkylating agent prodrug compound is selected from the compounds as defi ned in claim 7.
